(19) **Europäisches Patentamt / European Patent Office / Office européen des brevets**

(11) **EP 4 219 677 A1**

(12) # EUROPEAN PATENT APPLICATION
published in accordance with Art. 153(4) EPC

(43) Date of publication:
**02.08.2023 Bulletin 2023/31**

(21) Application number: **21872587.7**

(22) Date of filing: **24.09.2021**

(51) International Patent Classification (IPC):
*C12M 1/26* (2006.01)      *C12M 1/08* (2006.01)
*C12N 5/071* (2010.01)     *G01N 1/00* (2006.01)
*G01N 1/04* (2006.01)

(52) Cooperative Patent Classification (CPC):
**C12M 1/08; C12M 1/26; C12N 5/06; G01N 1/00; G01N 1/04**

(86) International application number:
**PCT/JP2021/035198**

(87) International publication number:
**WO 2022/065458 (31.03.2022 Gazette 2022/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.09.2020   JP 2020160264**

(71) Applicant: **NIKON CORPORATION**
**Minato-ku**
**Tokyo 108-6290 (JP)**

(72) Inventors:
• **MORIYAMA Masaki**
  **Tokyo 108-6290 (JP)**

• **ISHIZAWA Naoya**
  **Tokyo 108-6290 (JP)**
• **KOBAYASHI Ryo**
  **Tokyo 108-6290 (JP)**
• **TANAKA Shuhei**
  **Tokyo 108-6290 (JP)**
• **NAKAMURA Taichi**
  **Tokyo 108-6290 (JP)**
• **HAYASHI Seri**
  **Tokyo 108-6290 (JP)**
• **TAKUBO Makiko**
  **Tokyo 108-6290 (JP)**
• **YOKOYAMA Kaede**
  **Tokyo 108-6290 (JP)**

(74) Representative: **Hoffmann Eitle**
**Patent- und Rechtsanwälte PartmbB**
**Arabellastraße 30**
**81925 München (DE)**

(54) **METHOD OF APPLYING FORCE TO ORGANISM AND DEVICE FOR APPLYING FORCE TO ORGANISM**

(57)      According to a first aspect of the present invention, a method of applying a force to an organism is provided which includes: forming a gas-liquid interface between a liquid, in which an organism is immersed, and a gas in a flow channel or at an end portion of the flow channel after arranging the end portion in the liquid; controlling a vector of a force applied from the gas-liquid interface to the organism; and applying a force to the organism from the gas-liquid interface.

FIG.14

EP 4 219 677 A1

**Description**

BACKGROUND

1. TECHNICAL FIELD

The present invention relates to a method of applying a force to an organism and a device for applying a force to an organism.

2. RELATED ART

In cell biology research and the like, specific cells are sucked from many cells in a culture vessel. Patent Document 1 discloses a system that supports suction work for characteristic cells in a large amount of cells.

Prior Art Document

Patent Document 1: Japanese Patent Application Publication No. 2019-030263

GENERAL DISCLOSURE

In a first aspect of the present invention, a method of applying a force to an organism is provided. The method of applying a force to an organism may include forming a bubble forming a gas-liquid interface between a liquid, in which an organism is immersed, and a gas in a flow channel or at an end portion of the flow channel after arranging the end portion in the liquid. The method of applying a force to an organism may include controlling a vector of a force applied from the gas-liquid interface to the organism. The method of applying a force to the organism may include applying a force to the organism from the gas-liquid interface.
In a second aspect of the present invention, the controlling the vector may include controlling a magnitude of the force applied from the gas-liquid interface to the organism.
In a third aspect of the present invention, the controlling the vector may include controlling the magnitude of the force applied from the gas-liquid interface to the organism by controlling a gas pressure of the gas at the gas-liquid interface.
In a fourth aspect of the present invention, the forming the gas-liquid interface may be performed by immersing the end portion of the flow channel in the liquid and introducing a gas, which is supplied from a pump, from the end portion into the liquid, and the controlling the vector may include controlling the gas pressure of the gas at the gas-liquid interface by controlling pressurization of the pump.
In a fifth aspect of the present invention, the forming the gas-liquid interface may be performed by immersing the end portion of the flow channel in the liquid and introducing a gas from the end portion into the liquid, and the controlling the vector may include preparing a flow channel having an inner diameter corresponding to a magnitude of a force to be applied to the organism.
In a sixth aspect of the present invention, the controlling the vector may include controlling the magnitude of the force applied from the gas-liquid interface to the organism by controlling a curvature radius of a portion of the gas-liquid interface in contact with the organism.
In a seventh aspect of the invention, the controlling the vector may include controlling the magnitude of the force applied from the gas-liquid interface to the organism by controlling a surface tension between the gas and the liquid.
In an eighth aspect of the present invention, the controlling the vector may include controlling the magnitude of the force applied from the gas-liquid interface to the organism by controlling a movement acceleration of the gas-liquid interface.
In a ninth aspect of the present invention, the forming the gas-liquid interface may be performed by immersing the end portion of the flow channel in the liquid and introducing a gas from the end portion into the liquid, and the controlling the vector may include controlling the movement acceleration of the gas-liquid interface by controlling a movement acceleration of the flow channel.
In a tenth aspect of the present invention, the forming the gas-liquid interface may be performed by introducing a gas, which is supplied from a pump, from the end portion into the liquid, and the controlling the vector may include controlling the movement acceleration of the gas-liquid interface by controlling a pressurization acceleration of the pump.
In an eleventh aspect of the invention, the controlling the vector may include controlling a direction of the force applied from the gas-liquid interface to the organism.
In a twelfth aspect of the present invention, the controlling the vector may include controlling the direction of the force applied from the gas-liquid interface to the organism by controlling a direction and a moving direction of a surface of a portion of the gas-liquid interface in contact with the organism.

In a thirteenth aspect of the present invention, the controlling the vector may include controlling the direction of the surface of the portion of the gas-liquid interface in contact with the organism by controlling a curvature radius of the portion of the gas-liquid interface in contact with the organism.

In a fourteenth aspect of the present invention, the forming the gas-liquid interface may be performed by immersing the end portion of the flow channel in the liquid and introducing a gas from the end portion into the liquid, and the controlling the vector may include controlling the direction of the surface of the portion of the gas-liquid interface in contact with the organism by controlling a distance between the end portion and a bottom of a vessel accommodating the liquid.

In a fifteenth aspect of the present invention, the forming the gas-liquid interface may be performed by immersing the end portion of the flow channel in the liquid and introducing a gas from the end portion into the liquid, and the controlling the vector may include preparing a flow channel having an inner diameter corresponding to a direction of a force to be applied to the organism.

In a sixteenth aspect of the present invention, the controlling the vector may include controlling the direction of the force, which is applied from the gas-liquid interface to the organism, from a gas side of the gas-liquid interface to the liquid side or from the liquid side to the gas side of the gas-liquid interface.

In a seventeenth aspect of the invention, the controlling the vector may include control of moving the gas-liquid interface to be in contact with a predetermined organism.

In addition, in an eighteenth aspect of the present invention, a device for applying a force to an organism for manipulating an organism is provided. The device for applying a force to an organism may include a liquid in which the organism is immersed. The device for applying a force to an organism may include a flow channel which has an end portion arranged in the liquid and in which a bubble forming a gas-liquid interface between the liquid and a gas is formed at the end portion or inside the flow channel. The device for applying a force to an organism may include a pump configured to supply a gas to the flow channel or suck a gas from the flow channel. The device for applying a force to an organism may include a vector control unit configured to apply a force to the organism by the gas-liquid interface while controlling an operation of the pump and thereby controlling a vector of a force applied from the gas-liquid interface to the organism.

According to a nineteenth aspect of the present invention, a device for applying a force to an organism is provided. The device for applying a force to an organism may include a flow channel having an end portion arranged in a liquid stored in a vessel. The device for applying a force to an organism may include a pump configured to introduce a gas into the flow channel to form a gas-liquid interface at the end portion. The device for applying a force to an organism may include a position control unit configured to control a position of the vessel or the flow channel. The position control unit may be configured to move the flow channel or the vessel to a position at a first distance which is a distance allowing the gas-liquid interface to come into contact with a bottom portion of the vessel, and to move the flow channel, in a state where the gas-liquid interface formed at the end portion by the pump is brought into contact with the bottom portion of the vessel, to a second position where a distance between the end portion and the bottom portion of the vessel is shorter than the first distance.

The summary clause does not necessarily describe all necessary features of the embodiments of the present invention. The invention may also include a sub-combination of the features described above.

BRIEF DESCRIPTION OF THE DRAWINGS

Fig. 1A illustrates an example of a device configuration of an organism manipulation device 100 in the present embodiment.

Fig. 1B illustrates an example of the device configuration of the organism manipulation device 100 in the present embodiment.

Fig. 2A illustrates an example of a schematic diagram illustrating a structure of a nozzle 49 in the present embodiment.

Fig. 2B illustrates an example of the schematic diagram illustrating the structure of the nozzle 49 in the present embodiment.

Fig. 3A illustrates an example of the schematic diagram illustrating the structure of the nozzle 49 in the present embodiment.

Fig. 3B illustrates an example of the schematic diagram illustrating the structure of the nozzle 49 in the present embodiment.

Fig. 4 illustrates an example of a schematic diagram illustrating an example of a method of recovering an organism in the present embodiment.

Fig. 5 illustrates an example of a specific configuration of an information processing device 170 in the present embodiment.

Fig. 6 illustrates an example of a flow of a method of manipulating the organism in the present embodiment.

Fig. 7A illustrates an example of a GUI image displayed on an output unit 160 in the present embodiment.

Fig. 7B illustrates an example of the GUI image displayed on the output unit 160 in the present embodiment.

Fig. 7C illustrates an example of the GUI image displayed on the output unit 160 in the present embodiment.

Fig. 7D illustrates an example of the GUI image displayed on the output unit 160 in the present embodiment.

Fig. 7E illustrates an example of the GUI image displayed on the output unit 160 in the present embodiment.

Fig. 8 illustrates an example of a flow of replacing or adding the liquid in S600 in the present embodiment.

Fig. 9A illustrates an example of a flow of moving a relative position between the nozzle 49 and a cell in S200 in the present embodiment.

Fig. 9B illustrates an example of a flow of moving the relative position between the nozzle 49 and the cell in S200 in the present embodiment.

Fig. 9C illustrates an example of the flow of moving the relative position between the nozzle 49 and the cell in S200 in the present embodiment.

Fig. 10A illustrates an example of a flow of forming a bubble in S300 in the present embodiment.

Fig. 10B illustrates an example of the flow of forming the bubble in S300 in the present embodiment.

Fig. 11A illustrates an example of the flow for executing a manipulation of S400 in the present embodiment.

Fig. 11B illustrates an example of recovering cytoplasm and a cell membrane from the cell in the present embodiment.

Fig. 11C illustrates an example of separating the cell by attaching the cell to the bubble in the present embodiment.

Fig. 11D illustrates an example of a schematic diagram illustrating a method of recovering the cell in the present embodiment.

Fig. 11E illustrates an example of passaged cells in the present embodiment.

Fig. 11F illustrates an example of the passaged cells in the present embodiment.

Fig. 11G illustrates an example of analyzing recovered cells in the present embodiment.

Fig. 11H illustrates an example of a schematic diagram illustrating retained cells in the present embodiment.

Fig. 11I illustrates an example of a compressed cell in the present embodiment.

Fig. 12A is an example of a flow of removing the bubble in S500 in the present embodiment.

Fig. 12B is an example of the flow of removing the bubble in S500 in the present embodiment.

Fig. 13 illustrates an example of a flow of controlling a vector of a force applied to a manipulation target 35 by a gas-liquid interface 255 in the present embodiment.

Fig. 14 illustrates an example of a method of controlling the vector of the force applied from the gas-liquid interface 255 to the manipulation target 35 in the present embodiment.

Fig. 15 illustrates an example of a schematic diagram for explaining control of an internal pressure of a bubble 256 in the present embodiment.

Fig. 16 illustrates an example of a schematic diagram illustrating a relationship between an inner diameter of the nozzle 49 and a curvature radius r of the gas-liquid interface 255 in the present embodiment.

Fig. 17 illustrates an example of a schematic diagram illustrating a relationship between a distance between an end portion 254 of the nozzle 49 and a bottom portion 25a of a vessel 25 and the curvature radius r of the gas-liquid interface 255 in the present embodiment.

Fig. 18 illustrates an example of a schematic diagram for explaining a direction of the force applied from the gas-liquid interface 255 to the manipulation target 35 in the present embodiment.

Fig. 19 illustrates an example of a schematic diagram for explaining a method 921 of controlling the inner diameter of the nozzle 49 in the present embodiment.

Fig. 20 illustrates an example of a schematic diagram for explaining a method 922 of controlling the distance between the end portion 254 of the nozzle 49 and the bottom portion 25a of the vessel 25 in the present embodiment.

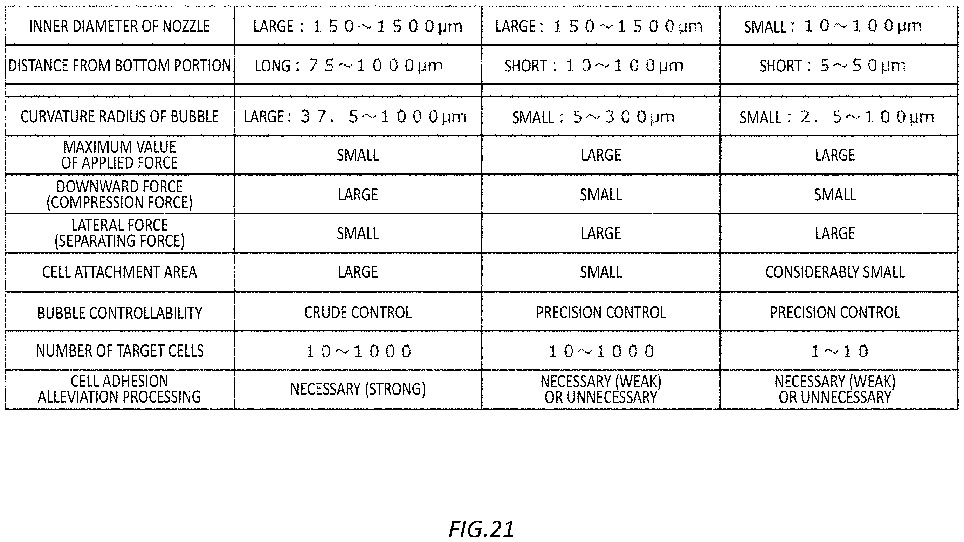

Fig. 21 illustrates an example of a schematic diagram illustrating a relationship among the inner diameter of the nozzle 49, the distance between the end portion 254 of the nozzle 49 and the bottom portion 25a of the vessel 25, the curvature radius of the gas-liquid interface 255, the maximum value of the force applied to the cell to be the manipulation target 35, the magnitude of a force (compression force) of a downward component, the magnitude of a force (separating force) of a lateral component, the area of the gas-liquid interface 255 on which the cell to be the manipulation target 35 can be attached, the controllability of the nozzle 49, the number of target cells, and the cell adhesion alleviation processing in the present embodiment.

Fig. 22 is a diagram for explaining detachment of the manipulation target 35 from the gas-liquid interface 255 in the present embodiment.

Fig. 23 illustrates an example of a hardware configuration of a computer.

DESCRIPTION OF EXEMPLARY EMBODIMENTS

Hereinafter, the invention will be described through embodiments of the invention, but the following embodiments do not limit the invention according to claims. In addition, not all combination of the features described in the embodiments are necessary for the solution of the invention. Note that in the drawings, same or similar parts are assigned with same reference signs, and duplicated descriptions may be omitted.

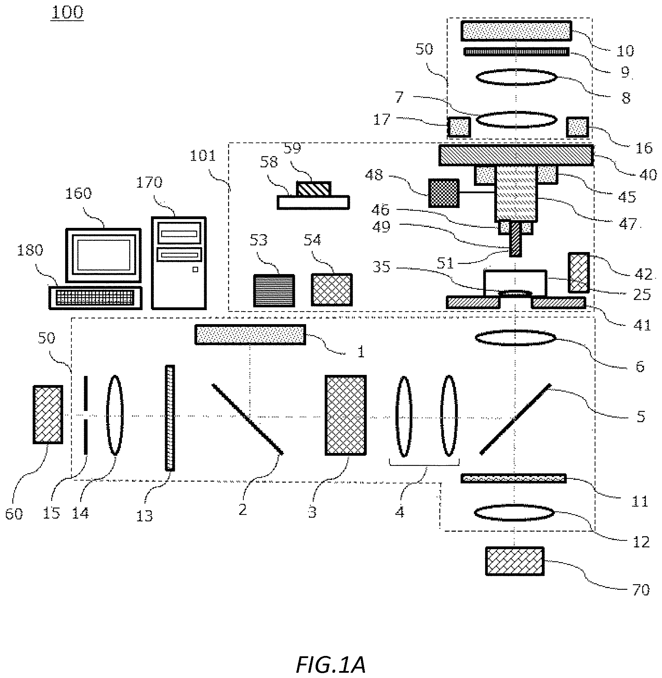

Fig. 1A illustrates an example of a device configuration of an organism manipulation device 100 in the present embodiment. The organism manipulation device 100 according to the present invention manipulates a minute organism such as a cell by using an interface between a gas and a liquid. For example, the organism manipulation device 100 can perform, on an organism, various manipulations such as attaching the organism to the interface and then separating the organism adhered on a solid phase. The organism manipulation device 100 includes a microscope unit 50, a camera 60, a camera 70, a manipulation unit 101, an output unit 160, an information processing device 170, and an input unit 180.

The microscope unit 50 is a device for observing or displaying a manipulation target 35 in an enlarged manner by using a microscope. The manipulation target 35 is an organism. The organism may be an organic creature. For example, the organism may be a cell. As an example, the cell may be an animal cell or a plant cell. As an example, the cell may be a living cell or a dead cell. In addition, for example, the organism may be a minute organism other than a cell. As an example, the minute organism may be a microorganism, a fungus, an alga, a biological tissue, a spheroid, or the like. In addition, the organism may contain an intracellular organelle.

The microscope unit 50 includes a fluorescence image observation light source 1, a dichroic mirror 2, an optical deflector 3, a relay lens 4, a dichroic mirror 5, an objective lens 6, a condenser lens 7, a condensing lens 8, a band pass filter 9, a transmission image observation light source 10, a barrier filter 11, a projection lens 12, a barrier filter 13, a projection lens 14, a pinhole 15, a light source 16, and a light source 17.

The fluorescence image observation light source 1 is a light source used when a fluorescence image of the manipulation target 35 is observed. The manipulation target 35 may be labeled with one type or two or more types of fluorescent substances, or may not be fluorescently labeled. The fluorescence image observation light source 1 emits, to the manipulation target 35, light to be excited or reflected.

The transmission image observation light source 10 is a light source used when a transmission image of the manipulation target 35 is observed. The transmission image observation light source 10 emits light to be transmitted through the manipulation target 35. The light to be transmitted through the manipulation target 35 may pass through the outside of the nozzle or may pass through the inside of the nozzle.

The configuration of the microscope unit 50 other than that described above will be described later. Note that the present invention is not limited to the example of the above description, and the microscope unit 50 may have a known configuration. For example, the configuration of the microscope unit 50 may have the configuration described in Japanese Patent Application Publication No. Hei 7-13083 or Japanese Patent No. 3814869.

The camera 60 captures a fluorescence image of the manipulation target 35 to generate an image. The image data generated by the camera 60 may be recorded in the information processing device 170 (for example, a recording unit 190 to be described later) and/or output to the output unit 160. For example, the camera 60 may be a camera that captures a fluorescence image, but is not limited thereto. In the following description, the camera 60 is a camera that captures a fluorescence image.

The camera 70 captures a transmission image of the manipulation target 35 to generate an image. The image data generated by the camera 70 may be recorded in the information processing device 170 (for example, the recording unit 190 to be described later) and/or output to the output unit 160. For example, the camera 70 may be a camera that captures a transmission image, but is not limited thereto. In the following description, the camera 70 is a camera that captures a transmission image.

The camera 60 and the camera 70 include an imaging sensor (not illustrated). The camera 60 and the camera 70 may be cooling cameras. The cooling camera is a camera that can cool the imaging sensor to suppress the noise generated by heat. The imaging sensor may be a CMOS imaging sensor (Complementary Metal Oxide Semiconductor) or a CCD imaging sensor (Charge Coupled Device). The camera 60 and the camera 70 may be housed in a housing different from the microscope unit 50.

The manipulation unit 101 manipulates the manipulation target 35 by using a gas-liquid interface between a gas and a liquid. For example, the manipulation unit 101 forms a bubble in the liquid to manipulate an organism (for example, a cell) in the liquid. The manipulation unit 101 includes all or at least some of a nozzle actuator 40, a sample actuator 41, a flow channel imaging camera 42, a light source 45, a light source 46, a pressure generation unit 47, a sensor unit 48, a nozzle 49, a flow channel 51, a flow channel exchange unit 53, a liquid storage unit 54, a sample lid 58, and a sample lid housing unit 59.

The nozzle actuator 40 mounts the nozzle 49 via the pressure generation unit 47 and moves the nozzle 49. As described later, the flow channel 51 is formed inside the nozzle 49, and a gas-liquid interface 255 of the bubble is formed at a tip portion of the flow channel 51. The nozzle actuator 40 may be operable in any of a longitudinal direction, a lateral direction, and an up-and-down direction. The nozzle actuator 40 may be operable only in the up-and-down direction. In this case, the longitudinal and lateral movements of the nozzle actuator 40 may be operated by the stage of the microscope unit 50. The nozzle actuator 40 may be operable only in the longitudinal and lateral directions. In this case, the movement of the nozzle actuator 40 in the up-and-down direction may be operated by the stage of the microscope unit 50. The nozzle actuator 40 may be fixed without operation. In this case, the movement of the nozzle actuator 40 in the longitudinal and lateral directions and the up-and-down direction may be operated by the stage of the microscope unit 50. The operation of the nozzle actuator 40 is controlled by a nozzle position control unit (not illustrated) of a bubble forming unit in the information processing device 170.

The sample actuator 41 moves a stage (not illustrated) on which a vessel 25 is mounted. The sample actuator 41 may be operable in any direction of the longitudinal direction, the lateral direction, and the up-and-down direction. The transparent vessel 25 that houses the manipulation target 35 may be mounted on the stage. The vessel 25 may be a liquid-filled culture vessel. The sample actuator 41 may be mounted with one or more vessels and/or tubes, but is not limited thereto. The operation of the sample actuator 41 is controlled by a stage position control unit (not illustrated) of the bubble forming unit in the information processing device 170. Note that the stage may be provided in the manipulation unit 101 or may be provided in the microscope unit 50.

The flow channel imaging camera 42 images the tip portion of the nozzle 49. The flow channel imaging camera 42 may image a bubble formed at the tip portion of the nozzle 49. The captured image may be sent to an image processing unit in the information processing device 170. On the basis of the captured image, a bubble forming unit 200 may instruct the nozzle actuator 40 and/or the sample actuator 41 to move a relative position between the nozzle 49 and the manipulation target 35. Note that instead of the flow channel imaging camera 42, the camera 60, the camera 70, or the like may image the tip portion of the nozzle 49. The present invention is not limited thereto, and in the following description, the flow channel imaging camera 42 may be a microscope-attached camera provided in the microscope unit 50. In the camera provided in the microscope unit 50, the fluorescence image observation light source 1, the transmission image observation light source 10, the light source 16, the light source 17, the light source 45, and the light source 46 may be used as illumination. The light source 16 and the light source 17 may be ring illumination, but are not limited thereto.

The light source 45 and the light source 46 illuminate the nozzle 49 and/or the manipulation target 35. The light source 45 and the light source 46 may be ring illumination, but are not limited thereto.

The pressure generation unit 47 generates a pressure to be loaded on the flow channel 51. The pressure generation unit 47 is connected to one end of the flow channel 51 which is not in contact with the liquid, and supplies a preset gas to the one end. For example, the pressure generation unit 47 may include an actuator that reciprocates a syringe pump and a plunger of the syringe pump. The actuator may supply the gas to the flow channel 51 by pushing the plunger of the syringe pump toward the flow channel 51, and the actuator may take in the gas from the flow channel 51 by drawing the plunger of the syringe pump from the flow channel 51. The pressure generation unit 47 is controlled by the bubble forming unit in the information processing device 170.

The liquid in which the manipulation target 35 is immersed may be a complete medium, a basic medium, or a buffer solution, but is not limited thereto. The complete medium is a medium containing maintenance and growth factors necessary for maintenance and growth of cells. The basic medium is a medium containing only a small portion of proteins, amino acids or salts. The buffer solution is a liquid that maintain a pH and an osmotic pressure suitable for cell survival. As the liquid, the complete medium, the basic medium, and the buffer solution, known one can be used.

The gas may be air. The gas may contain moisture.

The sensor unit 48 includes one or more sensors, and detects the states of the nozzle 49 and the liquid and gas of the nozzle 49. For example, the sensor unit 48 may detect the position, speed, and acceleration of the nozzle 49. The sensor unit 48 may detect the position of the nozzle actuator 40, the pressure generated in the pressure generation unit 47, the position of the plunger of the syringe pump in the pressure generation unit 47, and the like. The sensor unit 48 may detect an environmental temperature and the temperature of the liquid in the vessel 25. The sensor unit 48 may detect the humidity of the environment. In addition, the sensor unit 48 may detect the pH of the liquid in the vessel 25. The sensor unit 48 may detect the temperature and humidity of the gas in the nozzle 49. The sensor unit 48 transmits these pieces of information to the information processing device 170 (for example, the bubble forming unit 200 to be described later). As the sensor used in the sensor unit 48, a known sensor can be used. Note that the sensor unit 48 may be a housing different from the pressure generation unit 47, or may be stored inside the pressure generation unit 47.

The nozzle 49 is an instrument including the flow channel 51 to be described later. The nozzle 49 may have a rod shape or a flat plate shape.

The liquid and gas to be sucked (gas intake) or discharged (gas supply) pass through the flow channel 51. The flow channel 51 is provided inside the nozzle 49 to penetrate the nozzle 49 in a longer direction. The pressure generation unit 47 is connected to the other end side of the flow channel 51.

The flow channel exchange unit 53 is an instrument that houses and discards the nozzle 49. When exchanging the nozzle 49, the flow channel exchange unit 53 may remove the nozzle 49 installed in the nozzle actuator 40 and discard the nozzle in a nozzle discarding unit (not illustrated) of the flow channel exchange unit 53, and instead install, to the nozzle actuator 40, the nozzle 49 housed in a nozzle housing unit (not illustrated) of the flow channel exchange unit 53. The flow channel exchange unit 53 may be omitted, and in that case, the nozzle 49 may be exchanged by the hand of a manipulator.

The liquid storage unit 54 is an instrument that houses a liquid to be supplied to the vessel 25, recovers the liquid from the vessel 25, and discards the liquid. When exchanging the liquid, the liquid storage unit 54 may recover the liquid accommodated in the vessel 25 from the vessel 25, discard the liquid in a liquid discarding unit (not illustrated) of the liquid storage unit 54, and replenish the vessel 25 with the liquid housed in a liquid housing unit (not illustrated) of the liquid storage unit 54. In the exchange of liquids, the same type of liquids may be exchanged with each other. In the exchange of liquids, different types of liquids may be exchanged with each other. The liquid storage unit 54 may be omitted, and in that case, the liquid may be exchanged by the hand of the manipulator.

The sample lid 58 is a lid attached to the vessel 25. The sample lid 58 may be attached to the vessel 25 or may be stored in the sample lid housing unit 59. As necessary, by a sample lid actuator (not illustrated), the sample lid 58 may be taken out from the sample lid housing unit 59 and attached to the vessel 25, and may be removed from the vessel 25 and stored in the sample lid housing unit 59. In this case, the operation of the sample lid actuator may be controlled by a sample lid control unit (not illustrated) of the bubble forming unit 200 in the information processing device 170. The sample lid 58 and the sample lid housing unit 59 may be omitted, and in this case, the sample lid 58 may be attached to the vessel 25 and removed from the vessel 25 by the hand of the manipulator.

The output unit 160 outputs a processing result of the information processing device 170. For example, the output unit 160 outputs an image which has been subject to image processing by the inside (for example, an image processing unit 300 to be described later) of the information processing device 170. For example, the output unit 160 is a monitor connected to the information processing device 170.

The information processing device 170 interchanges commands and data with the microscope unit 50, the camera 60, the camera 70, the manipulation unit 101, the output unit 160, and the input unit 180. For example, the information processing device 170 is connected to the microscope unit 50 and the manipulation unit 101, and controls the microscope unit 50 and the manipulation unit 101.

Specifically, the information processing device 170 switches the combination of the type of the objective lens 6 and/or the type of a filter cube of a fluorescence filter arranged in the optical path of the microscope unit 50. For example, the transmission image observation and the fluorescence image observation are different in both the type of the filter cube and the type of the objective lens 6 arranged in the optical path. In addition, the two types of fluorescence image observation are different only in the type of the filter cube arranged in the optical path. In addition, the transmission image observation and the fluorescence image observation use different light sources (the transmission image observation light source 10 and the fluorescence image observation light source 1, respectively). Therefore, in the inside (for example, an imaging control unit 171 to be described later) of the information processing device 170, one or more of a filter block, the objective lens 6, and the light source may be switched according to whether to perform at least any one or more types of observation among the transmission image observation and one type or two or more types of fluorescence image observation.

When performing the fluorescence image observation, the information processing device 170 turns on the fluorescence image observation light source 1 and turns off the transmission image observation light source 10 in order to enable the optical path of the fluorescence image observation light source 1. When the fluorescence image observation is performed, the light emitted from the fluorescence image observation light source 1 illuminates the manipulation target 35 via the dichroic mirror 2, the optical deflector 3, the relay lens 4, the dichroic mirror 5, and the objective lens 6.

When the manipulation target 35 is fluorescently labeled, the fluorescent substance of the manipulation target 35 is excited and emits fluorescence. The fluorescence emitted from the manipulation target 35 reaches the light receiving surface of the camera 60 via the objective lens 6, the dichroic mirror 5, the relay lens 4, the optical deflector 3, the dichroic mirror 2, the barrier filter 13, the projection lens 14, and the pinhole 15 (when the microscope unit 50 is a confocal microscope). At this time, a fluorescence image of the manipulation target 35 is formed in the camera 60. Note that even when the manipulation target 35 is not fluorescently labeled, the manipulation target 35 can be observed by using the light obtained when the light emitted from the fluorescence image observation light source 1 hits the manipulation target 35 and is reflected from the manipulation target 35.

In order to enable the optical path of the transmission image observation light source 10, the information processing device 170 turns on the transmission image observation light source 10 and turns off the fluorescence image observation light source 1. When performing the transmission image observation, the light emitted from the transmission image observation light source 10 illuminates the manipulation target 35 via the band pass filter 9, the condensing lens 8, and the condenser lens 7. The light transmitted through the manipulation target 35 reaches the light receiving surface of the camera 70 via the objective lens 6, the dichroic mirror 5, the barrier filter 11, and the projection lens 12. At this time, a transmission image of the manipulation target 35 is formed in the camera 70. Note that when it is difficult to see the end portion of the nozzle 49 by fluorescence observation, the transmission image observation may be performed together.

In addition, the information processing device 170 controls a relative position of the nozzle 49 of the manipulation unit 101 and the stage. In addition, in addition to the control of the microscope unit 50 and the manipulation unit 101, the information processing device 170 may receive the manipulation target 35 imaged by the camera 60 and/or the camera 70, and/or an image captured by the flow channel imaging camera 42 of the manipulation unit 101 and perform image processing such as generating one composite image from a plurality of images. The information processing device 170 may perform the control of other operations of the organism manipulation device 100, data processing, and the like as necessary. The configuration of the information processing device 170 will be described later.

The input unit 180 inputs, to the information processing device 170, an instruction, data, and the like from the manipulator. For example, the input unit 180 inputs an instruction regarding selection of a manipulation application for the manipulation target 35 from the manipulator. In addition, the input unit 180 inputs, to the information processing device 170, the operation amount of the nozzle actuator 40 and/or the sample actuator 41 from the manipulator. For example, the input unit 180 is a keyboard or a mouse connected to the organism manipulation device 100.

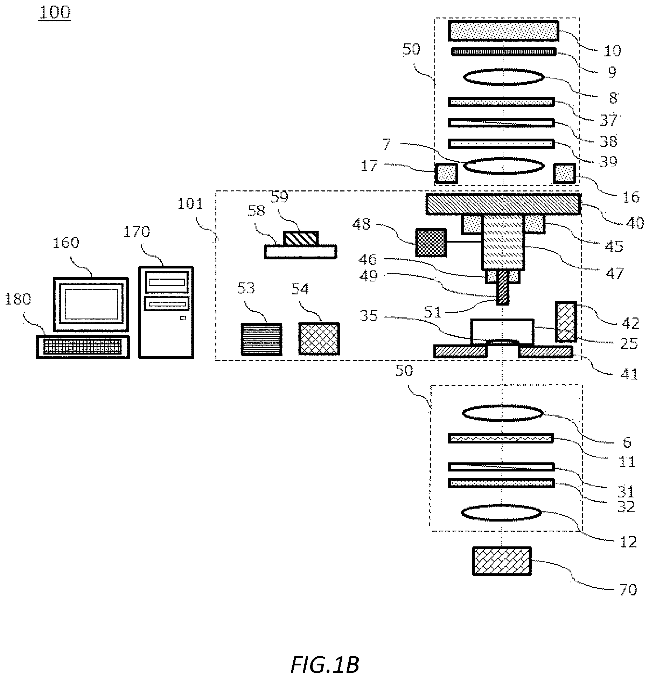

Fig. 1B illustrates another example of the device configuration of the organism manipulation device 100 in the present embodiment. Fig. 1B illustrates the organism manipulation device 100 when the microscope unit 50 is a phase contrast microscope or a differential interference microscope. When the microscope unit 50 is a phase contrast microscope, the microscope unit 50 may include the objective lens 6, the condenser lens 7, the condensing lens 8, the band pass filter 9, the transmission image observation light source 10, the barrier filter 11, the projection lens 12, the light source 16, the light source 17, and a ring aperture 39. When the microscope unit 50 is a differential interference microscope, the microscope unit 50 may include the objective lens 6, the condenser lens 7, the condensing lens 8, the band pass filter 9, the transmission image observation light source 10, the barrier filter 11, the projection lens 12, the light source 16, the light source 17, a Nomarski prism 31, an analyzer (polarizing plate) 32, a polarizer (polarizing plate) 37, and a Nomarski prism 38. In addition, the present invention is not limited thereto, and the microscope unit 50 may have a configuration other than those described above. The description of Fig. 1A may be applied to the configuration of the organism manipulation device 100 other than the microscope unit 50.

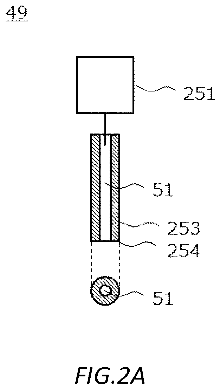

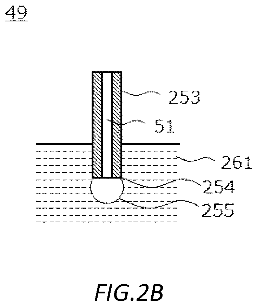

Figs. 2A and 2B are examples of schematic diagrams illustrating a structure of the nozzle 49 in the present embodiment. In Fig. 2A, the nozzle 49 includes a cylindrical portion 253 having the flow channel 51. The cylindrical portion 253 may have a hollow cylindrical shape. In this case, the cross section of the cylindrical portion 253 orthogonal to an axial direction has a circular shape. In addition, the flow channel 51 may be connected to a pump 251 (for example, the syringe pump of the pressure generation unit 47) on one end side. When receiving an instruction from the information processing device 170 (for example, a bubble forming unit 200 to be described later), the pump 251 adjusts the pressure and/or volume of the bubble by adjusting the amount of the gas supplied to the flow channel 51 or taken in from the flow channel 51.

In Fig. 2B, when an end portion 254 of the cylindrical portion 253 on the side not connected to the pump (not illustrated: for example, the syringe pump of the pressure generation unit 47) is arranged in the liquid 261, the pump can form a bubble at the end portion 254 by supplying gas to the flow channel 51. In this case, the gas-liquid interface 255 is formed at a boundary between the gas of the bubble and the liquid 261. Note that the shape of the bubble is not limited to a spherical shape, and may be deformed according to the shape of the end portion 254. Herein, when gas is retained at the end portion 254 of the flow channel 51, the gas-liquid interface 255 is formed at the end portion 254 of the flow channel 51, but when both gas and liquid are present inside the flow channel 51, the gas-liquid interface 255 can be formed at an interface between the both inside the flow channel 51.

When the gas-liquid interface 255 comes into contact with the organism adhered on the solid phase such as the inner bottom surface of the vessel 25 in the liquid 261, the gas-liquid interface 255 is moved, so that the gas-liquid interface 225 applies a force to the organism, and the organism can be separated from the solid phase and attached to the gas-liquid interface 255. The movement of the gas-liquid interface 255 may be performed by the nozzle actuator 40 moving the nozzle 49 in which the bubble is formed, may be performed by moving the liquid, or may be performed by changing the volume of the bubble. Herein, the solid phase may be a surface on which adherent cells can be adhered and cultured. For example, the solid phase may be a glass; resin such as polystyrene; a metal; a surface coated with one or more types of extracellular matrix components selected from collagen, fibronectin, laminin, polylysine, or the like; a surface coated with various types of polymers (as an example, a polymer capable of controlling hydrophilicity and adsorption to cells), and the like, but is not limited thereto. Note that in the present embodiment, the gas-liquid interface 255 is formed by the interface between the gas and the liquid, but is not limited thereto, and may be changed according to a phase or a substance in contact with the interface. Details of a method of separating the organism from the solid phase will be described later.

The opening area of the flow channel 51 at the end portion 254 is not particularly limited as long as the opening area has a size that allows the manipulation of the organism. For example, the opening area may be larger than the adhesion area per organism. The shape of the end portion 254 is not particularly limited. In addition, the inner diameter of the flow channel 51 may be the same over the entire length of the cylindrical portion 253.

In addition, the flow channel 51 may take the gas-liquid interface 225 into the flow channel 51 by the pump 251 taking in the gas in the bubble to which the organism is attached, and further recover the organism. Alternatively, separately from the flow channel 51, the nozzle 49 may further include another flow channel for recovering the organism.

In the embodiment of Figs. 2A and 2B, since only one flow channel 51 is formed in the nozzle 49 and only one pump 251 is connected to the flow channel 51, a considerably simple minimum configuration is obtained, and the maintenance and cost of the organism manipulation device 100 can be reduced.

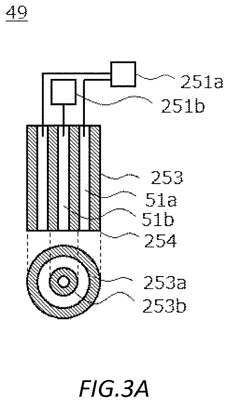

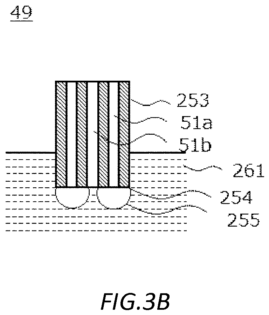

Figs. 3A and 3B are examples of schematic diagrams illustrating the structure of the nozzle 49 in another embodiment. In the examples of Figs. 2A and 2B, a case is illustrated in which the flow channel for forming the bubble and the flow channel for recovering the organism are the same, but in the examples of Figs. 3A and 3B, a case is illustrated in which the flow channel for forming the bubble and the flow channel for recovering the organism are different.

In Fig. 3A, the cylindrical portion 253 of the nozzle 49 has a double structure of an outer cylinder 253a and an inner cylinder 253b. A space between the outer cylinder 253a and the inner cylinder 253b is a first flow channel 51a through which gas flows, and the inside of the inner cylinder 253b is a second flow channel 51b. For example, the first flow channel 51a may be a gas supply flow channel, and the second flow channel 51b may be a gas recovery flow channel.

In addition, the first flow channel 51a and the second flow channel 51b of the nozzle 49 may be connected to a first pump 251a and a second pump 251b, respectively, on one end side. For example, the pressure generation unit 47 may include the first pump 251a and the second pump 251b as syringe pumps, and each may be controlled by a separate actuator. Each of the first pump 251a and the second pump 251b adjusts the pressure and/or volume of the bubble by the actuator, which has received an instruction from the bubble forming unit 200, adjusting the amount of gas supplied to or taken into the first flow channel 51a and the second flow channel 51b. The cross section of the cylindrical portion 253 orthogonal to the axial direction has a donut shape in the first flow channel 51a and a circular shape in the second flow channel 51b.

In Fig. 3B, when the end portion 254 of the cylindrical portion 253 on the side not connected to the first pump 251a and the second pump 251b is arranged in the liquid 261, a bubble can be formed at the end portion 254 by the first pump 251a supplying gas to the first flow channel 51a. In this case, the gas-liquid interface 255 is formed at a boundary between the gas of the bubble and the liquid 261.

When the gas-liquid interface 255 comes into contact with the organism adhered on the solid phase in the liquid 261, the gas-liquid interface 255 is moved so that the organism can be separated from the solid phase, and the organism can be attached to the gas-liquid interface 255. The second pump 251b may take in the bubble, to which the organism is attached, via the second flow channel 51b to take the gas-liquid interface 225 into the flow channel 51 and recover the organism.

In the above embodiment, the first pump 251a forms the bubble by supplying gas to the first flow channel 51a, and the second pump 251b recovers the organism by taking in gas in the second flow channel 51b, but the second pump 251b may form the bubble by supplying gas to the second flow channel 51b, and the first pump 251a may recover the organism by taking in gas in the first flow channel 51a. In addition, the first pump 251a and the second pump 251b may be the same syringe pump provided in the pressure generation unit 47. One of the first pump 251a and the second pump 251b may be omitted.

In the embodiment of Figs. 3A and 3B, forming the bubble in one flow channel to attach the organism to the gas-liquid interface 255 and recovering the organism attached in another flow channel can be performed simultaneously, so that there is an effect that the time for recovering cells is shortened. Note that in Figs. 3A and 3B, the embodiment is illustrated in which the cross section of the cylindrical portion 253 orthogonal to the axial direction has the donut shape in the first flow channel 51a and the circular shape in the second flow channel 51b, but the shape of the cross section is not limited to the donut shape or the circular shape, and the attachment and recovery of the organism can be performed simultaneously as long as two flow channels are provided.

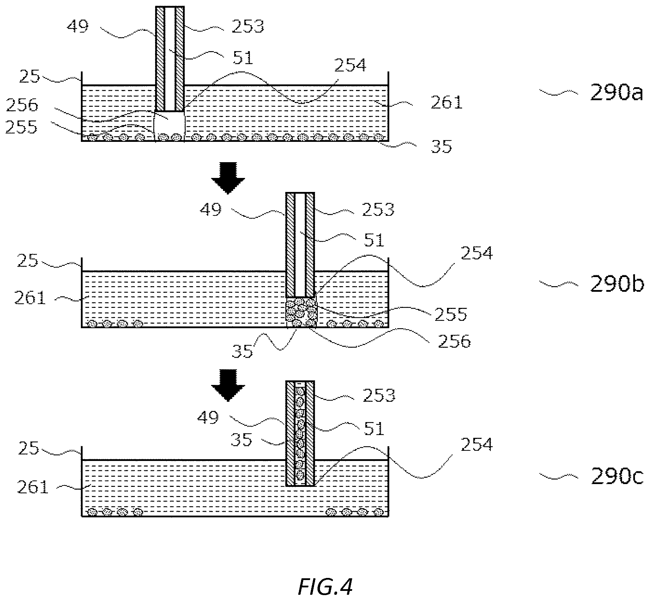

Fig. 4 is a schematic diagram illustrating an example of a method of recovering the manipulation target 35 in the present embodiment. In 290a, the manipulation target 35 is cultured in the solid phase on the inner bottom surface of the vessel 25. The manipulation target 35 may be cultured in the liquid 261. For example, the manipulation target 35 is an adherent cell. For example, the liquid may be a complete medium.

In 290a, the pump 251 supplies gas to the flow channel 51 of the nozzle 49 to form a bubble 256 at the end portion 254 of the nozzle 49. By bringing the bubble 256 into contact with the manipulation target 35, the gas-liquid interface 255 between the gas and the liquid 261 comes into contact with the manipulation target 35. In this case, the pump 251 regulates the supply and intake of the gas to maintain the formed bubble 256. Accordingly, it is possible to more easily perform the manipulation of the manipulation target 35 by the bubble 256.

Next, in 290b, the nozzle actuator 40 moves the nozzle 49 along the surface of the solid phase while keeping the bubble 256 in contact with the manipulation target 35. In Fig. 4, an aspect is illustrated in which the nozzle actuator 40 moves the nozzle 49 from left to right, but the direction in which the nozzle 49 is moved is not limited as long as the direction is parallel to the surface of the solid phase. When the nozzle actuator 40 moves the nozzle 49, the manipulation target 35 can be separated from the solid phase. At this time, the separated manipulation target 35 is attached to the gas-liquid interface 255 of the bubble 256. The bubble forming unit 200 controls the pump 251 to adjust the pressure and/or volume of the gas to be supplied or taken in and change the magnitude of the bubble 256, so that the manipulation target 35 within a desired range can be separated. Note that instead of moving the nozzle 49 along the surface of the solid phase, the stage may be moved.

Next, in 290c, the pump 251 may take in the gas in the flow channel 51 to recover the manipulation target 35 attached to the gas-liquid interface 255. In this manner, the manipulation target 35 can be selectively separated from the solid phase and recovered by using the bubble 256 formed in the nozzle 49.

When the manipulation target 35 is an adherent cell strongly adhered on the solid phase, the adhesion of the adherent cell may be alleviated in advance before the method of Fig. 4 is executed. The alleviation of the adhesion of the adherent cell can be performed by using a known method as described later.

In Fig. 4, an example is illustrated in which the nozzle 49 is moved to move the gas-liquid interface 255, and the cells are separated and recovered, but the movement of the gas-liquid interface 255 is not limited to the above example. For example, the volume of the bubble 256 may be increased after the bubble 256 is brought into contact with the manipulation target 35. In this case, a contact surface between the bubble 256 and the solid phase expands, and the manipulation target can be selectively separated from the solid phase and recovered. For example, after the bubble 256 is brought into contact with the manipulation target 35, the nozzle actuator 40 may move the nozzle 49 to approach the solid phase. Also in this case, when the bubble 256 is pressed against the solid phase, the contact surface between the bubble 256 and the solid phase expands, and the manipulation target can be selectively separated from the solid phase and recovered.

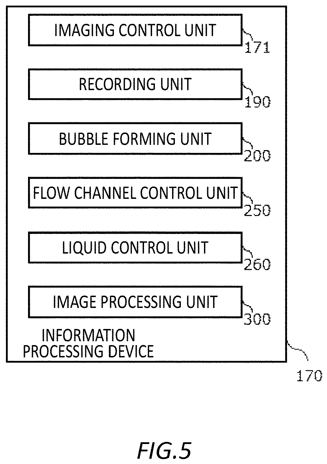

Fig. 5 illustrates an example of a specific configuration of the information processing device 170 in the present embodiment. The information processing device 170 includes the imaging control unit 171, the recording unit 190, the bubble forming unit 200, a flow channel control unit 250, a liquid control unit 260, and an image processing unit 300.

The imaging control unit 171 controls the fluorescence image observation light source 1, the objective lens 6, the fluorescence filter, the transmission image observation light source 10, the flow channel imaging camera 42, the light source 45, the light source 46, the camera 60, the camera 70, and the like described in Figs. 1A and 1B. For example, when the imaging condition of the manipulation target 35 is input to the input unit 180, according to the input imaging condition, the imaging control unit 171 performs necessary regulation for each imaging in the switching of the camera, the switching of the type of the objective lens 6 in the microscope unit 50, the switching of the light source, the switching of the type of the fluorescence filter, the position of the stage, and the height of the objective lens 6. After the imaging control unit 171 performs the necessary regulation, one or more cameras among the flow channel imaging camera 42, the camera 60, and the camera 70 image the manipulation target 35 or the nozzle 49, and generate an image of the manipulation target 35 or the nozzle 49. The one or more cameras transmit data of the generated image to the image processing unit 300. In addition, the generated image data may also be recorded in the recording unit 190 and/or output to the output unit 160.

The recording unit 190 may be a memory, an internal hard disk drive, or an external recording medium, but is not limited thereto. The information processing device 170 includes a central processing unit (CPU), and the CPU executes a computer program recorded in the recording unit 190 to realize the information processing device 170 or the like.

The bubble forming unit 200 controls the pressure and volume of the bubble formed in the flow channel 51, the gas supply, the gas intake, the movement of the nozzle 49, and the movement of the stage. The bubble forming unit 200 may include all or some of a nozzle position control unit, a stage position control unit, a volume control unit, a gas supply control unit, and a gas intake control unit.

The nozzle position control unit controls the nozzle actuator 40 to control the operation of the nozzle 49, the air flow in the bubble accompanying the operation of the nozzle 49, and the movement of the gas-liquid interface 255 accompanying the operation of the nozzle 49. In addition, the nozzle position control unit receives the position information of the nozzle 49 from the sensor unit 48 or the nozzle actuator 40.

The stage position control unit controls the sample actuator 41 to control the operation of the stage on which the vessel 25 for accommodating the manipulation target 35 is mounted, the air flow in the bubble accompanying the operation of the stage, and the movement of the gas-liquid interface 255 accompanying the operation of the stage. The stage position control unit receives the position information of the stage and the manipulation target 35 from the sensor unit 48 or the sample actuator 41.

The volume control unit controls the actuator of the pressure generation unit 47 to supply the gas from the syringe pump or take in the gas into the syringe pump, thereby controlling the pressure and/or volume of the bubble formed in the flow channel 51. In addition, the volume control unit receives the information on the pressure and/or volume of the bubble from the nozzle actuator 40, the pressure generation unit 47, or the sensor unit 48.

In addition, when the nozzle 49 includes a gas supply flow channel for supplying (gas supply) gas and a gas recovery flow channel for recovering (gas intake) gas, the volume control unit or the gas supply control unit controls the first pump 251a connected to the gas supply flow channel, thereby controlling the volume of the gas to be supplied to the gas supply flow channel. The volume control unit or the gas supply control unit receives, from the nozzle actuator 40, the pressure generation unit 47, or the sensor unit 48, the information on the amount of the gas to be supplied to the gas supply flow channel.

In addition, when the nozzle 49 includes a gas supply flow channel for supplying (gas supply) gas and a gas recovery flow channel for recovering (gas intake) gas, the volume control unit or the gas intake control unit controls the second pump 251b connected to the gas recovery flow channel, thereby controlling the amount (volume) of the gas to be taken in from the gas recovery flow channel. The volume control unit or the gas intake control unit receives, from the nozzle actuator 40, the pressure generation unit 47, or the sensor unit 48, the information on the amount of the gas to be taken in from the gas recovery flow channel.

The flow channel control unit 250 controls housing, installation, and discarding of the nozzle 49. The flow channel control unit 250 receives, from the input unit 180, a manipulation instruction regarding the installation and discarding of the nozzle 49 from the manipulator. In accordance with the received instruction, the flow channel control unit 250 instructs the flow channel exchange unit 53 to take out the nozzle 49 from a flow channel housing unit of the flow channel exchange unit 53 and install the nozzle 49 in the nozzle actuator 40 or to remove the nozzle 49 installed in the nozzle actuator 40 and discard the nozzle in the flow channel discarding unit of the flow channel exchange unit 53.

The liquid control unit 260 controls housing, replenishment, and discarding of the liquid. The liquid control unit 260 receives, from the input unit 180, a manipulation instruction regarding the replenishment and discarding of the liquid from the manipulator. In accordance with the received instruction, the liquid control unit 260 instructs the liquid storage unit 54 to replenish the vessel 25 with the liquid housed in the liquid housing unit of the liquid storage unit 54 or to recover the liquid accommodated in the vessel 25 from the vessel 25 and discard the liquid in the liquid discarding unit of the liquid storage unit 54.

The image processing unit 300 receives the images captured by the flow channel imaging camera 42, the camera 60, and the camera 70 from these cameras. The image processing unit 300 may use a plurality of images among the received images to perform combination into one composite image. For example, the image processing unit 300 may combine the fluorescence image captured by the camera 60 and the transmission image captured by the camera 70 to generate a composite image. The image processing unit 300 may record the images received from these cameras and/or the composite image in the recording unit 190 and/or output them to the output unit 160.

An energy control unit controls a difference (E1 - E2) between interface free energy E1 at the interface between the gas and the organism and interface free energy E2 at the interface between the gas and the liquid. The value of the difference (E1 - E2) may be a positive value, 0, or a negative value. The smaller the value of the difference (E1 - E2) is, the more organisms attached to the bubble. Herein, since the value of the interface free energy E1 is constant, there is little room for the energy control unit to control. Therefore, by the energy control unit controlling the interface free energy E2 at the interface between the gas and the liquid, the value of the difference (E1 - E2) can be controlled to a preset value. Details will be described later.

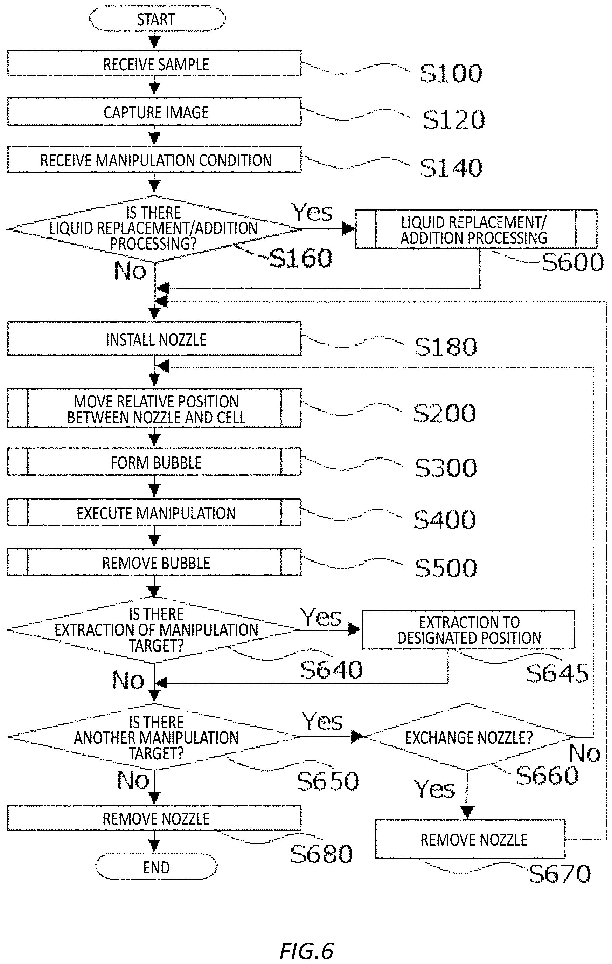

Fig. 6 is an example of a flow of a method of manipulating the organism in the present embodiment. The organism to be the manipulation target 35 of the present embodiment can be manipulated by performing processing of S100 to S680 of Fig. 6. Note that, for convenience of description, the processing of S100 to the processing of S680 will be described in order; however, at least some processing may be executed in parallel, and each step may be interchanged and executed within a range not deviating from the spirit of the present invention.

First, in S100, the sample actuator 41 receives an organism to be the manipulation target 35. For example, in S100, the sample actuator 41 mounts the vessel 25 accommodating the manipulation target 35 together with the liquid on the stage. In order to manipulate the manipulation target 35, the lid of the vessel 25 may be removed. The lid may be exchanged by an actuator for exchanging the lid, or may be exchanged by the hand of the manipulator. After the sample actuator 41 receives the organism to be the manipulation target 35, the information processing device 170 advances the processing to S120.

Next, in S120, the camera 60 or the camera 70 images a wide range of observation field including the manipulation target 35 to generate an image. The imaging control unit 171 sets an observation method to low magnification transmission image capturing and instructs the camera 70 to image the observation field. The imaging control unit 171 may set the observation method to fluorescence image capturing and instruct the camera 60 to capture an image of the observation field. The imaging control unit 171 may receive, via the input unit 180, the input of imaging conditions from the manipulator. The camera 60 or the camera 70 captures an image of the observation field. The image processing unit 300 may record the captured image in the recording unit 190 and/or output the captured image to the output unit 160. After the camera 60 or the camera 70 images the observation field, the imaging control unit 171 advances the processing to S140.

Next, in S140, the information processing device 170 receives, via the input unit 180, an input regarding the manipulation target 35 and the type of the manipulation from the manipulator. The manipulation target 35 may be a single cell, a population (colony) of cells, cytoplasm and/or a cell membrane of a cell, or a spheroid, but is not limited thereto. The type of the manipulation may be the recovery of the manipulation target 35, the removal of the manipulation target 35, the retention of the manipulation target 35, or the compression of the manipulation target 35, but is not limited thereto.

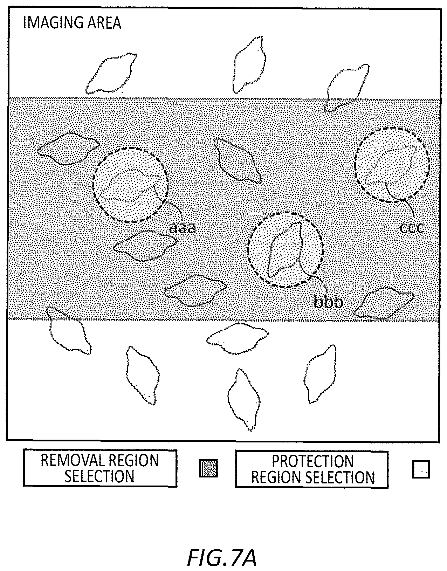

Fig. 7A is an example of a graphical user interface (GUI) image displayed on the output unit 160 and obtained by the camera 60 or the camera 70 imaging the observation field. In Fig. 7A, cells aaa, bbb, and ccc to be manipulation targets are designated as the manipulation target 35 via the input unit 180. As illustrated in Fig. 7A, the organism to be the manipulation target 35 is arbitrarily designated via the input unit 180. As illustrated in Fig. 7A, a removal region and/or a protection region may be provided in the observation field such that the removal region and/or the protection region is selected in the GUI image. By providing the removal region, it is possible to reduce a risk of recovering cells other than the cells to be recovered. In addition, by providing the protection region, it is possible to reduce a risk of erroneously removing recovered cells when removing the cells in the removal region.

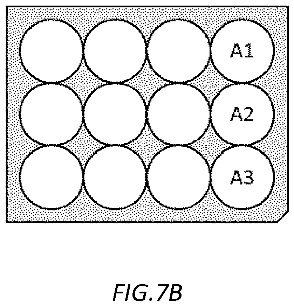

Fig. 7B is an example of a GUI image which is displayed on the output unit 160 and in which the recovery destinations and the movement destinations of the cells aaa, bbb, and ccc to be the manipulation target 35 are designated as A1, A2, and A3 of twelve hole plates, respectively. As illustrated in Fig. 7B, the movement destination is arbitrarily designated via the input unit 180. For example, the movement destination may be the same plate, a different plate, a petri dish, a micro test tube, a PCR tube, or a conical tube.

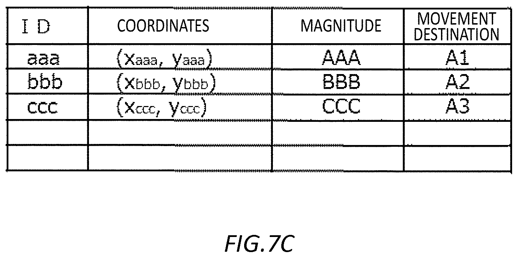

Fig. 7C is an example of a GUI image which is displayed on the output unit 160 and shows a table listing the ID number of the cell to be the manipulation target 35, the xy coordinates of the manipulation target 35 on the sample actuator 41, the magnitude of the manipulation target 35, and the movement destination of the manipulation target 35. In the table illustrated in Fig. 7C, a case is shown in which the ID number, the xy coordinate, the size, and the movement destination are set as items, but the items to be displayed are not limited thereto. In this manner, the image processing unit 300 may output the table to the output unit 160 by designating the manipulation target 35, the movement destination, and the like via the input unit 180.

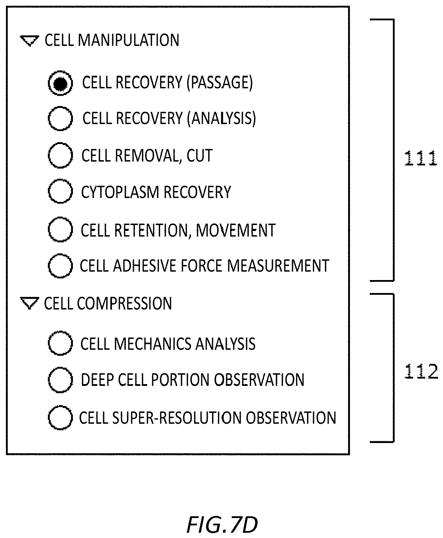

Fig. 7D is an example of a GUI screen, which is displayed on the output unit 160, for selecting the type of the manipulation of the manipulation target 35. The input unit 180 receives, from the manipulator, an instruction as to what manipulation is desired to be performed on the manipulation target 35, and inputs the instruction to the information processing device 170. For example, as shown in the display region 111, the manipulation on the cell may be passage or retention or movement of the cell, but is not limited thereto. For example, as shown in the display region 112, the manipulation on the cell may be to compress the cell and observe the deep portion of the cell, but is not limited thereto. Fig. 7D illustrates an example in which the type of the manipulation is selected by a radio button, but a selection method is not limited to the radio button.

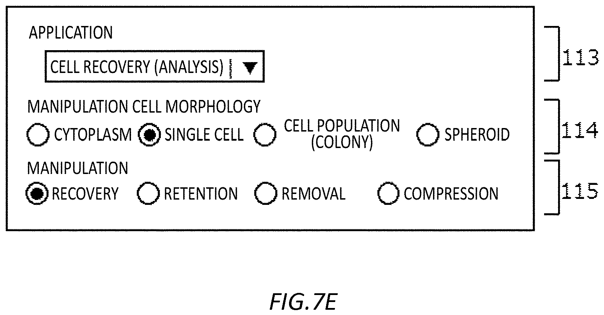

Fig. 7E is another example of the GUI screen, which is displayed on the output unit 160, for selecting the type of the manipulation of the manipulation target 35. For example, as shown in the display region 113, the type of the manipulation on the cell may be selected by a pull-down menu. For example, as shown in the display region 113, the display region 114, and the display region 115, the pull-down menu and the radio button may be used together for selection of the type of the manipulation.

The input unit 180 may transmit, to the information processing device 170, the instruction input from the manipulator on the basis of the screen illustrated in Figs. 7A to 7E. After the information processing device 170 receives the instruction, the imaging control unit 171 advances the processing to S160.

In S160, when the manipulation target 35 is an adherent cell which strongly adheres on the solid phase, a step of alleviating the adhesion of the adherent cell in advance may be additionally performed. In this case, in a step of receiving the manipulation condition of S140, the information processing device 170 may receive an input as to whether to perform processing of alleviating the adhesion.

The alleviation of the adhesion of the adherent cell can be performed by using a known method. For example, the alleviation of the adhesion of the adherent cell may be performed by removing a liquid (for example, medium), washing with a buffer solution, and then processing the adherent cell with an adhesion alleviation solution. For example, the adhesion alleviation solution may be a protease solution, a solution containing no metal ion, or a chelating agent solution. As an example, the adhesion alleviation solution is a trypsin-EDTA solution. The adhesion of the adherent cells may be alleviated by the liquid control unit 260 or may be alleviated by the hand of the manipulator. After processing the adherent cells with the adhesion alleviation solution to weaken an adhesive force, the processing may advance to S160. Note that the step is not limited to the alleviation of the adhesion of the adherent cell, and when the step is performed by the hand of the manipulator, the step may be started again from the sample reception step of S100. In addition, instead of the processing with the adhesion alleviation solution, the adhesive force may be weakened by using a substrate which alleviates the adhesion. For example, the substrate which alleviates the adhesion may be one that alleviates the adhesion in response to temperature or light irradiation.

Next, in S160, the information processing device 170 receives, via the input unit 180, an input regarding liquid replacement or addition processing from the manipulator. For example, when it is desired to regulate an attachment force between the organism to be the manipulation target 35 and the bubble, the information processing device 170 may receive an input to perform the liquid replacement or addition processing. When the information processing device 170 receives an instruction to perform the liquid replacement or addition processing, the information processing device 170 may advance the processing to S600. When the information processing device 170 receives an instruction not to perform the liquid replacement or addition processing, the information processing device 170 may advance the processing to S180.

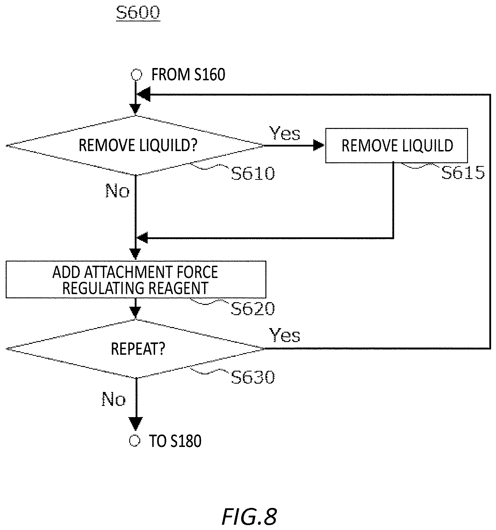

In S600, the liquid control unit 260 replaces the liquid in the vessel 25 accommodating the manipulation target 35 or adds another liquid to the liquid in the vessel 25. In S600, the step of performing the liquid replacement or addition processing includes steps S610 to S630 as illustrated in Fig. 8.

First, in S610, the information processing device 170 receives, via the input unit 180, an input regarding whether to remove the liquid from the manipulator. When the information processing device 170 receives an instruction to remove the liquid, the processing advances to S615. When the information processing device 170 receives an instruction not to remove the liquid, the processing advances to S620.

In S615, the liquid control unit 260 controls the liquid storage unit 54 to remove the liquid. For example, the liquid control unit 260 sends an instruction to the liquid storage unit 54 to recover, from the vessel 25, a preset amount of liquid accommodated in the vessel 25 and discard the liquid in the liquid discarding unit of the liquid storage unit 54. At this time, the liquid storage unit 54 may recover the entire amount of the liquid and discard the liquid. Alternatively, the liquid storage unit 54 may recover a part (for example, half amount) of the liquid and discard the liquid. After the liquid storage unit 54 recovers the liquid, the liquid control unit 260 advances the processing to S620.

In S620, the liquid control unit 260 sends an instruction to the liquid storage unit 54 to add an attachment force regulating reagent to the vessel 25. The attachment force regulating reagent is a reagent for regulating the attachment force between the organism and the bubble. For example, the attachment force regulating reagent may change the concentration of inorganic salts and/or the concentration of amphipathic substances in the liquid. As an example, the attachment force regulating reagent may be a buffer solution containing or not containing at least one of calcium ions or magnesium ions, may be a basal medium, may be a complete medium, or may be a chelating agent. At this time, the liquid storage unit 54 replenishes the vessel 25 with the attachment force regulating reagent housed in the liquid housing unit of the liquid storage unit 54.

In the above example, an example has been described in which the vessel 25 is replenished with the attachment force regulating reagent, but instead of adding the attachment force regulating reagent to the vessel 25, the attachment force between the organism and the bubble may be regulated by attaching an inorganic salt, an amphipathic substance, or the like contained in the liquid to a filter or the like to remove the inorganic salt, the amphipathic substance, or the like.

In S630, the information processing device 170 receives, via the input unit 180, an instruction regarding whether to repeat the above-described series of manipulations from the manipulator. When the information processing device 170 receives an instruction to repeat the series of manipulations, the information processing device 170 advances the processing to S610, and the liquid control unit 260 sends an instruction to the liquid storage unit 54 to remove the liquid in the vessel 25. When the information processing device 170 receives an instruction not to repeat the series of manipulations, the information processing device 170 advances the processing to S180. Note that the steps and sub-steps of S600 may be performed by the hand of the manipulator, or in this case, the processing may start again from the sample reception step of S100.

In S180, the nozzle actuator 40 installs the nozzle 49. For example, the information processing device 170 receives, from the manipulator via the input unit 180, an instruction to install the nozzle 49 to the nozzle actuator 40. According to the instruction, the flow channel control unit 250 takes out the nozzle 49 from the flow channel housing unit of the flow channel exchange unit 53, and sends an instruction to the flow channel exchange unit 53 to install the nozzle 49 to the nozzle actuator 40. At this time, the suitable nozzle 49 may be selected according to the magnitude of the manipulation target 35, the type of manipulation, and the like. The selection of the nozzle 49 may be designated by the manipulator via the input unit 180, or may be automatically designated by the flow channel control unit 250. After the nozzle actuator 40 installs the nozzle 49, the flow channel control unit 250 advances the processing to S200. Note that when the nozzle 49 does not need to be installed in the nozzle actuator 40, for example, in a state where the nozzle actuator 40 is installed in the nozzle actuator 40 in advance or a state where the nozzle 49 and the nozzle 49 are integrally formed, the step of S180 may be omitted.

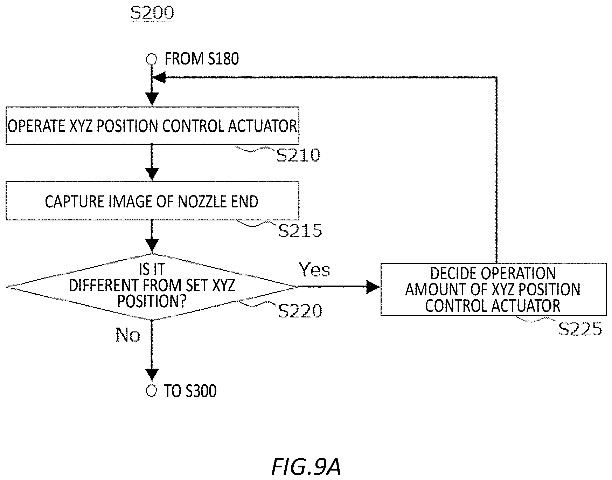

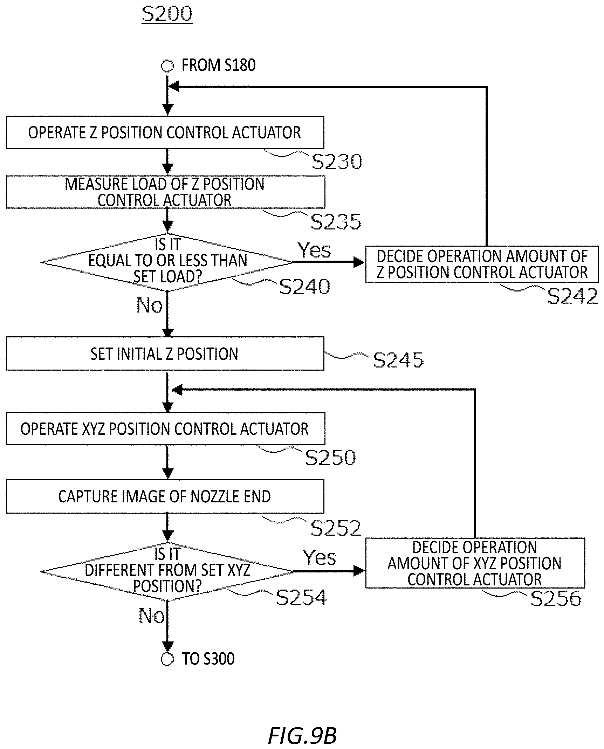

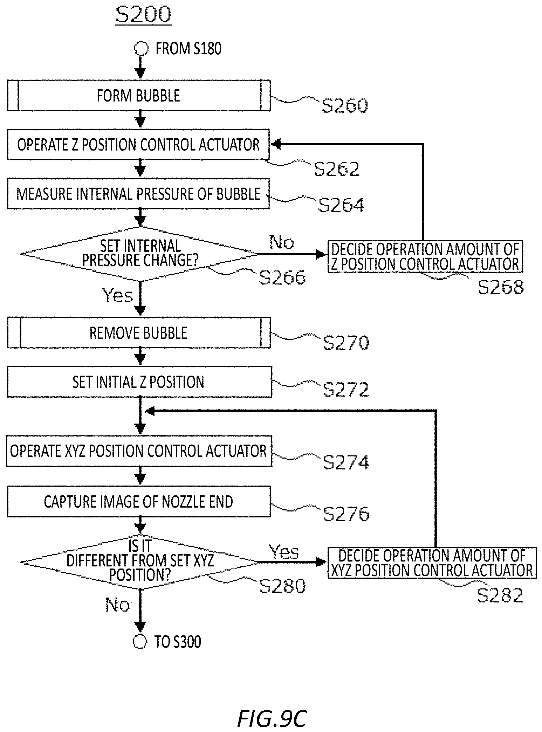

Next, in S200, the nozzle actuator 40 moves the relative position between the nozzle 49 and the manipulation target 35. For example, the bubble forming unit 200 sends an instruction to the nozzle actuator 40 to move the relative position between the nozzle 49 and the manipulation target 35. In S200, a step of moving the relative position includes steps of S210 to S225, as illustrated in Fig. 9A, steps of S230 to S256, as illustrated in Fig. 9B, or steps of S260 to S282, as illustrated in Fig. 9C.

Fig. 9A is an example of a flow in which the relative position between the nozzle 49 and the manipulation target 35 is moved on the basis of an image obtained by imaging the position of the end portion 254 of the nozzle 49.

First, in S210, the bubble forming unit 200 sends an instruction to the nozzle actuator 40 to operate the nozzle 49 to a preset position. The nozzle actuator 40 may be an actuator which controls an xyz position. Herein, a z position may be a position in a vertical direction (also referred to as a direction along gravity, an up-and-down direction, and a z direction), an x position may be a position in an arbitrary x direction (also referred to as a longitudinal direction) perpendicular to the z direction, and a y position may be a position in the x direction and a y direction (also referred to as a lateral direction) perpendicular to the z direction.

In the position of the nozzle 49, the z position may be set by first focusing the camera 60 and/or the camera 70 on the bottom surface of the vessel 25, then moving the focus of the camera 60 and/or the camera 70 upward by an arbitrary distance, and then the nozzle actuator 40 aligning the tip of the nozzle 49 with the focus of the camera 60 and/or the camera 70. For example, the arbitrary distance may be less than or equal to the radius of the bubble formed at the end portion 254 of the nozzle 49. When a distance between the tip of the nozzle 49 and the bottom surface of the vessel 25 is equal to or less than the radius of the bubble to be formed, the bubble comes into contact with the bottom surface, so that the organism positioned on the bottom surface can be manipulated by using the interface of the bubble. In this case, on the basis of an image obtained by imaging the manipulation target 35 by using the camera 60 and/or the camera 70, the xy position of the nozzle 49 can be set by using the nozzle actuator 40 or the sample actuator 41. Note that the position of the nozzle 49 may be set by using the flow channel imaging camera 42 instead of the camera 60 and/or the camera 70 or together with the camera 60 and/or the camera 70.

In addition, in the regulation of the z position of the nozzle 49, the tip of the nozzle 49 and the bottom surface of the vessel 25 may be imaged from the lateral direction of the nozzle 49 by using the flow channel imaging camera 42 to set the z position. Furthermore, the shape of the bubble, the liquid amount in the flow channel 51, or the like may be confirmed by imaging from the lateral direction of the nozzle 49 by using the flow channel imaging camera 42. After the nozzle actuator 40 moves the nozzle 49 to the preset position, the bubble forming unit 200 advances the processing to S215.

Next, in S215, the flow channel imaging camera 42 captures an image of the end portion 254 of the nozzle 49.

The flow channel imaging camera 42 sends the captured image to the image processing unit 300. The image processing unit 300 may record the image in the recording unit 190 and/or output the image to the bubble forming unit 200.

Next, in S220, on the basis of the captured image of the end portion 254 of the nozzle 49, the bubble forming unit 200 determines whether the position of the nozzle 49 is different from the preset position. For example, the bubble forming unit 200 calculates a difference in position from the captured image of the end portion 254 of the nozzle 49 and the image of the end portion 254 of the nozzle 49 at a preset set xyz position (that is, an initial position), and determines that the position of the nozzle 49 is different from the initial position when the difference is equal to or larger than a threshold.

When it is determined that the position of the nozzle 49 is different from the initial position, the bubble forming unit 200 advances the processing to S225, and otherwise, advances the processing to S300.

In S225, the bubble forming unit 200 decides the operation amount of the nozzle actuator 40. For example, the bubble forming unit 200 decides the operation amount of the nozzle actuator 40 in order to operate the nozzle 49 to the preset xyz position (that is, the initial position), and sends an instruction to the nozzle actuator 40 to operate by the decided operation amount. For example, the bubble forming unit 200 may decide the operation amount of an amount corresponding to the magnitude of the difference calculated in S220. The nozzle actuator 40 receives the instruction, and the processing advances to S210. In the second and subsequent S210, the bubble forming unit 200 sends an instruction to the nozzle actuator 40 to perform an operation by the amount corresponding to the operation amount.

Fig. 9B is an example of a flow of moving the relative position between the nozzle 49 and the manipulation target 35 on the basis of the load sensed by the nozzle actuator 40.

First, in S230, the bubble forming unit 200 sends an instruction to the nozzle actuator 40 to operate the nozzle 49 to the preset position. The nozzle actuator 40 may be an actuator which controls the z position. In this case, the z position is controlled on the basis of the value of the load sensed by the nozzle actuator 40, and contact or proximity information. The nozzle 49 may be positioned above a region where no organism is present on the bottom surface of the vessel 25. After the nozzle actuator 40 moves the nozzle 49 to the preset position, the bubble forming unit 200 advances the processing to S235.

Next, in S235, the sensor unit 48 measures the applied load and the contact or proximity information of the nozzle actuator 40, and sends the measured value to the bubble forming unit 200. As an example of the load detection, when the nozzle 49 reaches the bottom portion of the vessel 25, the load sensed by the nozzle actuator 40 rapidly increases. Therefore, by measuring the value of the load sensed by the nozzle actuator 40, the bubble forming unit 200 can determine whether the nozzle 49 has reached the bottom portion of the vessel 25. In addition, as another example of the load detection, the sensor unit 48 may sense the load while the nozzle actuator 40 moves the nozzle 49 downward. Note that instead of the sensor unit 48, the nozzle actuator 40 may send, to the bubble forming unit 200, the value of the load to be sensed.

Next, in S240, the bubble forming unit 200 determines whether the value of the measured load is equal to or less than a set load. When the value of the measured load is equal to or less than the set load, the bubble forming unit 200 advances the processing to S242, and otherwise, advances the processing to S245. As described above, a difference between the load set by the bubble forming unit 200 and the measured load is calculated, and when the value of the difference is equal to or larger than a threshold, it is determined that the nozzle 49 has not reached the bottom portion of the vessel 25.

In S242, the bubble forming unit 200 decides the operation amount of the nozzle actuator 40. For example, the bubble forming unit 200 decides the operation amount of the nozzle actuator 40 in order to operate the nozzle 49 to the preset position, and sends an instruction to the nozzle actuator 40 to operate by the decided operation amount. For example, the bubble forming unit 200 may decide the operation amount of an amount corresponding to the magnitude of the difference calculated in S240. The nozzle actuator 40 receives the instruction, and the processing advances to S230. In the second and subsequent S230, the bubble forming unit 200 sends an instruction to the nozzle actuator 40 to perform an operation by the amount corresponding to the operation amount.

In S245, the bubble forming unit 200 sets the initial z position of the nozzle 49. For example, the bubble forming unit 200 may set the z position of the nozzle 49 as the initial z position without moving the z position after the last S230. Alternatively, the bubble forming unit 200 may move the nozzle 49 in the z direction by an arbitrary distance set in advance from the bottom surface of the vessel 25 and set the position thereof as the initial z position. As a result, the initial z position of the nozzle 49 is positioned above the bottom surface by the arbitrary distance. For example, the arbitrary distance may be less than or equal to the radius of the bubble formed at the end portion 254 of the nozzle 49. After the bubble forming unit 200 sets the initial z position of the nozzle 49, the bubble forming unit 200 advances the processing to S250.

Next, in S250, the bubble forming unit 200 sends an instruction to the nozzle actuator 40 to operate the nozzle 49 to the preset set xyz position (that is, the initial position). The nozzle 49 may move on a xy plane. In this case, the control of the z position is performed on the basis of the value of the load sensed by the nozzle actuator 40. Furthermore, the movement of the nozzle 49 may include moving in the z direction as necessary in addition to the movement on the xy plane. After the nozzle actuator 40 moves the nozzle 49 to the set xyz position, the bubble forming unit 200 advances the processing to S252.

Next, in S252, the flow channel imaging camera 42 captures an image of the end portion 254 of the nozzle 49. The flow channel imaging camera 42 sends the captured image to the image processing unit 300. The image processing unit 300 may record the image in the recording unit 190 and/or output the image to the bubble forming unit 200.

Next, in S254, on the basis of the captured image of the end portion 254 of the nozzle 49, the bubble forming unit 200 determines whether the position of the nozzle 49 is different from the preset position. For example, the bubble forming unit 200 calculates a difference in position from the captured image of the end portion 254 of the nozzle 49 and the image of the end portion 254 of the nozzle 49 at the preset set xyz position (that is, the initial position), and when the difference is equal to or larger than a threshold, the bubble forming unit 200 determines that the position of the nozzle 49 is different from the initial position.

When it is determined that the position of the nozzle 49 is different from the initial position, the bubble forming unit 200 advances the processing to S256, and otherwise, advances the processing to S300.

In S256, the bubble forming unit 200 decides the operation amount of the nozzle actuator 40. For example, the bubble forming unit 200 decides the operation amount of the nozzle actuator 40 in order to operate the nozzle 49 to the preset set xyz position (that is, the initial position), and sends an instruction to the nozzle actuator 40 to operate by the decided operation amount. For example, the bubble forming unit 200 may decide the operation amount of an amount corresponding to the magnitude of the difference calculated in S254. The nozzle actuator 40 receives the instruction, and the bubble forming unit 200 advances the processing to S250. In the second and subsequent S250, the bubble forming unit 200 sends an instruction to the nozzle actuator 40 to perform an operation by the amount corresponding to the operation amount.

Fig. 9C is an example of a flow in which the relative position between the nozzle 49 and the manipulation target 35 is moved on the basis of the internal pressure of the bubble formed at the end portion 254 of the nozzle 49.

First, in S260, the bubble forming unit 200 controls the pressure generation unit 47 to form the bubble at the end portion 254 of the nozzle 49. Prior to forming the bubble, the bubble forming unit 200 may send an instruction to the nozzle actuator 40 to operate the end portion 254 of the nozzle 49 into the liquid. The step and sub-step of forming the bubble in S260 may be the same as the step and sub-step of S300 described later.

Next, in S262, the bubble forming unit 200 sends an instruction to the nozzle actuator 40 to operate the nozzle 49 to the preset position. The nozzle actuator 40 may be an actuator which controls the z position. In this case, the z position is controlled on the basis of the value of the internal pressure measured by the sensor unit 48. The nozzle 49 may be positioned above a region where no organism is present on the bottom surface of the vessel 25. After the nozzle actuator 40 moves the nozzle 49 to the preset position, the bubble forming unit 200 advances the processing to S264.

In S262, the tip of the nozzle 49 may detect a liquid level and control the z position on the basis of the value of the internal pressure measured by the sensor unit 48. When the bubble forming unit 200 maintains the internal pressure of the nozzle to be equal to or higher than the atmospheric pressure or equal to or lower than the atmospheric pressure, and the tip of the nozzle 49 reaches the liquid level, the value of the internal pressure measured by the sensor unit 48 changes due to an external force caused by the deformation of the gas-liquid interface due to the contact with the liquid level. Therefore, the bubble forming unit 200 can determine whether the tip of the nozzle 49 has reached the liquid level by measuring the value of the internal pressure of the bubble. Accordingly, even when the position to which the nozzle 49 is moved is not set in advance, the bubble forming unit 200 can send, to the nozzle actuator 40, an instruction of the z position control with the liquid level as a reference position to move the position of the nozzle 49.

Next, in S264, the sensor unit 48 measures the internal pressure of the formed bubble, and sends the measured value of the internal pressure of the bubble to the bubble forming unit 200. When the bubble reaches the bottom portion of the vessel 25, the bubble shape is deformed by interaction with the bottom portion, and the internal pressure of the bubble rapidly changes. Therefore, the bubble forming unit 200 can determine whether the bubble has reached the bottom portion of the vessel 25 by measuring the value of the internal pressure of the bubble. In addition, as another example of the internal pressure measurement, the sensor unit 48 may measure the internal pressure while the nozzle actuator 40 moves the nozzle 49 downward, the bubble forming unit 200 may control the pressure, or the pressure generation unit 47 may operate. By simultaneously operating in this manner, it is possible to increase the speed of the detection of the bottom portion or the like, or it is possible to use, as a detection index, a change process of the pressure in the process of forming the bubble. Note that, instead of the sensor unit 48, the nozzle actuator 40 may measure the internal pressure of the bubble and send the measured value of the internal pressure to the bubble forming unit 200. Furthermore, the value of the internal pressure of the bubble measured by the sensor unit 48 or the nozzle actuator 40 may be stored in the recording unit 190 in advance. In this case, the bubble forming unit 200 may determine whether the bubble has reached the bottom portion of the vessel 25 by comparing the value of the internal pressure stored in a storing unit 190 with the internal pressure of the bubble measured by the sensor unit 48 or the nozzle actuator 40.

Next, in S266, the bubble forming unit 200 determines whether the measured value of the internal pressure of the bubble is within a preset internal pressure range. When the measured value of the internal pressure is out of the range of the internal pressure set in advance, the bubble forming unit 200 advances the processing to S268, and otherwise, advances the processing to S270. As described above, an absolute value of a difference between the internal pressure set by the bubble forming unit 200 and the measured internal pressure of the bubble is calculated, and when the difference is equal to or larger than the threshold, the bubble forming unit 200 determines that the nozzle 49 has reached the bottom portion of the vessel 25.

In S268, the bubble forming unit 200 decides the operation amount of the nozzle actuator 40. For example, the bubble forming unit 200 decides the operation amount of the nozzle actuator 40 in order to operate the nozzle 49 to the preset position, and sends an instruction to the nozzle actuator 40 to operate by the decided operation amount. For example, the bubble forming unit 200 may decide the operation amount of an amount corresponding to the magnitude of the difference calculated in S266. The nozzle actuator 40 receives the instruction, and the processing advances to S262. In the second and subsequent S262, the bubble forming unit 200 sends an instruction to the nozzle actuator 40 to perform an operation by the amount corresponding to the operation amount.

In S270, the bubble forming unit 200 controls the pressure generation unit 47 to remove the bubble from the end portion 254 of the nozzle 49. The step and sub-step of removing the bubble in S270 may be the same as the step and sub-step of S500 described later.

Next, in S272, the bubble forming unit 200 sets the initial z position of the nozzle 49. The step of S272 may be the same as the step of S245. After the bubble forming unit 200 sets the initial z position of the nozzle 49, the bubble forming unit 200 advances the processing to S274.

Next, in S274, the bubble forming unit 200 sends an instruction to the nozzle actuator 40 to operate the nozzle 49 to the preset set xyz position (that is, the initial position). The movement of the nozzle 49 may move on the xy plane, and may include moving in the z direction as necessary. After the nozzle actuator 40 moves the nozzle 49 to the preset set xyz position, the bubble forming unit 200 advances the processing to S276.

Next, in S276, the flow channel imaging camera 42 captures an image of the end portion 254 of the nozzle 49. The flow channel imaging camera 42 sends the captured image to the image processing unit 300. The image processing unit 300 may record the image in the recording unit 190 and/or output the image to the bubble forming unit 200.

Next, in S280, on the basis of the captured image of the end portion 254 of the nozzle 49, the bubble forming unit 200 determines whether the position of the nozzle 49 is different from the preset position. For example, the bubble forming unit 200 may calculate a difference in position from the captured image of the end portion 254 of the nozzle 49 and the image of the nozzle end portion 254 at the preset set xyz position (that is, the initial position), and when the difference is equal to or larger than the threshold, the bubble forming unit 200 may determine that the position of the nozzle 49 is different from the initial position.

When it is determined that the position of the nozzle 49 is different from the initial position, the bubble forming unit 200 advances the processing to S282, and otherwise, advances the processing to S300.

In S282, the bubble forming unit 200 decides the operation amount of the nozzle actuator 40. For example, the bubble forming unit 200 decides the operation amount of the nozzle actuator 40 in order to operate the nozzle 49 to the preset set xyz position (that is, the initial position), and sends an instruction to the nozzle actuator 40 to operate by the decided operation amount. For example, the bubble forming unit 200 may decide the operation amount of an amount corresponding to the magnitude of the difference calculated in S280. The nozzle actuator 40 receives the instruction, and the bubble forming unit 200 advances the processing to S274. In the second and subsequent S274, the bubble forming unit 200 sends an instruction to the nozzle actuator 40 to perform an operation by the amount corresponding to the operation amount.

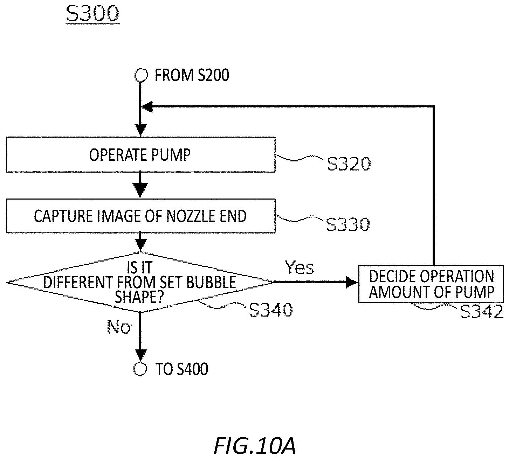

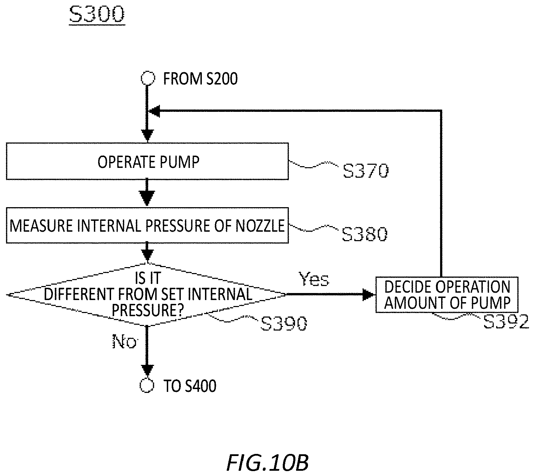

In S300, the bubble forming unit 200 controls the pressure generation unit 47 to enlarge the gas-liquid interface 255. For example, enlarging the gas-liquid interface 255 may include forming the bubble. Prior to forming the bubble, the bubble forming unit 200 may send an instruction to the nozzle actuator 40 to operate the end portion 254 of the nozzle 49 into the liquid. In S300, the step of enlarging the gas-liquid interface 255 includes steps of S320 to S342 as illustrated in Fig. 10A, or includes steps of S370 to S392 as illustrated in Fig. 10B.

Fig. 10 is an example of a flow of enlarging the gas-liquid interface 255 on the basis of the image obtained by capturing the position of the end portion 254 of the nozzle 49.

In S320, the bubble forming unit 200 sends an instruction to the pressure generation unit 47, which is connected to the flow channel 51, to enlarge (form the bubble) the gas-liquid interface 255 at the tip of the flow channel 51. For example, the bubble forming unit 200 sends an instruction to the actuator of the pressure generation unit 47 to push the plunger of the syringe pump of the pressure generation unit 47 by a preset distance or to push the plunger of the syringe pump until the pressure reaches a preset pressure. As a result, the gas pushed out from the syringe pump is supplied to the flow channel 51, and the gas-liquid interface 255 at the tip of the flow channel 51 is enlarged (the bubble is formed). After the gas-liquid interface 255 is enlarged (the bubble is formed), the bubble forming unit 200 advances the processing to S330.

Next, in S330, the flow channel imaging camera 42 captures an image of the bubble formed at the end portion 254 of the nozzle 49. The flow channel imaging camera 42 sends the captured image to the image processing unit 300. The image processing unit 300 may record the image in the recording unit 190 and/or output the image to the bubble forming unit 200.

Next, in S340, the bubble forming unit 200 determines whether the shape of the formed bubble is different from the preset shape of the bubble on the basis of the captured image of the bubble. The bubble forming unit 200 predicts the shape of the bubble to be formed at the end portion 254 of the nozzle 49 from information such as the set internal pressure of the nozzle 49, the inner diameter of the end portion 254 of the nozzle 49, the wettability (the contact angle of the liquid) of the nozzle 49, the type of the liquid, and the type of the gas. For example, the bubble forming unit 200 may determine whether the shape of the formed bubble is different from the set shape of the bubble by comparing the captured image of the bubble with the above shape of the bubble predicted from the information.

When the shape of the formed bubble is different from the set shape of the bubble, the bubble forming unit 200 advances the processing to S342, and otherwise, advances the processing to S400.

In S342, the bubble forming unit 200 decides the operation amount of the plunger of the syringe pump of the pressure generation unit 47. For example, the bubble forming unit 200 decides the operation amount (for example, a distance by which the plunger of the syringe pump is pushed or pulled) of the plunger of the syringe pump of the pressure generation unit 47 in order to form the bubble formed at the tip of the nozzle 49 to have a preset shape. The bubble forming unit 200 sends an instruction to the pressure generation unit 47 to operate by the decided operation amount. For example, the bubble forming unit 200 may decide the operation amount of an amount corresponding to the magnitude of the difference calculated in S340.

The operation amount may be the operation amount of the actuator of the pressure generation unit 47, or may be an additional pressure applied to the syringe pump. The pressure generation unit 47 receives the instruction, and the bubble forming unit 200 advances the processing to S320. In the second and subsequent S320, the pressure generation unit 47 operates by the amount corresponding to the operation amount.

Fig. 10B is an example of a flow of enlarging the gas-liquid interface 255 on the basis of the internal pressure of the nozzle 49.

The step of S370 may be the same as the step of S320. After completing S370, the bubble forming unit 200 advances the processing to S380.

Next, in S380, the sensor unit 48 measures the internal pressure of the nozzle 49, and sends the measured value of the internal pressure of the nozzle 49 to the bubble forming unit 200. Note that, instead of the sensor unit 48, the nozzle actuator 40 may measure the internal pressure of the nozzle 49, and send the measured value of the internal pressure to the bubble forming unit 200.

Next, in S390, the bubble forming unit 200 determines whether the measured value of the internal pressure of the nozzle 49 is within a preset internal pressure range. When the measured value of the internal pressure is out of the range of the set internal pressure, the bubble forming unit 200 advances the processing to S392, and otherwise, advances the processing to S400. For example, the bubble forming unit 200 may calculate a difference between the preset internal pressure and the measured internal pressure of the nozzle 49, and when the difference is equal to or greater than a threshold, determine that the set internal pressure has not been reached.

In S392, the bubble forming unit 200 decides the operation amount (for example, a distance by which the plunger of the syringe pump is pushed or pulled) of the plunger of the syringe pump of the pressure generation unit 47 in order to realize the set internal pressure of the nozzle 49. The bubble forming unit 200 sends an instruction to the pressure generation unit 47 to operate by the decided operation amount. For example, the bubble forming unit 200 may decide the operation amount of an amount corresponding to the magnitude of the difference calculated in S390.

The operation amount may be the operation amount of the actuator of the pressure generation unit 47, or may be an additional pressure applied to the syringe pump. The pressure generation unit 47 receives the instruction, and the bubble forming unit 200 advances the processing to S370. In the second and subsequent S370, the pressure generation unit 47 operates by the amount corresponding to the operation amount.

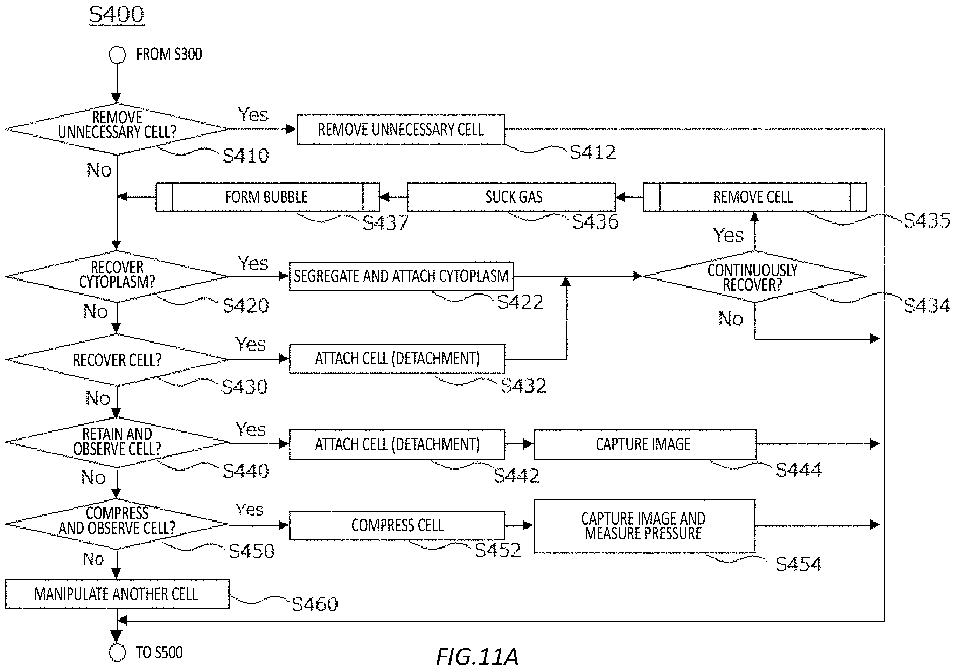

In S400, the manipulation unit 101 executes a manipulation on the manipulation target 35. For example, on the basis of the instruction received via the input unit 180, the bubble forming unit 200 sends an instruction to the manipulation unit 101 to execute the manipulation on the manipulation target 35. In S400, the step of executing the manipulation includes steps of S410 to S460 as illustrated in Fig. 11A. For example, the manipulation may be removal of unnecessary cells, recovery or movement of cell membranes and/or cell membranes, recovery of cells, retention of cells, or compression of cells.

Fig. 11A is an example of a flow of executing the manipulation on the manipulation target 35. In Fig. 11A, an example will be described in which the manipulation target 35 is a cell. Note that the manipulation target 35 is not limited to a cell, and may be another organism.

First, in S410, when an instruction to remove unnecessary cells is received in S140, the information processing device 170 advances the processing to S412. In S410, when an instruction not to remove unnecessary cells is received in S140, the information processing device 170 advances the processing to S420.

In S412, the bubble forming unit 200 attaches the cells to the formed bubble to remove the cells.

For example, the bubble forming unit 200 sends an instruction to the nozzle actuator 40 to move the position of the nozzle 49 in the xyz direction to the position where a target cell is present. After the nozzle actuator 40 moves the nozzle 49 to the target position, the bubble forming unit 200 may move the nozzle 49 and/or the stage to bring the gas-liquid interface 255 of the bubble into contact with the cell.

As an example, the nozzle actuator 40 or the sample actuator 41 specifies, from the image obtained by imaging the target cell by the camera 60 or the camera 70, the position where the target cell is present, and moves such that the center of the nozzle 49 is aligned with the target position. After the cells are brought into contact with the gas-liquid interface 255 of the bubble, the nozzle actuator 40 may recover the target cells into the flow channel 51 as illustrated in 290a to 290c of Fig. 4, and the liquid storage unit 54 may discard these cells in the liquid discarding unit of the liquid storage unit 54.

In addition, as another example, the bubble forming unit 200 may form the gas-liquid interface 255 having a predetermined size in the steps and sub-steps of S300, and control the gas-liquid interface 255 to be enlarged, thereby attaching the cells to the gas-liquid interface 255 and separating the cells. After the liquid control unit 260 removes the unnecessary cells, the bubble forming unit 200 advances the processing to S500.

In S420, when an instruction to recover the cytoplasm and/or cell membrane is received in S140, the bubble forming unit 200 advances the processing to S422, and otherwise, advances the processing to S430.

In S422, the bubble forming unit 200 segregates the cytoplasm and/or cell membrane by using the formed bubble, and attaches them to the bubble to recover them. For example, the bubble forming unit 200 controls the pressure generation unit 47 to form a bubble at the tip of the flow channel 51, and attaches the target cell to the bubble. Next, the bubble forming unit 200 compresses the cell by the bubble to sever only the cytoplasm and/or cell membrane portion and attaches the portion to the bubble. For example, the bubble forming unit 200 controls the pressure generation unit 47 to increase the internal pressure of the bubble or enlarge the bubble, or controls the nozzle actuator 40 to move the nozzle 49 toward the cell and press the bubble against the cell, thereby compressing the cell. Thereafter, the bubble forming unit 200 moves a part of the cell bulging to the outside of the cell due to the compression to sever the part from the cell at the gas-liquid interface, thereby severing the cytoplasm and/or cell membrane portion from the cell. Accordingly, the bubble forming unit 200 controls the nozzle actuator 40 or the pressure generation unit 47 to cut off necessary cytoplasm and/or cell membrane in the manipulation target 35.

For example, the bubble forming unit 200 may send an instruction to the nozzle actuator 40 to move the nozzle 49 in the xyz direction from the initial position to the position where the target cell is present. After the nozzle actuator 40 moves the nozzle 49 to the target position, the bubble forming unit 200 may move the nozzle 49 and/or the stage by the nozzle actuator 40 or the sample actuator 41 to bring the gas-liquid interface 255 of the bubble into contact with the cell. As an example, the bubble forming unit 200 specifies, from the image obtained by the camera 60 or the camera 70 imaging the target cell, the position where the target cell is present, and controls the nozzle actuator 40 to move the nozzle 49 such that the center of the nozzle 49 is aligned with the target position. Herein, the manipulator may specify the position where the target cell is present. In this case, the bubble forming unit 200 may receive, from the input unit 180, an input by the manipulator regarding the position where the target cell is present, and specify the position.

It is known that a cell membrane has a portion exhibiting relatively soft physical properties and a portion exhibiting relatively hard physical properties due to a difference in composition of lipids constituting a membrane component. The bubble forming unit 200 controls the nozzle actuator 40 or the pressure generation unit 47 to compress the cell with the bubble. Herein, in the compression of the cell by the bubble, the cell may be attached to the gas-liquid interface 255 and compressed by the bubble forming unit 200 forming the gas-liquid interface 255 having a predetermined size in the step and the sub-step of S300 and controlling the gas-liquid interface 255 to be enlarged. Next, by utilizing a fact that the portion exhibiting relatively soft physical properties of the cell membrane bulges outward, the bulging portion may be attached by using the gas-liquid interface 255 and moved in a direction away from the cell to sever the cell membrane, and the cell membrane may be recovered in the flow channel 51 while being attached to the gas-liquid interface 255. In addition, when the cell membrane is cut off in this way, the cell membrane bulges by being pushed by the cytoplasm from the inside, and thus the severed cell membrane contains a cytoplasmic component on the inside, and the cytoplasmic component can be recovered in the flow channel 51. After the nozzle actuator 40 severs the necessary cytoplasm and/or cell membrane portion, the bubble forming unit 200 advances the processing to S434.

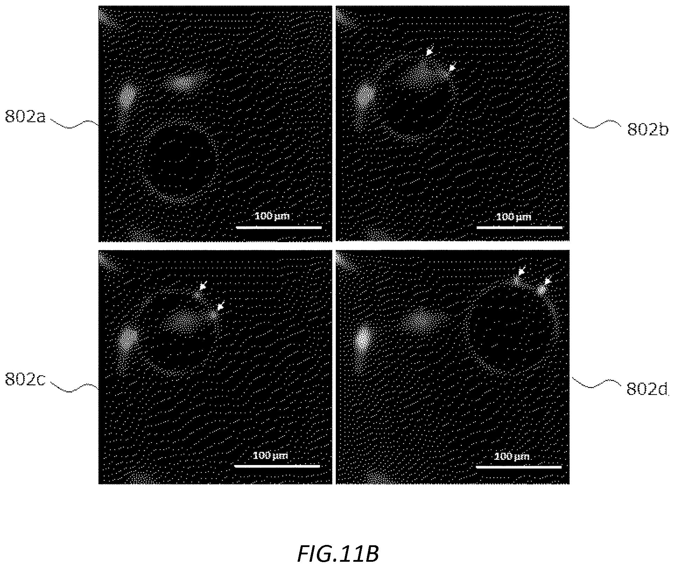

Fig. 11B illustrates an aspect in which the cytoplasm and cell membrane are recovered from the cell according to the present embodiment. By the bubble forming unit 200 controlling the pressure generation unit 47 and the nozzle actuator 40, a relative position between a HeLa cell (human cervical cancer cell) cultured on the solid phase of the vessel 25 and the nozzle 49 was brought close to each other in order to bring the cell into contact with the gas-liquid interface 255 of the bubble (802a). Next, the bubble forming unit 200 compressed the cell by controlling the gas-liquid interface 255 to be pressed against the cell (802b). At this time, an aspect was observed in which a soft portion of the cell membrane bulged outward due to the compression (the arrow of 802b). Next, the gas-liquid interface 255 in a direction away from the cell was moved to sever the cytoplasm and the cell membrane in the bulging portion (the arrow of 802c). Finally, the severed cytoplasm and cell membrane were attached to the gas-liquid interface 255 and recovered (802d). Note that when the operation of Fig. 11B is performed, the operation may be performed by enlarging the gas-liquid interface 255, the operation may be performed by moving the nozzle 49, or the operation may be performed by moving the stage.

Next, in S434, the bubble forming unit 200 determines whether the instruction received in S140 includes continuously recovering the cytoplasm and/or cell membrane of the manipulation target 35. When the determination is positive, the bubble forming unit 200 advances the processing to S435, and when the determination is negative, the bubble forming unit advances the processing to S500.

In S435, the bubble forming unit 200 controls the pressure generation unit 47 to remove the bubble formed at the end portion 254 of the nozzle 49. Herein, when the bubble is removed, the manipulation target 35 may be recovered simultaneously. For example, the manipulation target 35 may be recovered in the liquid present in the flow channel 51. The step and sub-step of removing the bubble in S435 may be the same as the step and sub-step in S500, and details thereof will be described later.

Next, in S436, the bubble forming unit 200 controls the pressure generation unit 47 and the nozzle actuator 40 to take in the gas in order to form a new bubble at the end portion 254 of the nozzle 49. The bubble forming unit 200 sends an instruction to the nozzle actuator 40 to take out the nozzle 49 from the liquid. After the nozzle actuator 40 takes out the nozzle 49 from the liquid, the pressure generation unit 47 may pull the plunger of the syringe pump to take in a necessary amount of gas.

Next, in S437, the bubble forming unit 200 controls the pressure generation unit 47 to form the new bubble at the end portion 254 of the nozzle 49. For the steps and sub-steps of S437, the content of S300 described above may be applied. After the pressure generation unit 47 forms the bubble at the end portion 254 of the nozzle 49, the bubble forming unit 200 advances the processing to S420.

In S430, the bubble forming unit 200 determines whether an instruction to recover the cell is received in S140. When the determination is positive, the bubble forming unit 200 advances the processing to S432, and when the determination is negative, the bubble forming unit 200 advances the processing to S440.

In S432, the bubble forming unit 200 controls the pressure generation unit 47 to form the bubble, and attaches the cell to the bubble. The bubble forming unit 200 may separate the cell attached to the bubble from the solid phase as necessary.

For example, the bubble forming unit 200 sends an instruction to the nozzle actuator 40 to move the nozzle 49 in the xyz direction from the initial position to the position where the target cell is present. After the nozzle actuator 40 moves the nozzle 49 to the target position, the bubble forming unit 200 controls the pressure generation unit 47 to supply gas to the flow channel 51 and form the bubble at the tip of the flow channel 51. Next, the bubble forming unit 200 may control the nozzle actuator 40 or the sample actuator 41 to move the nozzle 49 or the stage and bring the gas-liquid interface 255 of the bubble into contact with the cell.

As an example, the nozzle actuator 40 or the sample actuator 41 specifies, from the image obtained by imaging the target cell by the camera 60 or the camera 70, the position where the target cell is present, and moves such that the center of the nozzle 49 is aligned with the target position. After the nozzle actuator 40 or the sample actuator 41 moves the nozzle 49 to the target position, the bubble forming unit 200 controls the pressure generation unit 47 to supply gas to the flow channel 51 and form the bubble at the tip of the flow channel 51. Next, the bubble forming unit 200 may move the nozzle 49 and/or the stage by the nozzle actuator 40 or the sample actuator 41 to bring the gas-liquid interface 255 of the bubble into contact with the cell. For example, after the cell is brought into contact with the gas-liquid interface 255 of the bubble, the nozzle actuator 40 may separate the cell as necessary by moving the gas-liquid interface 255.

After the bubble forming unit 200 controls the pressure generation unit 47 to form a bubble and attach the cell to the bubble, the bubble forming unit 200 advances the processing to S434. The content described above may be applied to the steps after S434. Note that in this case, in the step of S434, what is continuously recovered is not limited to only the cytoplasm or only the cell, and may be both the cytoplasm and the cell.

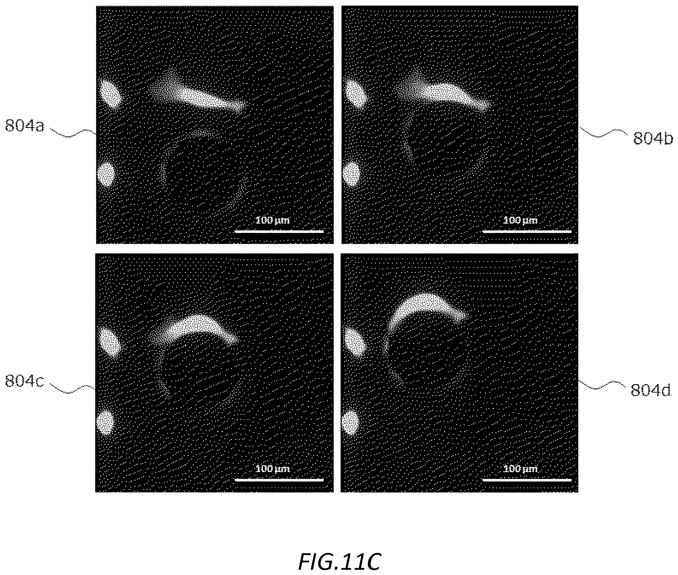

Fig. 11C illustrates an aspect in which a cell line is attached to the bubble to be separated according to the present embodiment. By the bubble forming unit 200 controlling the pressure generation unit 47 and the nozzle actuator 40 (alternatively, the sample actuator 41 instead of the nozzle actuator 40), a relative position between the HeLa cell cultured in the solid phase of the vessel 25 and the nozzle 49 was brought close to each other in order to bring the cell into contact with the gas-liquid interface 255 of the bubble (804a). Next, the cell was brought into contact with the gas-liquid interface 255 of the bubble (804b). Next, the nozzle actuator 40 moved the gas-liquid interface 255 (804c), and separated the cell by attaching the cell to the gas-liquid interface 255 (804d). Note that when the operation of Fig. 11C is performed, the operation may be performed by enlarging the gas-liquid interface 255, the operation may be performed by moving the nozzle 49, or the operation may be performed by moving the stage.

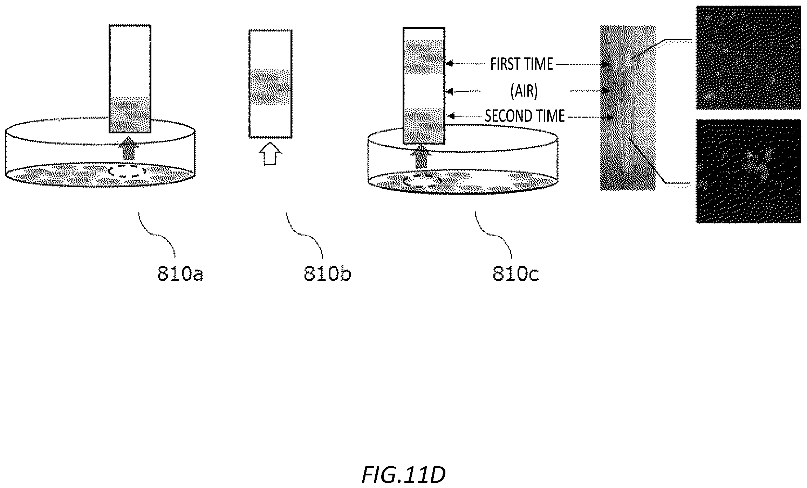

Fig. 11D is a schematic diagram when S430 -> S432 -> S434 -> S435 -> S436 -> S437 are repeated. When the cytoplasm and/or cell membrane is continuously recovered and when the cell is continuously recovered, the recovery may be performed as illustrated in the schematic diagram of Fig. 11D. In 810a, after the nozzle actuator 40 puts the nozzle 49 into the liquid, the bubble forming unit 200 controls the pressure generation unit 47 to cause a syringe pump (not illustrated) to supply gas, thereby forming a bubble at the end portion 254 of the nozzle 49. Next, the cell adhering to the bottom surface of the vessel 25 is attached to the gas-liquid interface 255 of the bubble to be separated, and is recovered in the flow channel 51 by causing the syringe pump to take in the gas. Next, in 810b, the nozzle actuator 40 pulls up the nozzle 49 from the liquid, and the syringe pump sucks the gas (for example, air) into the flow channel 51. Next, in 810c, after the nozzle actuator 40 puts the nozzle 49 into the liquid, the syringe pump forms a new bubble by supplying gas to the end portion 254 of the nozzle 49, and recovers the cell adhering to the bottom surface of the vessel 25 by attaching the cell to the gas-liquid interface 255 of the bubble. In this way, by controlling the pressure generation unit 47 and the nozzle actuator 40, the bubble forming unit 200 can continuously recover two cells (population) into the flow channel 51 via the gas without mixing the two cells. In actual, a photograph of two cells (population) recovered with air interposed therebetween by this method is also shown. Note that in Fig. 11D, an example in which the nozzle 49 is moved has been described as an example, but the operation of Fig. 11D may be performed by moving the stage instead of moving the nozzle 49.

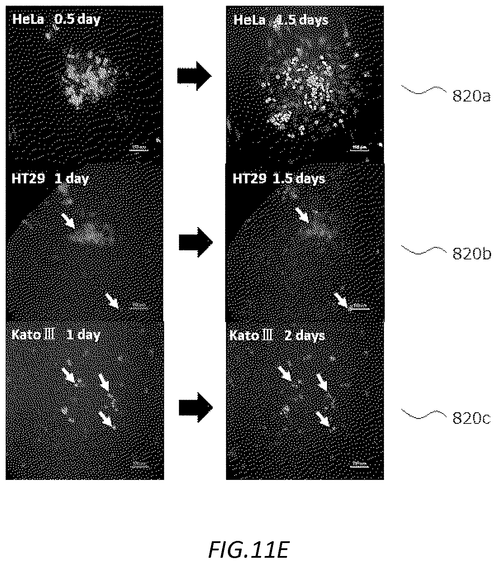

Fig. 11E illustrates an aspect of passage in which the cell lines are recovered, and the recovered cell lines are cultured according to the present embodiment. By the bubble forming unit 200 controlling the pressure generation unit 47 and the nozzle actuator 40, a HeLa cell (human cervical cancer cell, 820a), a HT29 cell (human colorectal cancer cell, 820b), and a Kato III cell (human gastric signet ring cell cancer cell, 820c), which were cell lines, were attached, separated, and recovered from the solid phase of the vessel 25 and extracted to a medium in another vessel by using the gas-liquid interface 255 of the bubble, and were cultured for 1.5 days (820a, and 820b) and 2 days (820c). All cells were confirmed to be growing after the passage. That is, according to the present embodiment, even when the cell lines were separated by using the gas-liquid interface 255 of the bubble, the cell could be grown without damaging the viability of the cell. Note that when the operation of Fig. 11E is performed, the operation may be performed by moving the nozzle 49, or the operation may be performed by moving the stage.

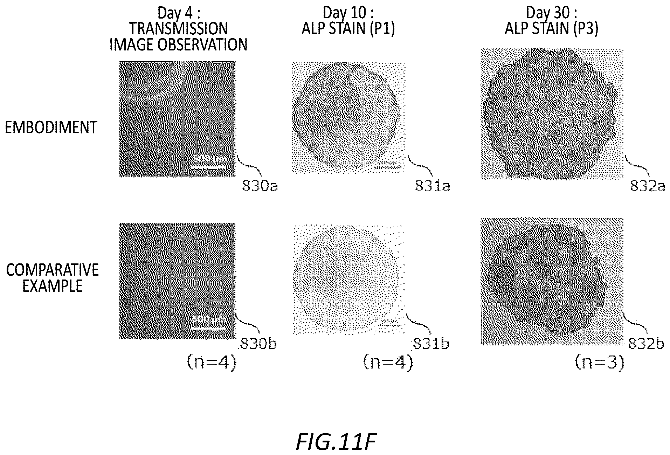

Fig. 11F illustrates an aspect in which an iPS cell is recovered and the recovered iPS cell is passaged according to the present embodiment. By the bubble forming unit 200 controlling the pressure generation unit 47 and the nozzle actuator 40, the colony of the cultured iPS cell was attached, separated, and recovered from the solid phase of the vessel 25 and extracted into a liquid medium in another vessel by using the gas-liquid interface 255 of the bubble, and was cultured for 4 days, and then a transmission image was observed. In addition, as a comparative example, an aspect was used in which the iPS cell is passaged by a normal cell passage method (mechanical passage). As a result, no morphological difference was recognized between the iPS cell (830a) of the present embodiment and the iPS cell (830b) of the comparative example. In addition, after culturing for 10 days, the iPS cells of the present embodiment and the comparative example were stained with alkaline phosphatase. Furthermore, the iPS cells of the present embodiment and the comparative example were passaged three times, cultured for 30 days, and then the iPS cells of the present embodiment and the comparative example were stained with alkaline phosphatase. As a result, no difference in stainability was recognized between the iPS cell of the present embodiment (the cell cultured for 10 days was 831a, and the cell cultured for 30 days was 832a) and the iPS cell of the comparative example (the cell cultured for 10 days are 831b, and the cell cultured for 30 days are 832b). It is known that alkaline phosphatase is highly expressed in iPS cells which are undifferentiated and maintain self-replication ability. That is, according to the present embodiment, even when the iPS cell was separated and recovered by using the gas-liquid interface 255, without affecting the maintainability of undifferentiation ability, the iPS cell could be grown while maintaining the undifferentiated state. Note that when the operation of Fig. 11F is performed, the operation may be performed by moving the nozzle 49, or the operation may be performed by moving the stage.

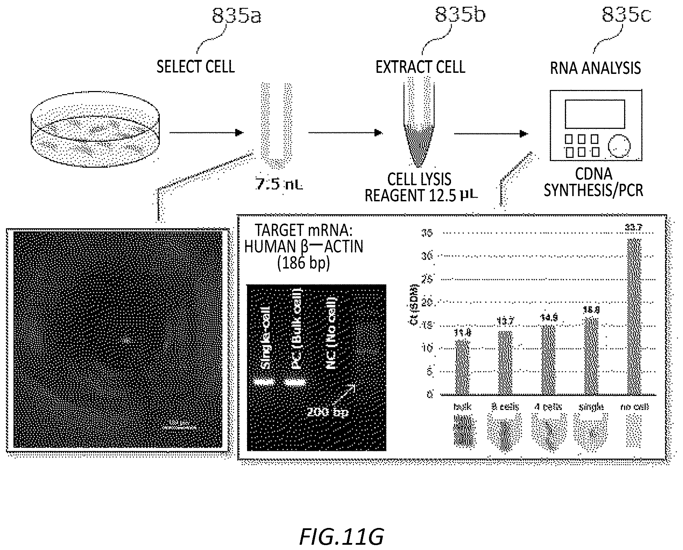

Fig. 11G illustrates an aspect in which cell lines are recovered, and the recovered cells are analyzed according to the present embodiment. HeLa cells, which were cell lines, were separated and recovered from the solid phase of the vessel 25 by using the gas-liquid interface 255 of the bubble. By the bubble forming unit 200 controlling the pressure generation unit 47 and the nozzle actuator 40, the HeLa cells were selected from one cell, four cells, and eight cells from the solid phase, and separated by using the gas-liquid interface 255 of the bubble, and these cells were recovered together with 7.5 nL of the liquid medium (835a). Next, the recovered HeLa cells were extracted into 12.5 μL of cell lysis reagent to lyse the cells (835b). In a cell lysate in which HeLa cells were lysed, cDNA was synthesized from mRNA of β-actin, and a PCR reaction was performed (835c). As a result, the amount of cDNA roughly proportional to the number of recovered cells could be detected. That is, according to the present embodiment, one cell or an arbitrary number of cells could be recovered by using the gas-liquid interface 255 to perform molecular biological analysis. Note that when the operation of Fig. 11G is performed, the operation may be performed by moving the nozzle 49, or the operation may be performed by moving the stage.

Next, in S440, the bubble forming unit 200 determines whether an instruction to retain and image the cell is received in S140. When the determination is positive, the bubble forming unit 200 advances the processing to S442, and when the determination is negative, the bubble forming unit advances the processing to S450.

In S442, the bubble forming unit 200 causes cells to be attached to the formed bubble and performs control to retain the attached cells. For example, the bubble forming unit 200 sends an instruction to the nozzle actuator 40 to move the nozzle 49 in the xyz direction from the initial position to the position where the target cell is present. After the nozzle actuator 40 moves the nozzle 49 to the target position, the bubble forming unit 200 controls the pressure generation unit 47 to supply gas to the flow channel 51 and form the bubble at the tip of the flow channel 51. The bubble forming unit 200 may cause the nozzle actuator 40 or the sample actuator 41 to move the nozzle 49 and/or the stage and bring the gas-liquid interface 255 of the bubble into contact with the cell. As an example, the nozzle actuator 40 or the sample actuator 41 specifies, from the image obtained by imaging the target cell by the camera 60 or the camera 70, the position where the target cell is present, and moves such that the center of the nozzle 49 is aligned with the target position. After the cell is brought into contact with the gas-liquid interface 255 of the bubble, the bubble forming unit 200 advances the processing to S444.

Herein, the manipulator may specify the position where the target cell is present. In this case, the bubble forming unit 200 may receive, from the input unit 180, an input by the manipulator regarding the position where the target cell is present, and specify the position. In addition, in the above example, a case has been described in which the tip surface of the bubble is brought into contact with the cell, but the bubble and the cell may be brought into contact with each other by bringing the side surface of the bubble into contact with the cell. In this case, the nozzle actuator 40 or the sample actuator 41 may move such that the center of the nozzle 49 is aligned with the vicinity of the target cell. In addition, for example, after the cell is brought into contact with the gas-liquid interface 255 of the bubble, the nozzle actuator 40 may separate the cell as necessary by moving the gas-liquid interface 255.

Next, in S444, the imaging control unit 171 sends an instruction to the camera 60 or the camera 70 to image the cell retained by the bubble. The camera 60 or the camera 70 captures an image and sends the image to the image processing unit 300. The image processing unit 300 may record the image in the recording unit 190 and/or output the image to the bubble forming unit 200.

After the camera 60 or the camera 70 image the retained cell, the bubble forming unit 200 advances the processing to S500.

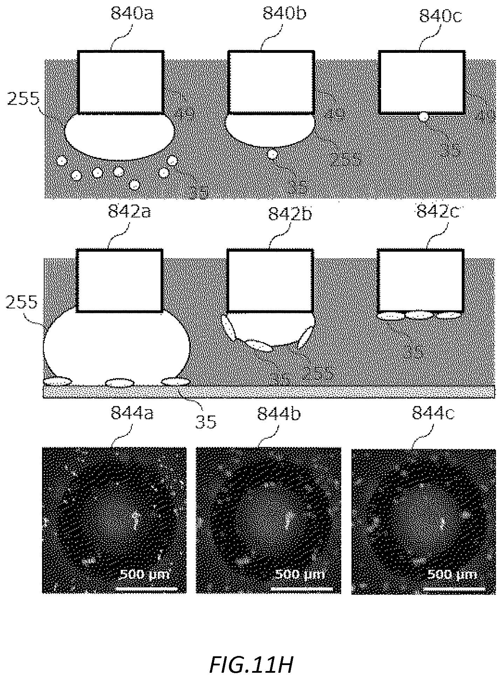

Fig. 11H is a schematic diagram illustrating an aspect in which cell lines are retained at the gas-liquid interface 255 of the bubble and observed. Floating cells or weakly adhered cells move freely in the culture solution by slight vibration or the like, and thus it is difficult to observe them as they are by using a microscope or the like. In 840a, the bubble forming unit 200 controls the nozzle actuator 40 to put the end portion 254 of the nozzle 49 into the liquid medium in the vessel 25 in which the floating cells (manipulation target 35) are cultured. Next, the pressure generation unit 47 supplies gas to the flow channel 51 to form a bubble at the tip of the nozzle 49, thereby forming the gas-liquid interface 255. The bubble forming unit 200 controls the nozzle actuator 40 or the pressure generation unit 47 to attach the floating cells to be the manipulation targets 35 to the formed gas-liquid interface 255. Next, in 840b, the pressure generation unit 47 controls the internal pressure of the bubble and temporarily retains the cell at the gas-liquid interface 255 of the bubble. Next, in 840c, the pressure generation unit 47 takes in gas from the flow channel 51 to reduce the bubble. The cells retained in that state can be observed by using the microscope or the like. Alternatively, the pressure generation unit 47 may retain the cells without reducing the bubble, and observe the retained cells by using the microscope or the like. In this manner, according to the present embodiment, the cells are retained at the gas-liquid interface 255 of the bubble, whereby the cells can be observed without being moved.

In addition, in the case of adherent cells scattered on the solid phase of the vessel 25, it is necessary to move the stage and observe the adherent cells in a wide field of view in order to observe the adherent cells with the microscope or the like. In 842a, the bubble forming unit 200 controls the nozzle actuator 40 to put the end portion 254 of the nozzle 49 into the liquid medium of the vessel 25 in which the adherent cells (manipulation target 35) are cultured. Next, the pressure generation unit 47 supplies gas to the flow channel 51 to form a bubble at the tip of the nozzle 49, thereby forming the gas-liquid interface 255. Next, by controlling the nozzle actuator 40 or the pressure generation unit 47, the bubble forming unit 200 controls the gas-liquid interface 255 to attach the cells to the gas-liquid interface 255 and separate the cells. Next, in 842b, the pressure generation unit 47 temporarily retains the cells at the gas-liquid interface 255 of the bubble. Next, in 842c, the pressure generation unit 47 takes in gas from the flow channel 51 to reduce the bubble. The cells retained in such a state are present at the same z position in a narrow range of the plane, and thus can be simultaneously observed by using the microscope or the like. In this manner, according to the present embodiment, the cells are retained at the gas-liquid interface of the bubble, so that it is possible to reduce a field of view to be observed.

As an example of such an observation method, Kato III cells which are floating cells were scattered on the solid phase, and some of the cells were attached to the gas-liquid interface 255 (844a). Thereafter, the bubble forming unit 200 controlled the internal pressure of the bubble via the pressure generation unit 47 to retain the cells at the gas-liquid interface 255 while reducing the bubble, and when the retained cells were focused, the surrounding cells were out of focus (844b). At this time, when the stage is moved, the surrounding cells move, but the cells retained at the gas-liquid interface 255 do not move, so that the retained cells can be easily observed with the microscope (844c).

In S450, the bubble forming unit 200 determines whether an instruction to compress and image the cell is received in S140. When the determination is positive, the bubble forming unit 200 advances the processing to S452, and when the determination is negative, the bubble forming unit advances the processing to S460.

In S452, the bubble forming unit 200 controls the pressure generation unit 47 and the nozzle actuator 40 to compress the cell by using the bubble. For example, the bubble forming unit 200 controls the pressure generation unit 47 to form a bubble at the tip of the flow channel 51, and brings the bubble into contact with the cell to be the manipulation target 35. Note that when the operation of Fig. 11H is performed, the operation may be performed by moving the nozzle 49, or the operation may be performed by moving the stage.

As an example, the bubble forming unit 200 sends an instruction to the nozzle actuator 40 to move the nozzle 49 in the xyz direction from the initial position to the position where the target cell is present. As an example, the bubble forming unit 200 specifies, from the image obtained by the camera 60 or the camera 70 imaging the target cell, the position where the target cell is present, and controls the nozzle actuator 40 to move the nozzle 49 such that the center of the nozzle 49 is aligned with the target position.

Herein, the manipulator may specify the position where the target cell is present. In this case, the bubble forming unit 200 may receive, from the input unit 180, an input by the manipulator regarding the position where the target cell is present, and specify the position. In addition, the contact between the bubble and the cell may be performed by bringing the tip surface of the bubble into contact with the cell, or may be performed by bringing the side surface of the bubble into contact with the cell. When the tip surface of the bubble is brought into contact with the cell, the nozzle actuator 40 or the sample actuator 41 may move such that the center of the nozzle 49 is aligned directly above the target cell. When the side surface of the bubble is brought into contact with the cell, the nozzle actuator 40 or the sample actuator 41 may move such that the center of the nozzle 49 is aligned with the vicinity of the target cell, and in this case, the bubble is formed beside the cell, and the cell can be gradually compressed from the side by moving the nozzle 49.

Next, after the nozzle actuator 40 moves the nozzle 49 to the target position, the bubble forming unit 200 controls the pressure generation unit 47 to supply gas to the flow channel 51 and form, at the target position, the bubble at the tip of the flow channel 51. The bubble forming unit 200 may cause the nozzle actuator 40 or the sample actuator 41 to move the nozzle 49 and/or the stage and bring the gas-liquid interface 255 of the bubble into contact with the cell. When the position of the nozzle 49 is fixed, the gas-liquid interface 255 and the cell may be brought into contact with each other by moving the stage. The cell may be brought into contact with the gas-liquid interface 255 by the bubble forming unit 200 controlling via the pressure generation unit 47 to enlarge the gas-liquid interface 255.

As an example, the bubble forming unit 200 operates the plunger of the syringe pump of the pressure generation unit 47 with a preset operation amount to form a bubble of a preset volume, and then moves the nozzle 49 and/or the stage by the nozzle actuator 40 or the sample actuator 41 such that the nozzle 49 is positioned at a position where the bubble compresses the cell. Next, the bubble forming unit 200 may control the pressure generation unit 47 to enlarge the bubble formed at the tip of the flow channel 51, or may control the nozzle actuator 40 to move the nozzle 49 toward the cell and press the bubble against the cell, thereby compressing the cell.

In addition, as an example, the bubble forming unit 200 may move the nozzle 49 very close to the cell and then control the pressure generation unit 47 to form the bubble of the preset volume at the tip of the flow channel 51, thereby compressing the cell.

Next, in S454, the imaging control unit 171 sends an instruction to the camera 60 or the camera 70 to image the compressed cell. The camera 60 or the camera 70 captures an image and sends the image to the image processing unit 300. The image processing unit 300 may record the image in the recording unit 190 and/or output the image to the output unit 160.

In addition, alternatively or additionally, the sensor unit 48 or the nozzle actuator 40 may measure the pressure at which the bubble compresses the cell and send the value of the measured pressure to the bubble forming unit 200. The camera 60 or the camera 70 may repeatedly perform image capturing while changing the pressure at which the bubble forming unit 200 compresses the cells

The pressure of compressing the cell can be changed by the bubble forming unit 200 controlling the pressure generation unit 47 to change the internal pressure and/or volume of the bubble.

As an example, the bubble forming unit 200 may move the nozzle 49 very close to the cell, then control the pressure generation unit 47 to form a bubble at the tip of the flow channel 51, and compress the entire cell or various sites of the cell while changing the internal pressure and/or volume of the bubble to maintain or change the pressure of compressing the cell. It is expected that various sites of the cell have different hardness depending on the composition or distribution of the cell membrane or the intracellular organelle. In this way, the composition or distribution of the cell membranes in the cell or the intracellular organelle can be analyzed by using, as indexes, the pressure and/or the observation image at the time of the compression by the bubble. By compressing the cell, the thickness of the cell is reduced so that a structure in the deep portion of the cell can be clearly observed, and by expanding the cell in the lateral direction, adjacent structures are separated to be individually segregated and observed. By observing the cell while compressing the cell, it is possible to obtain information on the force applied to the cell and the internal and external morphological change amounts of the cell, and it is possible to analyze the information on the mechanism of the cell. After the camera 60 or the camera 70 images the compressed cell, the bubble forming unit 200 advances the processing to S500.

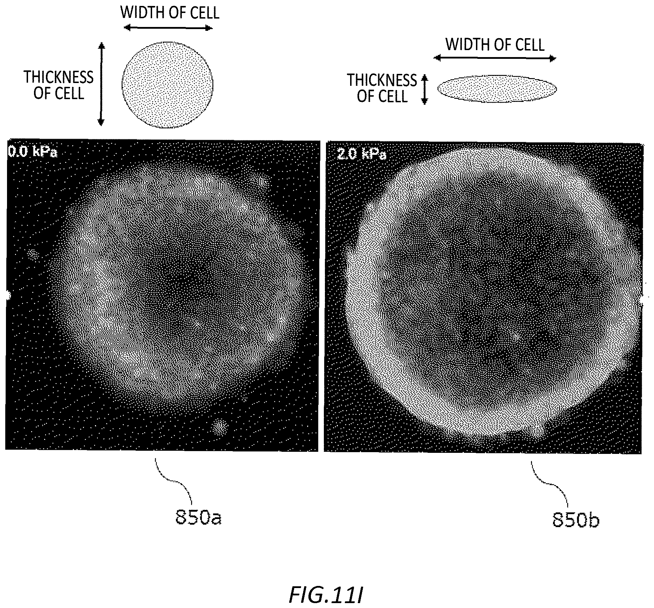

Fig. 11I illustrates an aspect of compressing the cell line by using the bubble and observing the deep portion of the cell according to the present embodiment. By staining the nucleus and cytoplasm of a spheroid (850a) formed from a HT29 cell in a living state and performing compression by using the bubble, it was possible to observe a structure, such as the nucleus, in the deep portion of the cell (850b). In particular, when comparison is performed with a thick central portion, the nucleus cannot be seen in the central portion in 850a before the compression, but the nucleus can be confirmed in the central portion in 850b obtained by observation with the compression. Conventionally, in order to observe a structure in the deep portion of the cell, observation can be performed by fixing the cell with formalin, methanol, or the like and thinly slicing the cell, or observation can be performed by using a special microscope specialized for deep portion observation. According to the present embodiment, the structure in the deep portion of the cell can be observed in a living state without using a special microscope. Furthermore, in the spheroid (850a) before the compression, the nuclei were in close contact with each other, and it was difficult to recognize individual nuclei, but in the spheroid (850b) obtained by observation with the compression, the spheroid expands in the longitudinal and lateral directions, so that a sufficient interval was generated between the nuclei, and the nuclei could be recognized independently. Conventionally, in order to independently recognize two or more adjacent organelles inside a cell, a microscopic system in which an optical system and a fluorescent labeling method are devised has been researched and developed, and called a super-resolution microscope. In these microscope technologies, a resolution increases, but an observation field becomes narrow, and an imaging time is lengthened. According to the present embodiment, without using a special microscope, two or more close organelles inside the cell can be independently recognized in the living state in a short imaging time while maintaining the observation field. In addition, the three-dimensional structure of the original cell can be reproduced from the observation image and the mechanism information of the compressed cell.

In S460, the bubble forming unit 200 controls the manipulation unit 101 such that necessary manipulations other than S410 to S450 in the instruction received in S140 are performed on the manipulation target 35. For example, the manipulation may be evaluation of cell adhesiveness, induction of cell differentiation, and the like, which will be described later. After completing S460, the bubble forming unit 200 advances the processing to S500.

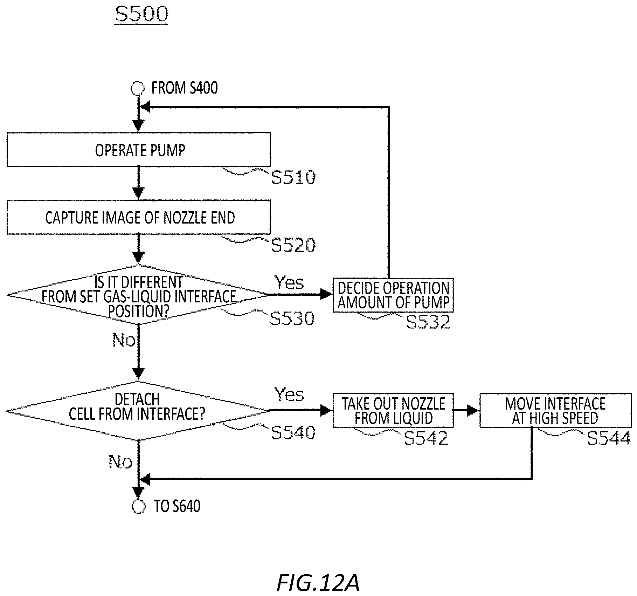

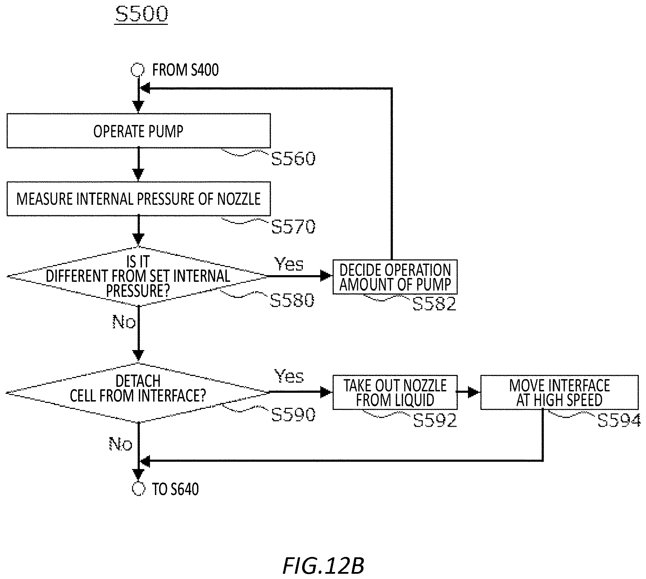

In S500, the bubble forming unit 200 controls the pressure generation unit 47 to reduce the gas-liquid interface 255. Reducing the gas-liquid interface 255 may include removing the bubble. Herein, when the bubble is reduced or removed, the manipulation target 35 may be recovered simultaneously. In S500, the step of reducing the gas-liquid interface 255 includes steps of S510 to S544 as illustrated in Fig. 12A, or includes steps of S560 to S594 as illustrated in Fig. 12B.

Fig. 12 is an example of a flow of reducing the gas-liquid interface 255 on the basis of the image obtained by capturing the position of the end portion 254 of the nozzle 49.

In S510, the bubble forming unit 200 controls the pressure generation unit 47 to perform the suction operation on the flow channel 51 and take in the gas-liquid interface 255 from the tip of the flow channel 51. At this time, the liquid is also taken in simultaneously. The liquid to be taken in may be used for detaching the manipulation target 35 from the gas-liquid interface 255. The liquid to be taken in may be a liquid accommodated in the vessel 25 or the like (for example, a medium), or may be another liquid housed in the liquid storage unit 54.

For example, the bubble forming unit 200 sends an instruction to the actuator of the pressure generation unit 47 to pull the plunger of the syringe pump of the pressure generation unit 47 by a preset distance or to pull the plunger of the syringe pump until the pressure reaches a preset pressure. When receiving an instruction from the volume control unit or the gas intake control unit in the bubble forming unit 200, the pressure generation unit 47 takes in gas. As a result, the gas-liquid interface 255 is reduced (the bubble is removed), and the gas-liquid interface 255 is taken into the flow channel 51. After the gas-liquid interface 255 is reduced (the bubble is removed), the bubble forming unit 200 advances the processing to S520.

Next, in S520, the flow channel imaging camera 42 captures an image of the end portion 254 of the nozzle 49, and sends the image to the image processing unit 300. The image processing unit 300 may record the image in the recording unit 190 and/or output the image to the bubble forming unit 200.

Next, in S530, on the basis of the captured image of the end portion 254 of the nozzle 49, the bubble forming unit 200 determines whether the position of the gas-liquid interface 255 taken in by the flow channel 51 is different from the position set in advance in the bubble forming unit 200. When the positions are different from each other, the bubble forming unit 200 advances the processing to S532, and otherwise, advances the processing to S540. For example, the bubble forming unit 200 may calculate a difference between the position of the gas-liquid interface 255 calculated on the basis of the captured image of the end portion 254 of the nozzle 49 and the preset position, and if the difference is equal to or larger than a threshold, the bubble forming unit 200 may determine that the position of the gas-liquid interface 255 taken in by the flow channel 51 is different from the set position.

In S532, the bubble forming unit 200 decides the operation amount of the plunger of the syringe pump of the pressure generation unit 47. For example, the bubble forming unit 200 decides the operation amount (for example, a distance by which the plunger of the syringe pump is pushed or pulled) of the plunger of the syringe pump of the pressure generation unit 47 in order to take the gas-liquid interface 255 to the preset position of the gas-liquid interface 255. The bubble forming unit 200 sends an instruction to the pressure generation unit 47 to operate by the decided operation amount. For example, the bubble forming unit 200 may decide the operation amount of an amount corresponding to the magnitude of the difference calculated in S530.

The operation amount may be the operation amount of the actuator of the pressure generation unit 47, or may be an additional pressure applied to the syringe pump. The pressure generation unit 47 receives the instruction, and the bubble forming unit 200 advances the processing to S510. In the second and subsequent S510, the pressure generation unit 47 operates by the amount corresponding to the operation amount.

In S540, when the instruction received in S140 includes detaching the cell from the interface (for example, cell recovery), the bubble forming unit 200 advances the processing to S542, and otherwise, the bubble forming unit 200 advances the processing to S640.

In S542, the bubble forming unit 200 sends, to the nozzle actuator 40, an instruction to take out the nozzle 49 from the liquid. The nozzle actuator 40 moves the nozzle 49 upward by a preset distance to take out the nozzle 49 from the liquid, and then the bubble forming unit 200 advances the processing to S544.

Next, in S544, the bubble forming unit 200 controls the pressure generation unit 47 to move the gas-liquid interface 255 between the gas and the liquid in the flow channel 51 at a high speed. Accordingly, the cell attached to the gas-liquid interface 255 is detached from the gas-liquid interface 255 and moves to the liquid. For example, the bubble forming unit 200 causes the pressure generation unit 47 to rapidly perform the reciprocating operation of the plunger of the syringe pump to repeat the gas supply and the gas intake in the flow channel 51, thereby moving the gas-liquid interface 255 at a high speed. In addition, additionally/alternatively, the bubble forming unit 200 may cause the nozzle actuator 40 to reciprocate the nozzle 49 at a high speed in the up-and-down direction ($\pm$ z direction) and/or the longitudinal and lateral directions ($\pm$ xy direction), thereby moving the gas-liquid interface 255 in the flow channel 51 at a high speed.

The bubble forming unit 200 may cause the high-speed movement of the gas-liquid interface 255 to be performed on the spot (at the place where S542 is performed), or may cause the high-speed movement to be performed after the nozzle actuator 40 is caused to put the nozzle 49 into the liquid of a designated movement destination. The bubble forming unit 200 can appropriately detach the cell attached to the gas-liquid interface 255 from the gas-liquid interface 255 by controlling the moving speed of the liquid or the like from the information such as the internal pressure of the nozzle 49 received from the sensor unit 48. In addition, the bubble forming unit 200 may vibrate the gas-liquid interface 255 by forming an electromagnetic field with respect to the nozzle 49.

Furthermore, the bubble forming unit 200 may control the cell to be detached from the gas-liquid interface 255 by bringing the bubble into contact with the filter. The bubble forming unit 200 may control the liquid storage unit 54 to add a liquid for reducing the free energy of the interface, thereby detaching the cell from the gas-liquid interface 255. Additionally, the bubble forming unit 200 may control the nozzle actuator 40 and the pressure generation unit 47 to form a bubble at the tip of the nozzle 49 at the designated movement destination, and detach the cell by rubbing the cell against the bottom surface of the vessel 25 at the designated movement destination. In addition, the bubble forming unit 200 may push out and detach the cell by controlling the pressure generation unit 47 to increase the internal pressure of the bubble. In addition, the cell may be detached from the gas-liquid interface 255 by making the liquid at the movement destination into a liquid in which the interface free energy decreases. After the cell is detached from the interface, the bubble forming unit 200 advances the processing to S640.

Fig. 12B is an example of a flow of reducing the gas-liquid interface 255 on the basis of the internal pressure of the nozzle 49.

In S560, the bubble forming unit 200 controls the pressure generation unit 47 to perform the suction operation on the flow channel 51 and take in the gas-liquid interface 255 from the tip of the flow channel 51. The step of S560 may be the same as the step of S510. Next, the bubble forming unit 200 advances the processing to S570.

Next, in S570, the sensor unit 48 measures the internal pressure of the nozzle 49, and sends the measured value of the internal pressure of the nozzle 49 to the bubble forming unit 200. Note that, instead of the sensor unit 48, the nozzle actuator 40 may measure the internal pressure of the nozzle 49, and send the measured value of the internal pressure to the bubble forming unit 200.

Next, in S580, the bubble forming unit 200 determines whether the measured value of the internal pressure of the nozzle 49 is within a preset internal pressure range. When the measured value of the internal pressure is out of the range of the internal pressure set in advance, the bubble forming unit 200 advances the processing to S582, and otherwise, advances the processing to S590. For example, the bubble forming unit 200 may calculate a difference between the preset internal pressure and the measured internal pressure of the nozzle 49, and when the difference is equal to or greater than a threshold, determine that the set internal pressure has not been reached.

In S582, the bubble forming unit 200 decides the operation amount (for example, a distance by which the plunger of the syringe pump is pushed or pulled) of the plunger of the syringe pump of the pressure generation unit 47 in order to realize the set internal pressure of the nozzle 49. The bubble forming unit 200 sends an instruction to the pressure generation unit 47 to operate by the decided operation amount. For example, the bubble forming unit 200 may decide the operation amount of an amount corresponding to the magnitude of the difference calculated in S580.

The operation amount may be the operation amount of the actuator of the pressure generation unit 47, or may be an additional pressure applied to the syringe pump. The pressure generation unit 47 receives the instruction, and the bubble forming unit 200 advances the processing to S560. In the second and subsequent S560, the pressure generation unit 47 operates by the amount corresponding to the operation amount.

In S590, when the instruction received in S140 includes detaching the cell from the gas-liquid interface 255 (for example, cell recovery), the bubble forming unit 200 advances the processing to S592. The steps S590 to S594 may be the same as the steps S540 to S544. After completing S594, the bubble forming unit 200 advances the processing to S640. When the instruction received in S140 does not include detaching from the gas-liquid interface 255 the cell from the gas-liquid interface 255, the bubble forming unit 200 advances the processing to S640.

Next, in S640, the information processing device 170 receives, via the input unit 180, an input regarding extraction of the manipulation target 35 from the manipulator. When the information processing device 170 has received the instruction to extract the manipulation target 35, the information processing device 170 advances the processing to S645, and otherwise, advances the processing to S650.

In S645, the bubble forming unit 200 may send, to the nozzle actuator 40, an instruction regarding the movement destination of the recovered manipulation target 35. For example, the movement destination of the manipulation target 35 may be designated by the manipulator in the display region of the GUI as illustrated in Fig. 7B. The nozzle actuator 40 may immerse, in the liquid of the movement destination, the nozzle 49 including the manipulation target 35 attached to or detached from the gas-liquid interface 255 and extract the manipulation target to the liquid of the movement destination. After the nozzle actuator 40 extracts the recovered cell to the liquid of the movement destination, the bubble forming unit 200 advances the processing to S650. Note that the cell extracted into the liquid of the movement destination may be observed by using the microscope unit 50.

When there is another manipulation target 35 in S650, the bubble forming unit 200 advances the processing to S660. When there is no other manipulation target 35 in S650, the bubble forming unit 200 advances the processing to S680.

In S660, when the nozzle 49 needs to be exchanged in manipulating another manipulation target 35, the bubble forming unit 200 advances the processing to S670, and when the nozzle 49 does not need to be exchanged, the bubble forming unit advances the processing to S200.

In S670, the flow channel control unit 250 sends, to the flow channel exchange unit 53, an instruction to remove the nozzle 49 installed in the nozzle actuator 40 and discard the nozzle to the nozzle discarding unit of the flow channel exchange unit 53. Note that the nozzle may be stored in a state where the cell is taken into the nozzle 49 without being extracted, and then the taken cell may be analyzed. In this case, the nozzle 49 may be housed in the nozzle housing unit of the flow channel exchange unit 53 without being discarded. After the flow channel exchange unit 53 discards the nozzle 49, the processing advances to S180.

In S680, the nozzle 49 may be discarded in a procedure similar to S670. The flow channel exchange unit 53 discards the nozzle 49, and the flow ends.

In the above flow, the case of removing unnecessary cells, recovering cytoplasm and/or a cell membrane, recovering and passaging a cell, retaining the cell, and compressing the cell has been described as an example of manipulating the manipulation target 35. Examples of the manipulation include some other than those listed above.

An example of the manipulation includes applying a culture substrate or an agent to the solid phase on the bottom surface of the vessel 25 and evaluating the adhesiveness of the culture substrate or the agent to cells. Since the adhesiveness to cells can be evaluated by using, as indices, the internal pressure of the bubble, the moving speed of the nozzle, and the load at the time of separating the cells, the effectiveness of the adhesion of the culture substrate and the agent to the cells can be evaluated.

Another example of the manipulation includes sorting cells. According to the above-described flow, the cells are compressed by the bubble. It is conceivable that a cell membrane, an intracellular component, or a physical property varies depending on the type of the cell. Therefore, it is conceivable that the shape change process of the cell and the shape of the cell vary after the bubble forming unit 200 controls the nozzle actuator 40 and/or the pressure generation unit 47 to start the compression of the cell by the bubble, to stop the compression, or to release the cell. In addition, it is also conceivable that in a certain type of cell, a cell appears which ruptures when compressed. The cells can be sorted by using these as indices.

Another example of the manipulation includes observing a change in shape in the process of compressing the cell. According to the above-described flow, the cells are compressed by the bubble. For example, in the process of compressing the cell, the entire cell or various sites of the cell may be compressed while changing the pressure of compressing the cell, and the change in the shape of the cell may be imaged.

Another example of the manipulation includes rupture or cleavage due to the compression of the cell. By applying a large pressure to the cell, the cell can be ruptured or cleaved. By rupturing or cleaving the cell, the cell membrane, the cytoplasm, the organelle, and/or the like can be recovered, and connection (for example, a synapse that is a connection between nerve cells) between cells can be cut.

Another example of the manipulation includes induction of cell differentiation. It is known that differentiation of osteoblasts, myocytes, vascular endothelial progenitor cells, and the like is induced by giving mechanical stimulation. The differentiation can be induced by the pressure generation unit 47 compressing these cells by using the bubble according to the above-described flow.

Another example of the manipulation includes introducing a gene into the cell. According to the above-described flow, by using the bubble, the bubble forming unit 200 attaches a vesicle-shaped object such as a cell membrane to the gas-liquid interface 255 to be contact with the cell membrane, whereby the contents of the vesicle are taken into the cell by membrane fusion. At this time, by incorporating the gene in the vesicle, the gene can be taken into the cell. In addition, not only genes but also other macromolecules can be taken into the cell through a pore. In addition, according to the above-described flow, when the bubble forming unit 200 uses the bubble to compress the cell by the gas-liquid interface 255, a minute gap is likely to be generated in a part of the membrane in the process of deforming the cell. At this time, by adding the gene into the medium of the cell, the gene can be taken into the cell through the gap. In addition, not only genes but also other macromolecules can be taken into the cell through the gap.

Another example of the manipulation includes cooperation with an external device, for example, a cell culture device such as a fermenter or a cell analysis device such as a cell sorter. According to the above-described flow, the bubble forming unit 200 may move the cell by using the bubble to take the cell into the flow channel 51 and extract the cell to a designated position of the external device to cooperate with. In addition, the cell may be moved by directly connecting the flow channel 51 to the external device to send, to the external device, the cell taken into the flow channel 51.

Another example of the manipulation includes the manipulation of an emulsion. The emulsion is a droplet in an oil liquid or an oil droplet in an aqueous solution. In order to stabilize the formed emulsion, the emulsion may contain a surfactant or the like which is an amphipathic substance. The surfactant or the like are arranged to surround a droplet or an oil droplet, and form a monomolecular membrane on the interface. Such a monomolecular membrane is also found in a part of an intracellular organelle such as an endosome and a lipid droplet. According to the above-described flow, the bubble forming unit 200 may use the bubble to attach the emulsion to the gas-liquid interface 255, or may perform a further manipulation.

As a method of separating and/or recovering a cell, there are a method of locally denaturing a substrate to separate a cell by using a special substrate that reacts with temperature or light, and a method of separating a cell by ultrasonic waves, but these methods require a means of recovering the cell. However, the method of the present invention does not require a special substrate, and includes both the means for separating the cell and the means for recovering the cell. In addition, as a means for recovering the separated cell, there is a method of recovering the cell by a liquid flow, such as an aspirator, which sucks a liquid, but there is a possibility that the cell and a large amount of liquid are recovered simultaneously, or cells other than the target cell are involved. However, in the method of the present invention, the cell attached to the gas-liquid interface can be recovered by taking the gas-liquid interface into the nozzle, and the target cell can be easily recovered with a considerably small amount of liquid without involving cells other than the target cell. In addition, it is known that a strong liquid flow is applied at the time of sucking a liquid, so as to adversely affect cells, but such an adverse effect can be avoided by using the method of the present invention.

In the method of recovering the manipulation target 35 illustrated in Fig. 4, details of the method of separating the manipulation target 35 from the bottom portion 25a of the vessel 25 will be described below. Conventionally, when the manipulation target 35 is recovered, the vector of the force applied to the manipulation target 35 by the gas-liquid interface 255 has not been controlled. In the present embodiment, by controlling the vector of the force applied by the gas-liquid interface 255 to the manipulation target 35, the manipulation target 35 is separated from the inner bottom surface of the vessel 25, and the recovery of the manipulation target 35 is facilitated.

By executing the method in the present embodiment, not only the manipulation in which the manipulation target 35 is separated from the bottom portion 25a of the vessel 25 and recovered, but also other manipulations such as compressing the manipulation target 35 to recover cytoplasm and/or cell membranes, compressing and stimulating the manipulation target 35, compressing and rupturing or cleaving the manipulation target 35, compressing and observing the manipulation target 35, moving the manipulation target 35 to collect the manipulation target 35 in a partial region, and detaching the manipulation target 35 from the gas-liquid interface 255 can be performed, for example.

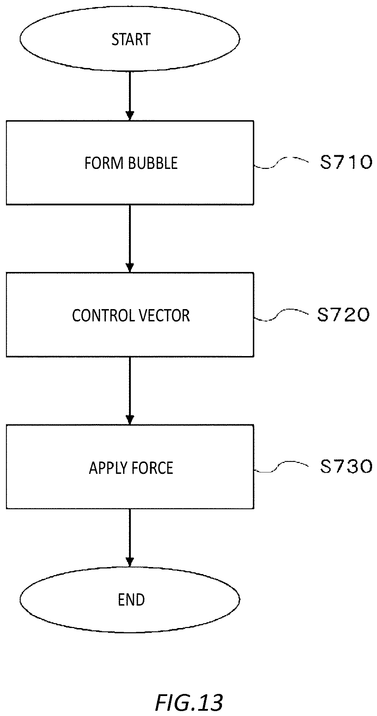

Fig. 13 is an example of a flow of controlling the vector of the force applied to the manipulation target 35 by the gas-liquid interface 255 in the present embodiment. In the present embodiment, by performing the processing of S710 to S730 in this order, the manipulation target 35 is separated from the inner bottom surface of the vessel 25, and the recovery of the manipulation target 35 is facilitated. However, the processing of S710 to S730 may not be performed in this order. For example, the processing of S720 may be performed before the processing of S710, or the processing of S710 and the processing of S720 may be simultaneously performed. In addition, S730 may be performed simultaneously with S720.

First, in S710, the end portion 254 of the nozzle 49 is arranged in the liquid 261 in which the manipulation target 35 is immersed, and gas is supplied to the flow channel 51 by a pump, thereby forming the bubble 256 in the flow channel 51 of the nozzle 49 or at the end portion 254 of the nozzle 49. By forming the bubble 256, the gas-liquid interface 255 between the liquid 261 and the gas is formed. The stage of forming the bubble 256 in the flow channel 51 of the nozzle 49 or at the end portion 254 of the nozzle 49 can be performed by the method described in the description of Fig. 2B or Fig. 4 above.

Next, in S720, the vector of the force applied from the gas-liquid interface 255 to the manipulation target 35 is controlled. In the present embodiment, the bubble forming unit 200 functions as a vector control unit which controls the vector of the force applied from the gas-liquid interface 255 to the manipulation target 35.

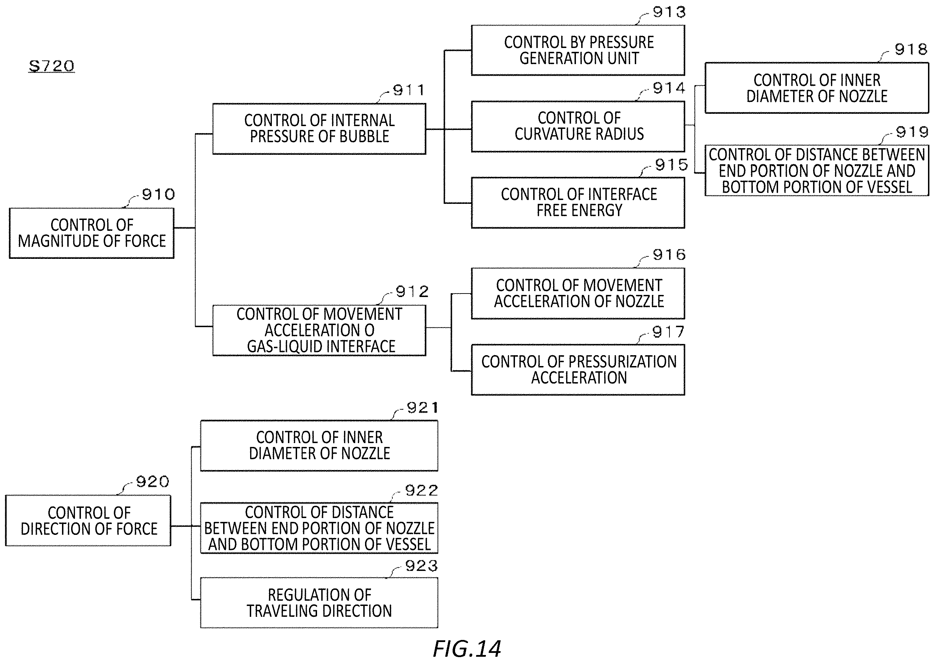

Fig. 14 illustrates an example of a method of controlling the vector of the force applied from the gas-liquid interface 255 to the manipulation target 35 in the present embodiment. As illustrated in Fig. 14, the method of controlling the vector of the force applied from the gas-liquid interface 255 to the manipulation target 35 includes a method 910 of controlling the magnitude of the force applied from the gas-liquid interface 255 to the manipulation target 35 and a method 920 of controlling the direction of the force applied from the gas-liquid interface 255 to the manipulation target 35.

The method 910 of controlling the magnitude of the force applied from the gas-liquid interface 255 to the manipulation target 35 includes a method 911 of controlling the internal pressure (gas pressure) of the bubble 256 forming the gas-liquid interface 255 and a method 912 of controlling the movement acceleration of the gas-liquid interface 255. Herein, the method 911 of controlling the internal pressure of the bubble 256 and the method 912 of controlling the movement acceleration of the gas-liquid interface 255 can be used in combination.

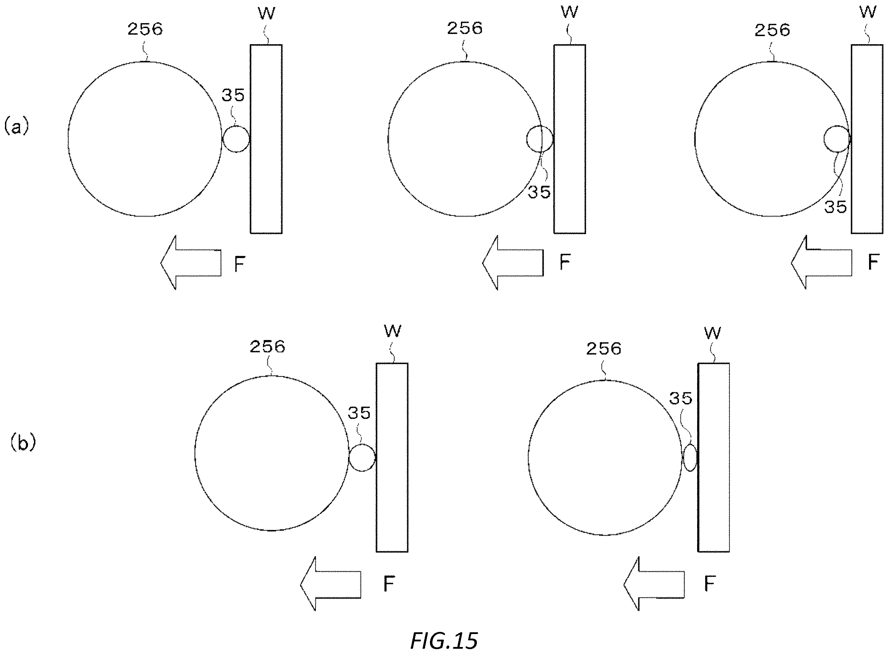

Fig. 15 illustrates an example of a schematic diagram for explaining the control of the internal pressure of the bubble 256 in the present embodiment. Figs. 15(a) and 15(b) illustrate a case where the manipulation target 35 is pushed back by the force of the size F when the bubble 256 presses the manipulation target 35 against a wall W. Fig. 15(a) illustrates a case where the force applied by the internal pressure of the bubble 256 is smaller than F, and Fig. 15(b) illustrates a case where the force applied by the internal pressure of the bubble 256 is larger than F. The method 911 of controlling the internal pressure of the bubble 256 will be described with reference to Figs. 15(a) and (b).

As illustrated in Fig. 15(a), in a case where the wall W is in the direction of pushing the manipulation target 35, when the force applied by the internal pressure of the bubble 256 is smaller than a force F pushed back by the manipulation target 35, the manipulation target 35 cannot be pushed by the bubble 256, and the manipulation target 35 enters the bubble 256. On the other hand, as illustrated in Fig. 15(b), when the force applied by the internal pressure of the bubble 256 is larger than the force F pushed back by the manipulation target 35, the bubble 256 can push the manipulation target 35, and the manipulation target 35 can be crushed to be deformed.

As described above, when the wall W is in the direction of pushing the manipulation target 35, the manipulation target 35 cannot be pushed with a force equal to or larger than the internal pressure of the bubble 256. Therefore, the internal pressure of the bubble 256 defines the maximum value of the force of pushing the manipulation target 35 when the wall W is in the direction of pushing the manipulation target 35. Thus, the internal pressure of the bubble 256 is controlled in order to control the maximum value of the force applied in the direction in which the gas-liquid interface 255 pushes the manipulation target 35.

Although a case where there is the wall W is assumed in Fig. 15 for understanding, the present invention can also be applied to understanding of a case where the manipulation target 35 adheres to the bottom portion 25a of the vessel 25. When the gas-liquid interface 255 of the bubble 256 presses the manipulation target 35 in the lateral direction, in a case where the maximum value of the force applied by the internal pressure of the bubble 256 is smaller than the adhering force of the manipulation target 35, the manipulation target 35 cannot be pushed by the bubble 256, and the manipulation target 35 enters the bubble 256. On the other hand, when the maximum value of the force applied by the internal pressure of the bubble 256 is larger than the adhering force of the manipulation target 35, the manipulation target 35 can be pushed by the bubble 256, and the manipulation target 35 can be pushed to be separated.

Returning to Fig. 14, the method of controlling the internal pressure of the bubble 256 includes a method 913 of controlling the internal pressure of the bubble 256 by the pressure generation unit 47, a method 914 of controlling the curvature radius at the portion where the gas-liquid interface 255 and the manipulation target 35 are in contact with each other, and a method 915 of controlling the interface free energy E2 of the gas-liquid interface 255. These methods 913 to 915 may be used in combination.

In the method 913 of controlling the internal pressure of the bubble 256 by the pressure generation unit 47, the end portion 254 of the flow channel 51 of the nozzle 49 is immersed in the liquid 261, and the gas supplied from the syringe pump is introduced into the liquid 261 from the end portion 254 of the flow channel 51. Then, the pressure generation unit 47 connected to the nozzle 49 reciprocates the plunger of the syringe pump such that the gas is supplied to the flow channel 51 of the nozzle 49 to perform pressurization or the gas is taken in from the flow channel 51 of the nozzle 49 to perform depressurization, thereby regulating the internal pressure (gas pressure) of the bubble 256.

The following Mathematical Formula 1 is a mathematical formula for explaining the method 914 of controlling the internal pressure of the bubble 256 by the curvature radius of the gas-liquid interface 255 and the method 915 of controlling the internal pressure by the interface free energy E2 of the gas-liquid interface 255. In the following Mathematical Formula 1, Pin represents the internal pressure of the bubble 256, Pout represents a liquid pressure, $\Delta$P represents the absolute value of a difference between the internal pressure of the bubble 256 and the liquid pressure, E2 represents the interface free energy (surface tension) of the gas-liquid interface 255, and r represents the curvature radius of the gas-liquid interface 255. When Pout is constant, the last equation of Mathematical Formula 1 is obtained.

$$[\text{Mathematical Formula 1}] \quad \Delta P = |\text{Pout} - \text{Pin}| = E2/r$$

$$\therefore \ \text{Pin} = E2/r$$

From Mathematical Formula 1, it can be seen that the internal pressure of the bubble 256 is proportional to the interface free energy E2 of the gas-liquid interface 255 and is inversely proportional to the curvature radius r of the gas-liquid interface 255. Therefore, the internal pressure of the bubble 256 can be controlled by controlling the interface free energy E2 of the gas-liquid interface 255 and the curvature radius r at the contact point of the gas-liquid interface 255 with the manipulation target 35.

Hereinafter, the method 914 of controlling the curvature radius r of the gas-liquid interface 255 will be described. As illustrated in Fig. 14, the method 914 of controlling the curvature radius r of the gas-liquid interface 255 includes a method 918 of controlling the inner diameter of the nozzle 49 and a method 919 of controlling a distance between the end portion 254 of the nozzle 49 and the bottom portion 25a of the vessel 25. These methods 918 and 919 can be used in combination. Note that the method 914 of controlling the curvature radius r of the gas-liquid interface 255 may be performed before the processing of S710 in Fig. 13.

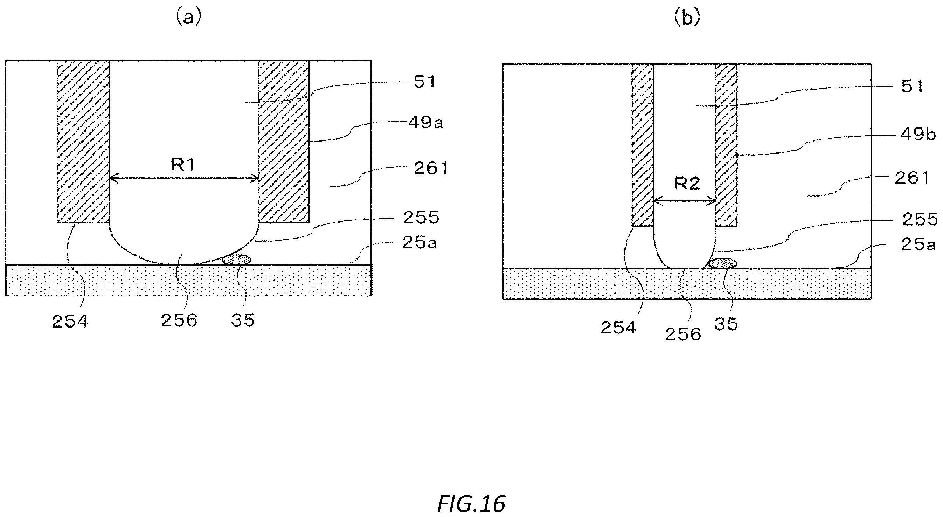

Fig. 16 illustrates an example of a schematic diagram illustrating a relationship between the inner diameter of the nozzle 49 and the curvature radius r of the gas-liquid interface 255 in the present embodiment. Fig. 16(a) illustrates a nozzle 49a having a large inner diameter R1, and Fig. 16(b) illustrates a nozzle 49b having a small inner diameter R2. Note that the inner diameter of the nozzle 49 indicates the internal diameter of the hollow cross section of the flow channel 51 of the nozzle 49. As illustrated in Fig. 16(a), it can be seen that the curvature radius r of the gas-liquid interface 255 increases when the nozzle 49a having a large inner diameter R1 is used, and as illustrated in Fig. 16(b), the curvature radius r of the gas-liquid interface 255 decreases when the nozzle 49b having a small inner diameter R2 is used. Note that although Fig. 16 illustrates an example in which the nozzle 49 has a cylindrical shape, that is, the cross-sectional shape of the nozzle 49 is a circle, the cross-sectional shape of the nozzle 49 may not be a circle. In such a case, the maximum inner diameter or the average inner diameter of the nozzle 49 correlates with the curvature radius r of the gas-liquid interface 255.

From the above, in the method 918 of controlling the inner diameter of the nozzle 49, the curvature radius r of the gas-liquid interface 255 can be controlled by preparing the nozzle 49 having the inner diameter corresponding to the maximum value of the pushing force to be applied to the manipulation target 35, and thus the maximum value of the pushing force to be applied to the manipulation target 35 can be controlled.

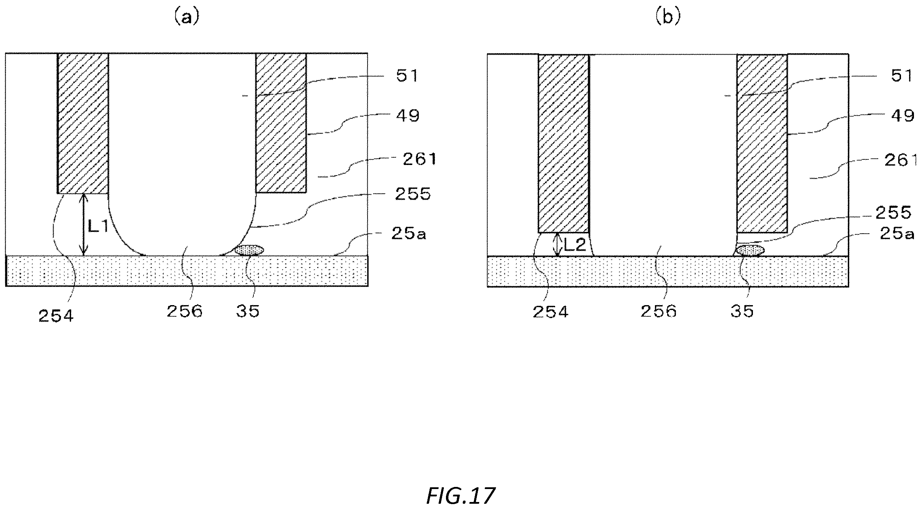

Fig. 17 illustrates an example of a schematic diagram illustrating a relationship between the distance between the end portion 254 of the nozzle 49 and the bottom portion 25a of the vessel 25 and the curvature radius r of the gas-liquid interface 255 in the present embodiment. Fig. 17(a) illustrates a case where a distance L1 between the end portion 254 of the nozzle 49 and the bottom portion 25a of the vessel 25 is long, and Fig. 17(b) illustrates a case where a distance L2 between the end portion 254 of the nozzle 49 and the bottom portion 25a of the vessel 25 is short. As illustrated in Fig. 17(a), when the distance L1 is long, the bubble 256 formed at the end portion 254 of the nozzle 49 is not sufficiently crushed, so that the curvature radius r of the gas-liquid interface 255 is large, and as illustrated in Fig. 17(b), when the distance L2 is short, the bubble 256 formed at the end portion 254 of the nozzle 49 is sufficiently crushed, so that the curvature radius r of the gas-liquid interface 255 is small. Note that when the distance L2 between the end portion 254 of the nozzle 49 and the bottom portion 25a of the vessel 25 is short, a relationship between the distance L2 and the curvature radius r of the gas-liquid interface 255 may be measured in advance and stored in the recording unit 190.

From the above, in the method 919 of controlling the distance between the end portion 254 of the nozzle 49 and the bottom portion 25a of the vessel 25, the curvature radius r of the gas-liquid interface 255 can be controlled by controlling the distance between the end portion 254 of the nozzle 49 and the bottom portion 25a of the vessel 25, and thus the maximum value of the force of pushing the manipulation target 35 can be controlled.

In the method 915 of controlling the interface free energy E2 of the bubble 256, as shown in the above Mathematical Formula 1, the internal pressure of the bubble 256 and the interface free energy E2 of the gas-liquid interface 255 are in a proportional relationship, and it can be seen that the internal pressure of the bubble 256 increases by increasing the interface free energy E2 of the gas-liquid interface 255 between the gas and the liquid 261, and the internal pressure of the bubble 256 decreases by decreasing the interface free energy E2. Accordingly, the magnitude of the force applied from the gas-liquid interface 255 to the manipulation target 35 can be controlled.

The control of the interface free energy E2 of the gas-liquid interface 255 can be adjusted by the energy control unit changing the type and composition of a solute. For example, the interface free energy E2 can be increased by adding an inorganic salt as the solute to the liquid, and the interface free energy E2 can be decreased by adding a polar organic compound or a surfactant as the solute.

In addition, the magnitude of the interface free energy E2 of the gas-liquid interface 255 may be controlled by adding another liquid to the liquid in the vessel 25. When the solution is a complete medium, a buffer solution, a basic medium, or water may be added as the another liquid. In this case, the concentration of the solute contained in the complete medium is changed by adding the another liquid. Therefore, the interface free energy E2 of the gas-liquid interface 255 can be adjusted.

The control of the interface free energy E2 of the gas-liquid interface 255 can be adjusted by the energy control unit changing the type and composition of the gas.

Furthermore, the magnitude of the interface free energy E2 may be adjusted by removing an inorganic salt or a polar organic compound from the solution. The removal of the inorganic salt may be performed by adding a chelating agent such as EDTA to the solution. The removal of the polar organic compound or surfactant may be performed by inserting a substrate such as a filter, a column, or a bead into the solution so that the substrate adsorbs the polar organic compound or surfactant in the solution.

Hereinafter, the method 912 of controlling the movement acceleration of the gas-liquid interface 255 will be described. The force applied to the manipulation target 35 is a product of the mass of the manipulation target 35 and the movement acceleration of the gas-liquid interface 255 to which the manipulation target 35 is attached. That is, the force applied to the manipulation target 35 attached to the gas-liquid interface 255 is proportional to the movement acceleration of the gas-liquid interface 255 to which the manipulation target 35 is attached. Therefore, the magnitude of the force applied from the gas-liquid interface 255 to the manipulation target 35 can be controlled by controlling the movement acceleration of the gas-liquid interface 255. Originally, the sum of the products of the mass and the acceleration in each minute portion of the manipulation target 35 becomes the force applied to the manipulation target 35, and the force to be accurately applied is calculated by considering the normal force and the frictional force applied to the manipulation target 35. On the other hand, the manipulation target 35 is considerably small, and thus even when these influences are ignored and the above simple idea is used, a result in which the theory and the effect are matched can be obtained.

Returning to FIG 14, the method 912 of controlling the movement acceleration of the gas-liquid interface 255 includes a method 916 of controlling the acceleration of moving the nozzle 49 and a method 917 of controlling the pressurization acceleration of the syringe pump. These methods 916 and 917 can be used in combination.

In the method 916 of controlling the acceleration of moving the nozzle 49, the movement acceleration of the gas-liquid interface 255 is controlled by controlling the movement acceleration of the flow channel 51 of the nozzle 49 in which the bubble 256 is formed. The bubble forming unit 200 decides the movement acceleration of the nozzle actuator 40 which operates the nozzle 49, and sends an instruction to the nozzle actuator 40 to operate at the decided movement acceleration. The nozzle actuator 40 operates the nozzle 49 at the decided movement acceleration.

In the method 917 of controlling the pressurization acceleration of the syringe pump, the movement acceleration of the gas-liquid interface 255 is controlled by controlling the acceleration of the gas supply from or gas intake to the syringe pump. In this case, the pressure generation unit 47 of the bubble forming unit 200 controls the plunger to control the acceleration at which gas is supplied from the syringe pump or the acceleration at which gas is taken into the syringe pump. Accordingly, the movement acceleration of the gas-liquid interface 255 is controlled by controlling the pressurization acceleration of the bubble 256 formed at the end portion 254 of the nozzle 49.

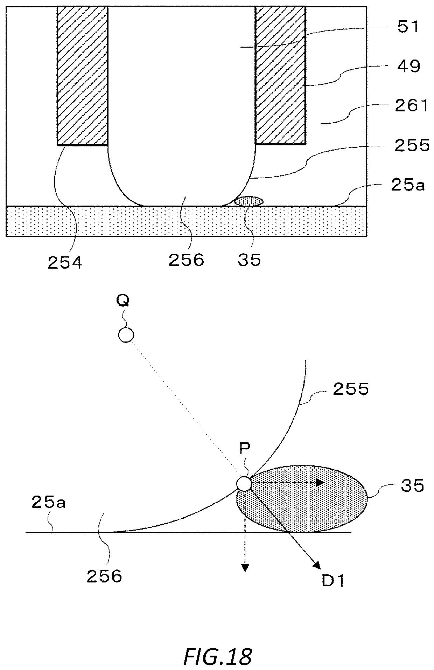

Fig. 18 illustrates an example of a schematic diagram for explaining the direction of the force applied from the gas-liquid interface 255 to the manipulation target 35 in the present embodiment. Note that the lower part of Fig. 18 is a partially enlarged diagram of the vicinity of the manipulation target 35 in the upper part of Fig. 18. The method 920 of controlling the direction of the force applied from the gas-liquid interface 255 to the manipulation target 35 will be described with reference to Fig. 18. As illustrated in Fig. 18, the direction of the force applied from the gas-liquid interface 255 to the manipulation target 35 is a direction along a straight line connecting a contact point P at which the gas-liquid interface 255 and the manipulation target 35 are in contact with each other and a center point Q. of a circle when a curve of the gas-liquid interface 255 at the contact point P is assumed to be the circle, and is decided depending on whether the gas-liquid interface 255 moves from the gas to the liquid side or from the liquid to the gas side. Therefore, in the vector control stage of S720, the direction of the force applied from the gas-liquid interface 255 to the manipulation target 35 can be controlled by controlling the direction of the surface and the moving direction of the surface at the contact point P at which the gas-liquid interface 255 and the manipulation target 35 are in contact with each other. Note that the vector control stage of S720 in the present embodiment may include forming and maintaining the bubble 256 at the end portion 254 of the nozzle 49.

In the example illustrated in Fig. 18, the gas-liquid interface 255 pushes the manipulation target 35 in a lower right direction, and thus a force is applied from the gas-liquid interface 255 to the manipulation target 35 in a direction D1. That is, the direction of the force applied from the gas-liquid interface 255 to the manipulation target 35 is set from the gas side to the liquid side of the gas-liquid interface 255. However, when the gas-liquid interface 255 pulls the manipulation target 35 while the manipulation target 35 is attached to the gas-liquid interface 255, contrary to the above, the direction of the force applied from the gas-liquid interface 255 to the manipulation target 35 is set from the liquid side to the gas side of the gas-liquid interface 255. In addition, the example illustrated in Fig. 18 is described with an assumption that the gas-liquid interface 255 is stationary, but when the gas-liquid interface 255 moves in a specific direction, the direction of the force applied from the gas-liquid interface 255 to the manipulation target 35 is determined by the direction of the surface at the contact point P at which the gas-liquid interface 255 and the manipulation target 35 come into contact with each other and the moving direction of the gas-liquid interface 255. For example, when the gas-liquid interface 255 moves in the right direction in Fig. 18, the direction of the force applied from the gas-liquid interface 255 to the manipulation target 35 has a large component in the right direction, and is a slightly lateral direction as compared with the direction D1.

Returning to Fig. 14, the method 920 of controlling the direction of the force applied to the manipulation target 35 from the gas-liquid interface 255 includes a method 921 of controlling the inner diameter of the nozzle 49, a method 922 of controlling the distance between the end portion 254 of the nozzle 49 and the bottom portion 25a of the vessel 25, and a method 923 of adjusting the traveling direction of the gas-liquid interface 255. These methods 921 to 923 can be used in combination.

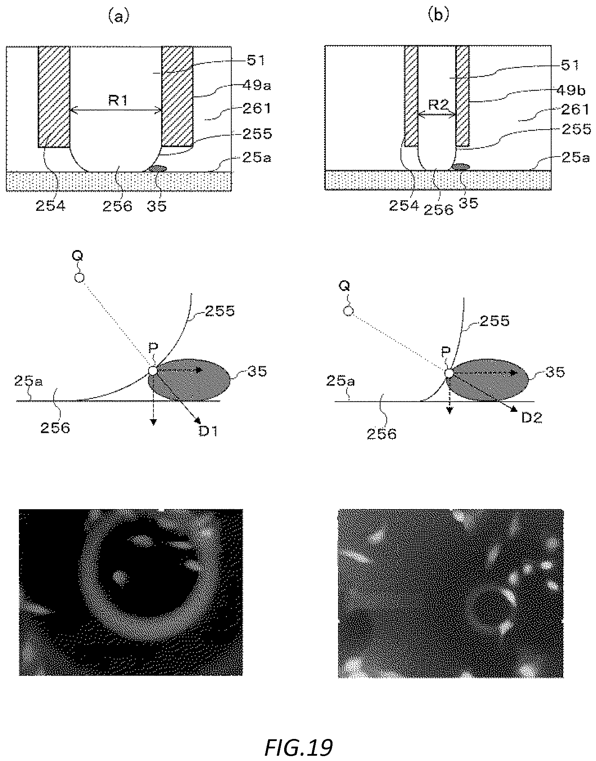

Fig. 19 illustrates an example of a schematic diagram for explaining the method 921 of controlling the direction of the force by controlling the inner diameter of the nozzle 49 in the present embodiment. Fig. 19(a) illustrates a case where the nozzle 49a having the large inner diameter R1 is used, and Fig. 19(b) illustrates a case where the nozzle 49b having the small inner diameter R2 is used. Note that the middle diagrams in Figs. 19(a) and 19(b) are partial enlarged diagrams of the vicinity of the manipulation target 35 in the upper diagrams in Figs. 19(a) and 19(b), and the lower diagrams in Figs. 19(a) and 19(b) illustrate experimental images of the manipulation target 35.

As illustrated in Fig. 19(a), when the nozzle 49a having the large inner diameter R1 is used, the curvature radius r at the contact point P of the bubble 256 becomes large, and the straight line connecting with the center point Q. of the circle becomes relatively downward. Therefore, the gas-liquid interface 255 comes into contact with the manipulation target 35 from a slightly upper direction. Thus, when the gas-liquid interface 255 moves from the gas toward the liquid side, the direction in which the gas-liquid interface 255 applies a force to the manipulation target 35 is the direction D1. In this case, as illustrated in the experimental image in the lower diagram of Fig. 19(a), the manipulation target 35 enters under the bubble 256 without separation and is compressed. On the other hand, as illustrated in Fig. 19(b), when the nozzle 49b having the small inner diameter R2 is used, the curvature radius r at the contact point P of the bubble 256 becomes small, and the straight line connecting with the center point Q. of the circle becomes relatively lateral. Therefore, the gas-liquid interface 255 comes into contact with the manipulation target 35 from a slightly lateral direction. Thus, when the gas-liquid interface 255 moves from the gas toward the liquid side, the direction in which the gas-liquid interface 255 applies a force to the manipulation target 35 is the direction D2. In this case, as shown in the experimental image in the lower diagram of Fig. 19(b), the manipulation target 35 is separated from the bottom portion 25a of the vessel 25. Note that the direction of the force of the gas-liquid interface 255 pushing the manipulation target 35 is also determined by the magnitude relationship between the inner diameter of the nozzle 49 and the diameter of the manipulation target 35, and for example, when the inner diameter of the nozzle 49 and the diameter of the manipulation target 35 are substantially the same, the direction of the force of the gas-liquid interface 255 pushing the manipulation target 35 is a relatively lateral direction.

As described above, when the nozzle 49a having the large inner diameter R1 is used, a force is applied in the direction D1 in which the manipulation target 35 is crushed from above, and when the nozzle 49b having the small inner diameter R2 is used, a force is applied slightly in the lateral direction D2 with respect to the manipulation target 35, so that the manipulation target can be separated from the bottom portion 25a. As described above, by preparing the nozzle 49 having the inner diameter corresponding to the magnitude of the force to be applied to the manipulation target 35, it is possible to control the curvature radius r at the contact point P where the gas-liquid interface 255 and the manipulation target 35 are in contact with each other, to control the direction of the surface and the moving direction of the surface at the contact point P at which the gas-liquid interface 255 and the manipulation target 35 are in contact with each other, and to control the direction of the force applied to the manipulation target 35. Note that the method 921 of controlling the inner diameter of the nozzle 49 may be performed before the processing of S710 in Fig. 13.

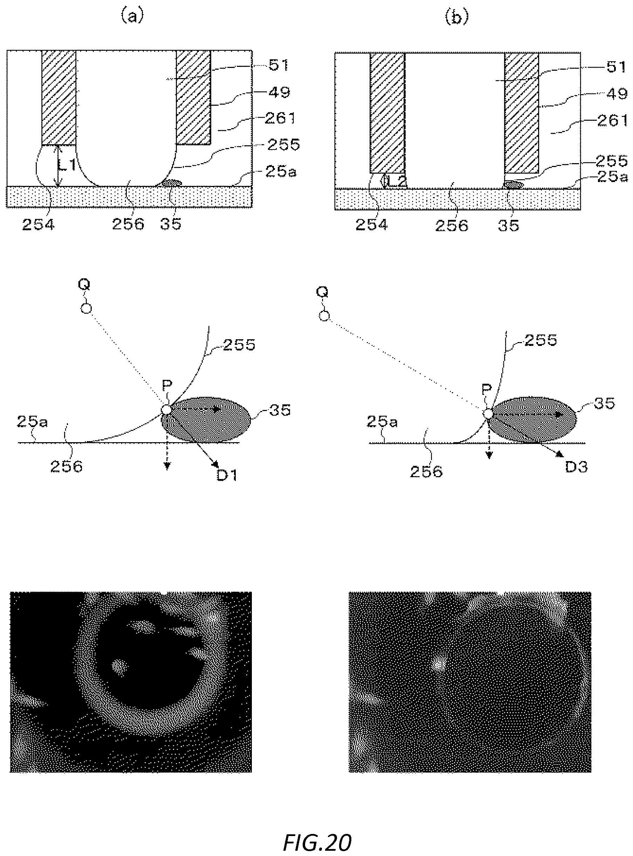

Fig. 20 illustrates an example of a schematic diagram for explaining the method 922 of controlling the direction of force by controlling the distance between the end portion 254 of the nozzle 49 and the bottom portion 25a of the vessel 25 in the present embodiment. Fig. 20(a) illustrates a case where the distance L1 between the end portion 254 of the nozzle 49 and the bottom portion 25a of the vessel 25 is long, and Fig. 20(b) illustrates a case where the distance L2 between the end portion 254 of the nozzle 49 and the bottom portion 25a of the vessel 25 is short. Note that the middle diagrams in Figs. 20(a) and 20(b) are partial enlarged diagrams of the vicinity of the manipulation target 35 in the upper diagrams in Figs. 20(a) and 20(b), and the lower diagrams in Figs. 20(a) and 20(b) illustrate experimental images of the manipulation target 35.

As illustrated in Fig. 20(a), when the distance L1 is long, the bubble 256 is not sufficiently crushed, the curvature radius r at the contact point P of the bubble 256 becomes large, and the straight line connecting with the center point Q. of the circle becomes relatively downward. Therefore, the gas-liquid interface 255 comes into contact with the manipulation target 35 from a slightly upper direction. Thus, when the gas-liquid interface 255 moves from the gas toward the liquid side, the direction in which the gas-liquid interface 255 applies a force to the manipulation target 35 is the direction D1. In this case, as illustrated in the experimental image in the lower diagram of Fig. 20(a), the manipulation target 35 enters under the bubble 256 without separation and is compressed. On the other hand, as illustrated in Fig. 20(b), when the distance L2 is short, the bubble 256 is sufficiently crushed, the curvature radius r at the contact point P of the bubble 256 becomes small, and the straight line connecting with the center point Q. of the circle becomes relatively lateral. Therefore, the gas-liquid interface 255 comes into contact with the manipulation target 35 from a slightly lateral direction. Thus, when the gas-liquid interface 255 moves from the gas toward the liquid side, the direction in which the gas-liquid interface 255 applies a force to the manipulation target 35 is the direction D3. In this case, as shown in the experimental image in the lower diagram of Fig. 20(b), the manipulation target 35 is separated from the bottom portion 25a of the vessel 25.

As described above, when the distance L1 between the end portion 254 of the nozzle 49 and the bottom portion 25a of the vessel 25 is long, a force is applied in a form of crushing the manipulation target 35 from above, and when the distance L2 is short, a force can be applied slightly laterally to the manipulation target 35. Therefore, the nozzle position control unit can move the flow channel 51 or the vessel 25 to a position at a first distance which is the distance allowing the gas-liquid interface 255 to come into contact with the bottom portion 25a of the vessel 25, and can move the flow channel 51 to a second position at which the distance between the end portion 254 and the bottom portion 25a of the vessel 25 is shorter than the first distance in a state where the gas-liquid interface 255 formed at the end portion 254 by the syringe pump is brought into contact with the bottom portion 25a of the vessel 25. As described above, by controlling the distance between the end portion 254 of the nozzle 49 and the bottom portion 25a of the vessel 25, it is possible to control the curvature radius r at the contact point P where the gas-liquid interface 255 and the manipulation target 35 are in contact with each other, control the direction of the surface and the moving direction of the surface at the contact point P, and control the direction of the force applied to the manipulation target 35.

In the method 923 of regulating the traveling direction of the gas-liquid interface 255, the nozzle actuator 40 regulates the traveling direction of the gas-liquid interface 255 such that the nozzle 49 is operated in a desired direction to control the direction of the force applied to the manipulation target 35. For example, by moving the nozzle 49 in the right direction in a state where the gas-liquid interface 255 and the manipulation target 35 are in contact with each other, the direction of the force applied to the manipulation target 35 by the gas-liquid interface 255 can be made slightly rightward. In addition, by moving the nozzle 49 downward in a state where the gas-liquid interface 255 and the manipulation target 35 are in contact with each other, the direction of the force applied to the manipulation target 35 by the gas-liquid interface 255 can be made slightly downward. The traveling direction of the gas-liquid interface 255 can also be regulated by changing the pressure of the syringe pump to move the gas-liquid interface 255.

As described above, the control of the vector of the force applied from the gas-liquid interface 255 to the manipulation target 35 illustrated in S720 of Fig. 13 ends. Subsequently, the processing advances to the stage of applying a force to the manipulation target 35 in S730 of Fig. 13. In the stage of applying a force to the manipulation target 35 in S730, a force is applied to the manipulation target 35 on the basis of the vector of force applied to the manipulation target 35 decided in S720. The force application to the manipulation target 35 is performed by the nozzle actuator 40 of the bubble forming unit 200 operating the nozzle 49 and/or the stage to move the gas-liquid interface 255 relative to the bottom portion 25a of the vessel 25. In addition, the pressure generation unit 47 connected to the nozzle 49 may reciprocate the plunger of the syringe pump to supply or take in gas with respect to the bubble 256, thereby applying a force to the manipulation target 35.

Fig. 21 illustrates an example of a schematic diagram illustrating a relationship among the inner diameter of the nozzle 49, the distance between the end portion 254 of the nozzle 49 and the bottom portion 25a of the vessel 25, the curvature radius of the gas-liquid interface 255, the maximum value of the force applied to the cell to be the manipulation target 35, the magnitude of a force (compression force) of a downward component, the magnitude of a force (separating force) of a lateral component, the area of the gas-liquid interface 255 on which the cell to be the manipulation target 35 can be attached, the controllability of the nozzle 49, the number of target cells, and the cell adhesion alleviation processing in the present embodiment. Regarding the compression force and the separating force, it is assumed that the gas-liquid interface 255 is moved so that the cell is pushed in a state where the manipulation target 35 is in contact with the bottom portion 25a. As illustrated in Fig. 21, in a case where the inner diameter of the nozzle 49 is large, and the distance between the end portion 254 of the nozzle 49 and the bottom portion 25a of the vessel 25 is long, when the cell which is the manipulation target 35 is tried to be pushed, the component of the compression force against the cell becomes large, and the component of the separating force becomes small, which results in compressing the cell from above, and in order to separate the cell, it is necessary to perform strong cell adhesion alleviation processing in advance to alleviate the cell adhesion.

On the other hand, in a case where the inner diameter of the nozzle 49 is large, and the distance between the end portion 254 of the nozzle 49 and the bottom portion 25a of the vessel 25 is short, when the cell which is the manipulation target 35 is tried to be pushed, the component of the compression force against the cell becomes small, and the component of the separating force becomes large, which results in pushing the cell in the lateral direction, and in order to separate the cell, weak cell adhesion alleviation processing may be performed in advance, or the cell adhesion alleviation processing becomes unnecessary.

In addition, even in a case where the inner diameter of the nozzle 49 is small, and the distance between the end portion 254 of the nozzle 49 and the bottom portion 25a of the vessel 25 is short, when the cell which is the manipulation target 35 is tried to be pushed, the component of the compression force against the cell is small, and the component of the separating force is large, which results in pushing the cell in the lateral direction, and in order to separate the cell, weak cell adhesion alleviation processing may be performed in advance, or the cell adhesion alleviation processing becomes unnecessary.

As described above, by controlling the vector of the force applied to the manipulation target 35 by the gas-liquid interface 255, for example, the lateral force is effectively applied to the manipulation target 35 to separate the manipulation target 35 from the bottom portion 25a of the vessel 25, and the recovery of the manipulation target 35 can be facilitated. In addition, by increasing the component of the downward force on the manipulation target 35 in the process of moving the gas-liquid interface 255 to push the cell, the manipulation target 35 can be compressed from above, and the cell as the manipulation target 35 can be observed in the state of being compressed from above. At this time, the maximum value of the force applied to the manipulation target 35 is the internal pressure of the bubble 256, and thus, for example, by performing observation while monitoring the internal pressure of the bubble 256, the force applied to the manipulation target 35 and the change in the observation image can be easily captured in real time. Furthermore, when it is desired to observe the manipulation target 35 in the state of being compressed from directly above, the gas-liquid interface 255 may be brought into contact with the manipulation target 35 from directly above to compress the manipulation target 35 from directly above. In this case, control is performed such that the vicinity of the apex of the bubble 256 comes into contact with the specific manipulation target 35.

The vector control stage in S720 may include control of moving the gas-liquid interface 255 to be in contact with a predetermined manipulation target 35. In this case, the bubble forming unit 200 acquires the horizontal position (XY position) of the gas-liquid interface 255 and the target manipulation target 35 from the image captured by the camera 60 or the camera 70, acquires the vertical position (Z position) of the gas-liquid interface 255 and the target manipulation target 35 from the image captured by the flow channel imaging camera 42, and specifies the relative positional relationship between the gas-liquid interface 255 and the target manipulation target 35. Subsequently, on the basis of the specified relative positional relationship, the bubble forming unit 200 causes the nozzle actuator 40 to move the nozzle 49 and/or the stage so that the gas-liquid interface 255 is brought into contact with the target manipulation target 35. In addition, the manipulator may input the operation amount of the nozzle 49 and/or the stage on the basis of the specified relative positional relationship to move the nozzle 49 and/or the stage so that the gas-liquid interface 255 is brought into contact with the target manipulation target 35.

In addition, the vector control stage of S720 may be performed while an ultra-small pressure gauge is provided at the bottom portion 25a of the vessel 25 accommodating the liquid 261, and the pressure from the gas-liquid interface 255 is detected by the ultra-small pressure gauge. In this case, the sensor unit 48 detects the pressure generated in the bottom portion 25a of the vessel 25, and the sensor unit 48 sends this information to the bubble forming unit 200. The bubble forming unit 200 may perform feedback control of the pressure generation unit 47 on the basis of the detected pressure.

In addition, the bubble forming unit 200 may acquire a contact image between the gas-liquid interface 255 and the manipulation target 35 by at least one of the cameras 60 and 70 and the flow channel imaging camera 42, grasp a contact situation between the gas-liquid interface 255 and the manipulation target 35, and estimate the direction of the force applied to the manipulation target 35 by the gas-liquid interface 255. The bubble forming unit 200 may move the nozzle 49 and/or the stage on the basis of the estimated force direction and perform feedback control such that the force direction becomes a predetermined direction.

For example, when the contact image illustrated in the lower diagram of Fig. 19(a) is acquired, and the gas-liquid interface 255 moves from the gas toward the liquid side, it is estimated that the direction of the force applied to the manipulation target 35 by the gas-liquid interface 255 is the direction D1. Herein, when it is desired to separate the manipulation target 35 from the bottom portion 25a of the vessel 25 by applying a lateral force to the manipulation target 35, the direction D1 has a slightly large downward component and compresses the manipulation target 35 from above, which is not a desirable direction. In this case, by moving the nozzle 49 in the right direction of Fig. 19(a) in a state where the gas-liquid interface 255 and the manipulation target 35 are in contact with each other, the direction of the force applied to the manipulation target 35 by the gas-liquid interface 255 can be made slightly rightward, and can be brought close to the direction D2 in the lower diagram of Fig. 19(b). Accordingly, the manipulation target 35 can be separated from the bottom portion 25a of the vessel 25 by effectively applying the lateral force to the manipulation target 35. In addition, in the state illustrated in Fig. 19(b), by moving the nozzle 49 in the right direction, the direction of the force applied to the manipulation target 35 by the gas-liquid interface 255 can be made further rightward from the direction D2.

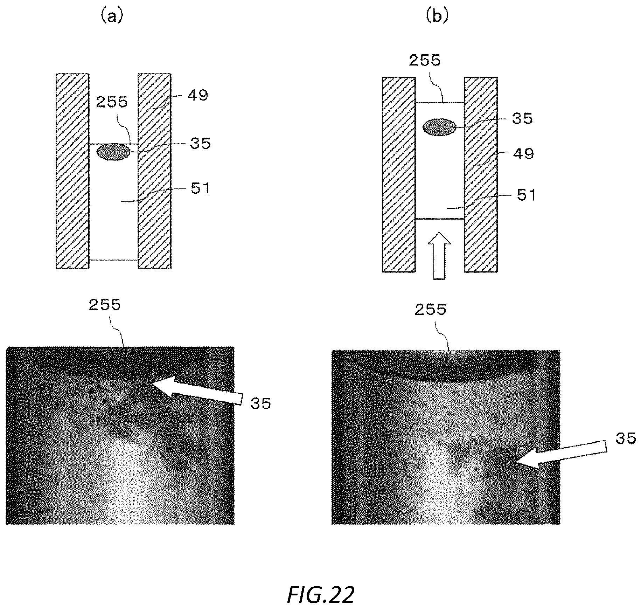

Fig. 22 is a diagram for explaining detachment of the manipulation target 35 from the gas-liquid interface 255 in the present embodiment. The lower diagrams in Figs. 22(a) and 22(b) illustrate the experimental images of the upper diagrams in Figs. 22(a) and 22(b). By controlling the vector of the force applied to the manipulation target 35 by the gas-liquid interface 255, the manipulation target 35 attached to the gas-liquid interface 255 can be detached. As illustrated in Figs. 22(a) and 22(b), the bubble forming unit 200 takes in the gas by controlling the pressure generation unit 47, so that the gas-liquid interface 255 can be moved upward in Figs. 22(a) and 22(b) from the liquid toward the gas side to detach the manipulation target 35 from the gas-liquid interface 255. In the lower diagram of Fig. 22(a), the manipulation target 35 attached to the gas-liquid interface 255 is detached from the gas-liquid interface 255 in the lower diagram of Fig. 22(b).

In addition, in a state where the manipulation target 35 is attached to the gas-liquid interface 255, the direction of the surface and the moving direction of the surface at the contact point may be controlled to apply a force to the manipulation target 35 in a specific direction (movement from the liquid toward the gas side). When the force of pulling the manipulation target 35 is larger than the force of attaching the manipulation target 35 to the gas-liquid interface 255, the manipulation target 35 is detached from the gas-liquid interface 255. For example, the pressure generation unit 47 connected to the nozzle 49 pulls the plunger of the syringe pump to take in gas, and moves the gas-liquid interface 255, to which the manipulation target 35 is attached, from the liquid toward the gas side, so that the manipulation target 35 can be detached from the gas-liquid interface 255. In addition, when the pressure generation unit 47 reciprocates the plunger of the syringe pump, a pulling force is effectively applied to the manipulation target 35, and the manipulation target 35 attached to the gas-liquid interface 255 can be detached from the gas-liquid interface 255. In addition, the nozzle 49 may be moved to vibrate the gas-liquid interface 255 to which the manipulation target 35 is attached. Accordingly, a pulling force is effectively applied to the manipulation target 35, and the manipulation target 35 attached to the gas-liquid interface 255 can be detached from the gas-liquid interface 255.

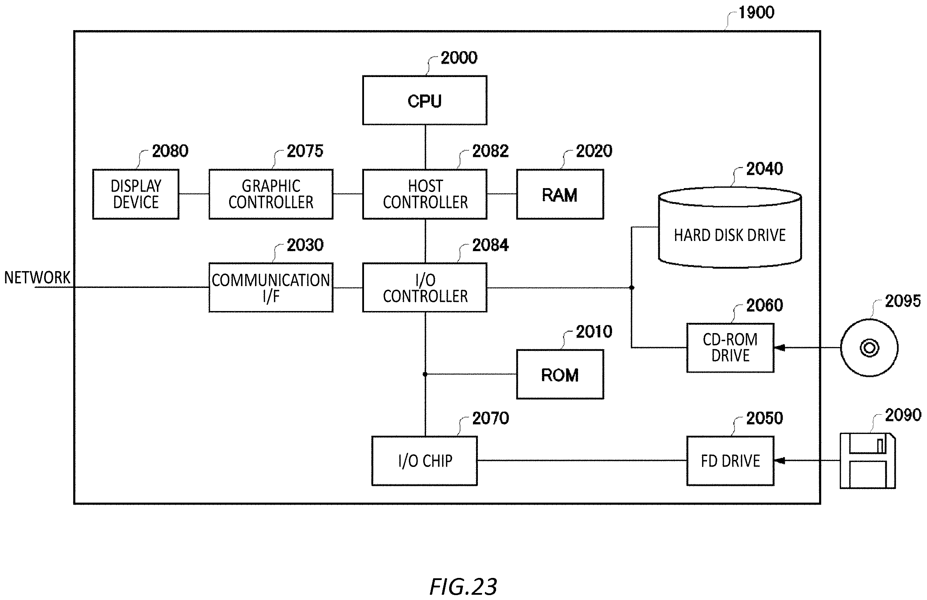

Fig. 23 illustrates an example of a hardware configuration of a computer 1900 which functions as the information processing device 170. The computer 1900 according to the present embodiment includes: a CPU-peripheral portion having a CPU 2000, a RAM 2020, a graphics controller 2075, and a display device 2080 interconnected by a host controller 2082; and an input/output unit having communication interface 2030, a hard disk drive 2040, and a CD-ROM drive 2060 connected to the host controller 2082 by an input/output controller 2084; and a legacy input/output unit having a ROM 2010, a flexible disk drive 2050, and an input/output chip 2070 connected to the input/output controller 2084.

The host controller 2082 connects the RAM 2020 with the CPU 2000 and the graphic controller 2075 accessing the RAM 2020 at a high transfer rate. The CPU 2000 operates on the basis of programs stored in the ROM 2010 and the RAM 2020, and controls each unit. The graphics controller 2075 is configured to acquire image data generated by the CPU 2000 or the like on a frame buffer provided inside the RAM 2020 and display the image data on the display device 2080. Alternatively, the graphics controller 2075 may include therein a frame buffer storing the image data generated by the CPU 2000 or the like. The display device 2080 can display various types of information (for example, an image, position information of the manipulation target 35, and the like) generated inside the information processing device 170.

The input/output controller 2084 connects the communication interface 2030, the hard disk drive 2040, and the CD-ROM drive 2060 which are relatively fast input/output devices to the host controller 2082. The communication interface 2030 is configured to communicate with other devices via a network by wire or wirelessly. In addition, the communication interface is configured to function as a hardware to perform communications. The hard disk drive 2040 stores a program and data to be used by the CPU 2000 in the computer 1900. The CD-ROM drive 2060 is configured to read a program or data from the CD-ROM 2095 and provide the hard disk drive 2040 through the RAM 2020.

In addition, the ROM 2010, and the flexible disk drive 2050 and input/output chip 2070, which are relatively low-speed input/output devices, are connected to the input/output controller 2084. The ROM 2010 stores a boot program performed when the computer 1900 starts up, and/or a program relying on the hardware of the computer 1900, and the like. The flexible disk drive 2050 is configured to read out a program or data from the flexible disk 2090, and provide it to the hard disk drive 2040 via the RAM 2020. The input/output chip 2070 connects the flexible disk drive 2050 to the input/output controller 2084, and connects various types of input/output devices to the input/output controller 2084, for example, via a parallel port, a serial port, a keyboard port, a mouse port, or the like.

The program provided to the hard disk drive 2040 via the RAM 2020 is stored in a recording medium, such as the flexible disk 2090, the CD-ROM 2095, or an IC card, and provided by a user. The program is read out from the recording medium, installed on the hard disk drive 2040 in the computer 1900 via the RAM 2020, and executed in the CPU 2000.

A program installed in the computer 1900 and causing the computer 1900 to function as the information processing device 170 includes a bubble forming module, an energy control module, and a manipulation module. These programs or modules may work on the CPU 2000 or the like to cause the computer 1900 to function as the bubble forming unit 200, the liquid control unit 260, or the like.

The information processing described in these programs is read by the computer 1900 to cause the computer to function as the bubble forming unit 200, the liquid control unit 260, or the like which is a specific means realized by cooperation of software and the various types of hardware resources described above. These specific means implement operations or processing of information corresponding to the intended use of the computer 1900 in the present embodiment, and the information processing device 170 is thereby constructed to be specific for the intended use.

As an example, when communication is performed between the computer 1900 and an external device or the like, the CPU 2000 is configured to execute the communication program loaded on the RAM 2020, and provide the communication interface 2030 with communication processing instructions on the basis of the content of the process written in the communication program. In response to the control by the CPU 2000, the communication interface 2030 is configured to read out the transmission data stored in the transmission buffer region or the like provided on the storage device, such as the RAM 2020, the hard disk drive 2040, the flexible disk 2090, the CD-ROM 2095, or the like, and transmit this transmission data to the network, and write reception data received from the network onto a reception buffer region or the like provided on the storage device. In this way, the communication interface 2030 may transfer transmission/reception data to the storage device through DMA (Direct Memory Access) scheme, and alternatively, the CPU 2000 may transfer the transmission/reception data by reading the data from the storage device or communication interface 2030 that are the origins of transfer, and writing the data onto the communication interface 2030 or the storage device that are the destinations of transfer.

In addition, the CPU 2000 causes all or necessary portions of files or database stored in an external storage device such as the hard disk drive 2040, the CD-ROM drive 2060 (CD-ROM 2095), and the flexible disk drive 2050 (flexible disk 2090) to be read into the RAM 2020 by means of DMA transfer or the like, and then performs various types of processing on the data in the RAM 2020. The CPU 2000 writes back the data on which processing is completed into an external storage device by DMA transfer or the like. In such processing, the RAM 2020 can be regarded as carrying contents of the external storage device temporarily, and thus the RAM 2020, the external storage device and the like are collectively called a memory, a recording unit, a storage device or the like in the present embodiment.

Herein, the storage device or the like stores information necessary for information processing of the information processing device 170, for example, moving image data or the like as necessary, and supplies the information to each component of the information processing device 170 as necessary.

Various types of information such as various types of programs, data, tables, databases or the like in the present embodiment according to the present embodiment are stored on such a storage device, and are subjected to information processing. Note that, the CPU 2000 can retain a part of the RAM 2020 in a cache memory and read from or write to the cache memory. In such a configuration as well, the cache memory serves a part of the function of the RAM 2020, and therefore the cache memory is also included with the RAM 2020, the memory, and/or the storage device in the present embodiment, except when it is shown with distinction.

In addition, the CPU 2000 is configured to execute various types of processing including various types of computations, information processing, conditional determination, information search/replacement, or the like described in the present embodiment for the data read from the RAM 2020, as specified by the instruction sequence of the program, and writes the result back onto the RAM 2020. For example, when performing conditional determination, the CPU 2000 compares various types of variables shown in the present embodiment to determine whether they satisfy conditions such as being larger than, smaller than, equal to or greater than, less than or equal to, equal to or like other variables or constants, and if a condition is satisfied (or if it is not satisfied) branches to a different instruction sequence or calls up a subroutine.

In addition, the CPU 2000 can search for information stored in a file in the storage device or the database, or the like. For example, if a plurality of entries, each having an attribute value of a second attribute associated with an attribute value of a first attribute, are stored in a storage device, the CPU 2000 searches, from among the plurality of entries stored in the storage device, an entry having an attribute value of the first attribute that matches a specified condition, and reads out the attribute value of the second attribute stored in the entry, and it is thereby possible to obtain the attribute value of the second attribute associated with the first attribute that satisfies a predetermined condition.

The programs or modules shown above may also be stored in an external recording medium. As a recording medium, other than the flexible disk 2090 and the CD-ROM 2095, an optical recording medium such as DVD or CD, a magneto-optical recording medium such as MO, a tape medium, a semiconductor memory, such as IC card, or the like can be used. Also, a storage device such as a hard disk or RAM that is provided with a server system connected to the Internet or a specialized communication network may be used as the recording medium to provide the programs to the computer 1900 via the network.

In the present disclosure, a configuration in which the information processing device 170 includes the CPU 2000 as a processor has been described, but the type of the processor is not particularly limited. For example, a GPU, an ASIA, an FPGA, or the like can be appropriately used as the processor. In addition, in the present disclosure, a configuration in which the information processing device 170 includes the hard disk drive 2040 as an auxiliary storage device has been described, but the type of the auxiliary storage device is not particularly limited. For example, instead of the hard disk drive 2040 or in addition to the hard disk drive 2040, another storage device such as a solid state drive may be used.

While the present invention has been described with the embodiments, the technical scope of the present invention is not limited to the above-described embodiments. It is apparent to persons skilled in the art that various alterations and improvements can be added to the above-described embodiments. It is also apparent from the scope of the claims that the embodiments added with such alterations or improvements can be included in the technical scope of the invention.

The operations, procedures, steps, and stages of each process performed by a device, system, program, and method shown in the claims, embodiments, or diagrams can be performed in arbitrary order as long as the order is not indicated by "prior to," "before," or the like and as long as the output from a previous process is not used in a later process. Even if the operation flow is described by using phrases such as "first" or "next" in the scope of the claims, specification, or drawings, it does not necessarily mean that the process must be performed in this order.

(Supplement)

[Item 1] A method of applying a force to an organism including:

forming a gas-liquid interface between a liquid, in which an organism is immersed, and a gas in a flow channel or at an end portion of the flow channel after arranging the end portion in the liquid;

controlling a vector of a force applied from the gas-liquid interface to the organism; and

applying a force to the organism from the gas-liquid interface.

[Item 2] The method according to item 1, wherein
the controlling the vector includes controlling a magnitude of the force applied from the gas-liquid interface to the organism.

[Item 3] The method according to item 2, wherein
the controlling the vector includes controlling the magnitude of the force applied from the gas-liquid interface to the organism by controlling a gas pressure of the gas at the gas-liquid interface.

[Item 4] The method according to item 3, wherein

the forming the gas-liquid interface is performed by immersing the end portion of the flow channel in the liquid and introducing a gas, which is supplied from a pump, from the end portion into the liquid, and

the controlling the vector includes controlling the gas pressure of the gas at the gas-liquid interface by controlling pressurization of the pump.

[Item 5] The method according to item 3 or 4, wherein

the forming the gas-liquid interface is performed by immersing the end portion of the flow channel in the liquid and introducing a gas from the end portion into the liquid, and

the controlling the vector includes preparing a flow channel having an inner diameter corresponding to a magnitude of a force to be applied to the organism.

[Item 6] The method according to any one of items 3 to 5, wherein
the controlling the vector includes controlling the magnitude of the force applied from the gas-liquid interface to the organism by controlling a curvature radius of a portion of the gas-liquid interface in contact with the organism.

[Item 7] The method according to any one of items 3 to 6, wherein
the controlling the vector includes controlling the magnitude of the force applied from the gas-liquid interface to the organism by controlling a surface tension between the gas and the liquid.

[Item 8] The method according to any one of items 2 to 7, wherein
the controlling the vector includes controlling the magnitude of the force applied from the gas-liquid interface to the organism by controlling a movement acceleration of the gas-liquid interface.

[Item 9] The method according to item 8, wherein

the forming the gas-liquid interface is performed by immersing the end portion of the flow channel in the liquid and introducing a gas from the end portion into the liquid, and

the controlling the vector includes controlling the movement acceleration of the gas-liquid interface by controlling a movement acceleration of the flow channel.

[Item 10]The method according to item 8 or 9, wherein

the forming the gas-liquid interface is performed by introducing a gas, which is supplied from a pump, from the end portion into the liquid, and

the controlling the vector includes controlling the movement acceleration of the gas-liquid interface by controlling a pressurization acceleration of the pump.

[Item 11]The method according to item 1, wherein
the controlling the vector includes performing while detecting a pressure from the gas-liquid interface with a pressure sensor provided at a bottom of a vessel accommodating a liquid.
[Item 12]The method according to item 1, wherein
the controlling the vector includes controlling a direction of the force applied from the gas-liquid interface to the organism.
[Item 13]The method according to item 12, wherein
the controlling the vector includes controlling the direction of the force applied from the gas-liquid interface to the organism by controlling a direction and a moving direction of a surface of a portion of the gas-liquid interface in contact with the organism.
[Item 14]The method according to item 13, wherein
the controlling the vector includes controlling the direction of the surface of the portion of the gas-liquid interface in contact with the organism by controlling a curvature radius of the portion of the gas-liquid interface in contact with the organism.
[Item 15]The method according to item 13 or 14, wherein

the forming the gas-liquid interface is performed by immersing the end portion of the flow channel in the liquid and introducing a gas from the end portion into the liquid, and

the controlling the vector includes controlling the direction of the surface of the portion of the gas-liquid interface in contact with the organism by controlling a distance between the end portion and a bottom of a vessel accommodating the liquid.

[Item 16]The method according to any one of items 13 to 15, wherein

the forming the gas-liquid interface is performed by immersing the end portion of the flow channel in the liquid and introducing a gas from the end portion into the liquid, and

the controlling the vector includes preparing a flow channel having an inner diameter corresponding to a direction of a force to be applied to the organism.

[Item 17]The method according to item 12, wherein
the controlling the vector includes controlling the direction of the force, which is applied from the gas-liquid interface to the organism, from a gas side of the gas-liquid interface to the liquid side or from the liquid side to the gas side of the gas-liquid interface.
[Item 18]The method according to any one of items 1 to 17, wherein
the controlling the vector includes control of moving the gas-liquid interface to be in contact with a predetermined organism.
[Item 19] A device for applying a force to an organism for manipulating an organism, including:

a liquid in which the organism is immersed;

a flow channel having an end portion arranged in the liquid, the flow channel having a gas-liquid interface between the liquid and a gas formed at the end portion or inside the flow channel;

a pump configured to supply a gas to the flow channel or suck a gas from the flow channel; and

a vector control unit configured to apply a force to the organism by the gas-liquid interface while controlling an operation of the pump and thereby controlling a vector of a force applied from the gas-liquid interface to the organism.

[Item 20]The device for applying a force to an organism according to item 19, wherein
the vector control unit is configured to control a magnitude of the force applied from the gas-liquid interface to the organism.
[Item 21]The device for applying a force to an organism according to item 20, wherein
the vector control unit is configured to control the magnitude of the force applied from the gas-liquid interface to the organism by controlling a gas pressure at the gas-liquid interface.
[Item 22]The device for applying a force to an organism according to any one of items 19 to 21, wherein
the vector control unit is configured to control the magnitude of the force applied from the gas-liquid interface to the organism by controlling a movement acceleration of the gas-liquid interface.
[Item 23]The device for applying a force to an organism according to any one of items 19 to 22, wherein
the vector control unit is configured to control a direction of the force applied from the gas-liquid interface to the organism.
[Item 24] A computer program having instructions therein,

the instructions, when executed by a processor or programmable circuitry,

control the processor or programmable circuitry to perform operations including:

forming a gas-liquid interface between a liquid, in which an organism is immersed, and a gas in a flow channel or at an end portion of the flow channel after arranging the end portion in the liquid;

controlling a vector of a force applied from the gas-liquid interface to the organism; and

applying a force to the organism from the gas-liquid interface.

[Item 25]An organism manipulation device including:

a flow channel having an end portion arranged in a liquid stored in a vessel;

a pump configured to introduce a gas into the flow channel to form a gas-liquid interface at the end portion; and

a position control unit configured to control a position of the vessel or the flow channel, wherein

the position control unit is configured to move the flow channel or the vessel to a position at a first distance which is a distance allowing the gas-liquid interface to come into contact with a bottom portion of the vessel, and

to move the flow channel, in a state where the gas-liquid interface formed at the end portion by the pump is brought into contact with the bottom portion of the vessel, to a second position where a distance between the end portion and the bottom portion of the vessel is shorter than the first distance.

EXPLANATION OF REFERENCES

1: fluorescence image observation light source;
2: dichroic mirror;
3: optical deflector;
4: relay lens;
5: dichroic mirror;
6: objective lens;
7: condenser lens;
8: condensing lens;
9: band pass filter;
10: transmission image observation light source;
11: barrier filter;
12: projection lens;
13: barrier filter;
14: projection lens;
15: pinhole;
16: light source;

17: light source;

25: vessel;

25a: bottom portion;

35: manipulation target;

40: nozzle actuator;

41: sample actuator;

42: flow channel imaging camera;

45: light source;

46: light source;

47: pressure generation unit;

48: sensor unit;

49, 49a, 49b: nozzle;

50: microscope unit;

51: flow channel;

51a: first flow channel;

51b: second flow channel;

53: flow channel exchange unit;

54: liquid storage unit;

60: camera;

70: camera;

100: organism manipulation device;

101: manipulation unit;

111: display region;

112: display region;

113: display region;

114: display region;

115: display region;

160: output unit;

170: information processing device;

171: imaging control unit;

180: input unit;

190: recording unit;

200: bubble forming unit;

250: flow channel control unit;

251: pump;

251a: first pump;

251b: second pump;

253: cylindrical portion;

253a: outer cylinder;

253b: inner cylinder;

254: end portion;

255: gas-liquid interface;

256: bubble;

260: liquid control unit;

261: liquid;

300: image processing unit;

1900: computer;

2000: CPU;

2010: ROM;

2020: RAM;

2030: communication interface;

2040: hard disk drive;

2050: flexible disk drive;

2060: CD-ROM drive;

2070: input/output chip;

2075: graphics controller;

2080: display device;

2082: host controller;

2084: input/output controller;
2090: flexible disk; and
2095: CD-ROM.

**Claims**

1. A method of applying a force to an organism comprising:

   forming a gas-liquid interface between a liquid, in which an organism is immersed, and a gas in a flow channel or at an end portion of the flow channel after arranging the end portion in the liquid;
   controlling a vector of a force applied from the gas-liquid interface to the organism; and
   applying a force to the organism from the gas-liquid interface.

2. The method according to claim 1, wherein
   the controlling the vector includes controlling a magnitude of the force applied from the gas-liquid interface to the organism.

3. The method according to claim 2, wherein
   the controlling the vector includes controlling the magnitude of the force applied from the gas-liquid interface to the organism by controlling a gas pressure of the gas at the gas-liquid interface.

4. The method according to claim 3, wherein

   the forming the gas-liquid interface is performed by immersing the end portion of the flow channel in the liquid and introducing a gas, which is supplied from a pump, from the end portion into the liquid, and
   the controlling the vector includes controlling the gas pressure of the gas at the gas-liquid interface by controlling pressurization of the pump.

5. The method according to claim 3 or 4, wherein

   the forming the gas-liquid interface is performed by immersing the end portion of the flow channel in the liquid and introducing a gas from the end portion into the liquid, and
   the controlling the vector includes preparing a flow channel having an inner diameter corresponding to a magnitude of a force to be applied to the organism.

6. The method according to any one of claims 3 to 5, wherein
   the controlling the vector includes controlling the magnitude of the force applied from the gas-liquid interface to the organism by controlling a curvature radius of a portion of the gas-liquid interface in contact with the organism.

7. The method according to any one of claims 3 to 6, wherein
   the controlling the vector includes controlling the magnitude of the force applied from the gas-liquid interface to the organism by controlling a surface tension between the gas and the liquid.

8. The method according to any one of claims 2 to 7, wherein
   the controlling the vector includes controlling the magnitude of the force applied from the gas-liquid interface to the organism by controlling a movement acceleration of the gas-liquid interface.

9. The method according to claim 8, wherein

   the forming the gas-liquid interface is performed by immersing the end portion of the flow channel in the liquid and introducing a gas from the end portion into the liquid, and
   the controlling the vector includes controlling the movement acceleration of the gas-liquid interface by controlling a movement acceleration of the flow channel.

10. The method according to claim 8 or 9, wherein

    the forming the gas-liquid interface is performed by introducing a gas, which is supplied from a pump, from the end portion into the liquid, and
the controlling the vector includes controlling the movement acceleration of the gas-liquid interface by controlling a pressurization acceleration of the pump.

11. The method according to claim 1, wherein
the controlling the vector includes controlling a direction of the force applied from the gas-liquid interface to the organism.

12. The method according to claim 11, wherein
the controlling the vector includes controlling the direction of the force applied from the gas-liquid interface to the organism by controlling a direction and a moving direction of a surface of a portion of the gas-liquid interface in contact with the organism.

13. The method according to claim 12, wherein
the controlling the vector includes controlling the direction of the surface of the portion of the gas-liquid interface in contact with the organism by controlling a curvature radius of the portion of the gas-liquid interface in contact with the organism.

14. The method according to claim 12 or 13, wherein

the forming the gas-liquid interface is performed by immersing the end portion of the flow channel in the liquid and introducing a gas from the end portion into the liquid, and
the controlling the vector includes controlling the direction of the surface of the portion of the gas-liquid interface in contact with the organism by controlling a distance between the end portion and a bottom of a vessel accommodating the liquid.

15. The method according to any one of claims 12 to 14, wherein

the forming the gas-liquid interface is performed by immersing the end portion of the flow channel in the liquid and introducing a gas from the end portion into the liquid, and
the controlling the vector includes preparing a flow channel having an inner diameter corresponding to a direction of a force to be applied to the organism.

16. The method according to claim 11 or 12, wherein
the controlling the vector includes controlling the direction of the force, which is applied from the gas-liquid interface to the organism, from a gas side of the gas-liquid interface to the liquid side or from the liquid side to the gas side of the gas-liquid interface.

17. The method according to any one of claims 1 to 16, wherein
the controlling the vector includes control of moving the gas-liquid interface to be in contact with a predetermined organism.

18. A device for applying a force to an organism for manipulating an organism, comprising:

a liquid in which the organism is immersed;
a flow channel having an end portion arranged in the liquid, the flow channel having a gas-liquid interface between the liquid and a gas formed at the end portion or inside the flow channel;
a pump configured to supply a gas to the flow channel or suck a gas from the flow channel; and
a vector control unit configured to apply a force to the organism by the gas-liquid interface while controlling an operation of the pump and thereby controlling a vector of a force applied from the gas-liquid interface to the organism.

19. An organism manipulation device comprising:

a flow channel having an end portion arranged in a liquid stored in a vessel;
a pump configured to introduce a gas into the flow channel to form a gas-liquid interface at the end portion; and
a position control unit configured to control a position of the vessel or the flow channel, wherein
the position control unit is configured to move the flow channel or the vessel to a position at a first distance which is a distance allowing the gas-liquid interface to come into contact with a bottom portion of the vessel, and to move the flow channel, in a state where the gas-liquid interface formed at the end portion by the pump is brought into contact with the bottom portion of the vessel, to a second position where a distance between the end portion and the bottom portion of the vessel is shorter than the first distance.

*FIG.1A*

100

*FIG.1B*

49

251

51

253
254

51

FIG.2A

<u>49</u>

*FIG.2B*

49

251a
251b

253
51a
51b
254

253a
253b

FIG.3A

49

253
51a
51b
261
254
255

FIG.3B

*FIG.4*

```
┌─────────────────────────────────┐
│  ┌───────────────────────────┐  │
│  │   IMAGING CONTROL UNIT     │  │ 171
│  └───────────────────────────┘  │
│  ┌───────────────────────────┐  │
│  │      RECORDING UNIT        │  │ 190
│  └───────────────────────────┘  │
│  ┌───────────────────────────┐  │
│  │    BUBBLE FORMING UNIT     │  │ 200
│  └───────────────────────────┘  │
│  ┌───────────────────────────┐  │
│  │ FLOW CHANNEL CONTROL UNIT  │  │ 250
│  └───────────────────────────┘  │
│  ┌───────────────────────────┐  │
│  │    LIQUID CONTROL UNIT     │  │ 260
│  └───────────────────────────┘  │
│  ┌───────────────────────────┐  │
│  │   IMAGE PROCESSING UNIT    │  │ 300
│  └───────────────────────────┘  │
│  INFORMATION                     │
│  PROCESSING DEVICE               │
└─────────────────────────────────┘
                              170
```

FIG.5

*FIG.6*

*FIG.7A*

*FIG.7B*

| I D | COORDINATES | MAGNITUDE | MOVEMENT DESTINATION |
|---|---|---|---|
| aaa | $(X_{aaa}, Y_{aaa})$ | AAA | A1 |
| bbb | $(X_{bbb}, Y_{bbb})$ | BBB | A2 |
| ccc | $(X_{ccc}, Y_{ccc})$ | CCC | A3 |
| | | | |
| | | | |

FIG.7C

▽ CELL MANIPULATION

⊙ CELL RECOVERY (PASSAGE)

○ CELL RECOVERY (ANALYSIS)

○ CELL REMOVAL, CUT

○ CYTOPLASM RECOVERY

○ CELL RETENTION, MOVEMENT

○ CELL ADHESIVE FORCE MEASUREMENT

111

▽ CELL COMPRESSION

○ CELL MECHANICS ANALYSIS

○ DEEP CELL PORTION OBSERVATION

○ CELL SUPER-RESOLUTION OBSERVATION

112

*FIG.7D*

APPLICATION

| CELL RECOVERY (ANALYSIS) ▼ |     ⎤
                                    ⎥ 113

MANIPULATION CELL MORPHOLOGY

○ CYTOPLASM  ⦿ SINGLE CELL  ○ CELL POPULATION (COLONY)  ○ SPHEROID     ⎤ 114

MANIPULATION

⦿ RECOVERY  ○ RETENTION  ○ REMOVAL  ○ COMPRESSION     ⎤ 115

*FIG.7E*

S600

FIG.8

S200

○ FROM S180

OPERATE XYZ POSITION CONTROL ACTUATOR
S210

CAPTURE IMAGE OF NOZZLE END
S215

IS IT DIFFERENT FROM SET XYZ POSITION? — Yes → DECIDE OPERATION AMOUNT OF XYZ POSITION CONTROL ACTUATOR
S220
S225

No

○ TO S300

*FIG.9A*

S200

○ FROM S180

OPERATE Z POSITION CONTROL ACTUATOR
S230

MEASURE LOAD OF Z POSITION
CONTROL ACTUATOR
S235

IS IT
EQUAL TO OR LESS THAN
SET LOAD?
S240

Yes → DECIDE OPERATION AMOUNT OF
Z POSITION CONTROL ACTUATOR
S242

No

SET INITIAL Z POSITION
S245

OPERATE XYZ POSITION CONTROL ACTUATOR
S250

CAPTURE IMAGE OF NOZZLE END
S252

IS IT
DIFFERENT FROM SET XYZ
POSITION?
S254

Yes → DECIDE OPERATION
AMOUNT OF XYZ POSITION
CONTROL ACTUATOR
S256

No

○ TO S300

*FIG.9B*

## S200

○ FROM S180

FORM BUBBLE — S260

OPERATE Z POSITION CONTROL ACTUATOR — S262

MEASURE INTERNAL PRESSURE OF BUBBLE — S264

SET INTERNAL PRESSURE CHANGE? — S266

No → DECIDE OPERATION AMOUNT OF Z POSITION CONTROL ACTUATOR — S268

Yes

REMOVE BUBBLE — S270

SET INITIAL Z POSITION — S272

OPERATE XYZ POSITION CONTROL ACTUATOR — S274

CAPTURE IMAGE OF NOZZLE END — S276

IS IT DIFFERENT FROM SET XYZ POSITION? — S280

Yes → DECIDE OPERATION AMOUNT OF XYZ POSITION CONTROL ACTUATOR — S282

No

○ TO S300

*FIG.9C*

60

S300

FROM S200

OPERATE PUMP — S320

CAPTURE IMAGE OF NOZZLE END — S330

IS IT DIFFERENT FROM SET BUBBLE SHAPE? — S340

Yes

No

DECIDE OPERATION AMOUNT OF PUMP — S342

TO S400

*FIG.10A*

S300

FIG.10B

FIG.11A

802a

802b

802c

802d

*FIG.11B*

804a

804b

804c

804d

*FIG.11C*

FIG.11D

*FIG.11E*

|  | Day 4 :<br>TRANSMISSION<br>IMAGE OBSERVATION | Day 10 :<br>ALP STAIN (P1) | Day 30 :<br>ALP STAIN (P3) |
| --- | --- | --- | --- |
| EMBODIMENT | 830a | 831a | 832a |
| COMPARATIVE<br>EXAMPLE | 830b<br>(n=4) | 831b<br>(n=4) | 832b<br>(n=3) |

*FIG.11F*

FIG.11G

FIG.11H

FIG.11I

S500

FROM S400

OPERATE PUMP
S510

CAPTURE IMAGE OF NOZZLE END
S520

IS IT DIFFERENT FROM SET GAS-LIQUID INTERFACE POSITION?
S530
Yes
DECIDE OPERATION AMOUNT OF PUMP
S532

No

DETACH CELL FROM INTERFACE?
S540
Yes
TAKE OUT NOZZLE FROM LIQUID
S542
MOVE INTERFACE AT HIGH SPEED
S544

No

TO S640

*FIG.12A*

EP 4 219 677 A1

S500

FROM S400

OPERATE PUMP — S560

MEASURE INTERNAL PRESSURE OF NOZZLE — S570

IS IT DIFFERENT FROM SET INTERNAL PRESSURE? — S580

Yes → DECIDE OPERATION AMOUNT OF PUMP — S582

No

DETACH CELL FROM INTERFACE? — S590

Yes → TAKE OUT NOZZLE FROM LIQUID — S592 → MOVE INTERFACE AT HIGH SPEED — S594

No

TO S640

FIG.12B

73

FIG.13

S720

```
CONTROL OF
MAGNITUDE OF FORCE          910
```

CONTROL OF INTERNAL
PRESSURE OF BUBBLE          911

CONTROL BY PRESSURE
GENERATION UNIT          913

CONTROL OF
CURVATURE RADIUS          914

CONTROL OF INNER
DIAMETER OF NOZZLE          918

CONTROL OF DISTANCE BETWEEN
END PORTION OF NOZZLE AND
BOTTOM PORTION OF VESSEL          919

CONTROL OF INTERFACE
FREE ENERGY          915

CONTROL OF MOVEMENT
ACCELERATION O
GAS-LIQUID INTERFACE          912

CONTROL OF MOVEMENT
ACCELERATION OF NOZZLE          916

CONTROL OF PRESSURIZATION
ACCELERATION          917

CONTROL OF
DIRECTION OF FORCE          920

CONTROL OF INNER
DIAMETER OF NOZZLE          921

CONTROL OF DISTANCE
BETWEEN END PORTION OF NOZZLE
AND BOTTOM PORTION OF VESSEL          922

REGULATION OF
TRAVELING DIRECTION          923

*FIG.14*

*FIG.15*

*FIG.16*

*FIG.17*

FIG.18

(a)

(b)

51
49a
261
255
25a

254   256   35

51
49b
261
255
25a

254 256  35

Q

P

255

25a

256

D1

35

Q

P

255

25a

256

D2

35

*FIG.19*

(a)

(b)

FIG.20

| INNER DIAMETER OF NOZZLE | LARGE : 150～1500μm | LARGE : 150～1500μm | SMALL : 10～100μm |
|---|---|---|---|
| DISTANCE FROM BOTTOM PORTION | LONG : 75～1000μm | SHORT : 10～100μm | SHORT : 5～50μm |
| CURVATURE RADIUS OF BUBBLE | LARGE : 37. 5～1000μm | SMALL : 5～300μm | SMALL : 2. 5～100μm |
| MAXIMUM VALUE OF APPLIED FORCE | SMALL | LARGE | LARGE |
| DOWNWARD FORCE (COMPRESSION FORCE) | LARGE | SMALL | SMALL |
| LATERAL FORCE (SEPARATING FORCE) | SMALL | LARGE | LARGE |
| CELL ATTACHMENT AREA | LARGE | SMALL | CONSIDERABLY SMALL |
| BUBBLE CONTROLLABILITY | CRUDE CONTROL | PRECISION CONTROL | PRECISION CONTROL |
| NUMBER OF TARGET CELLS | 10～1000 | 10～1000 | 1～10 |
| CELL ADHESION ALLEVIATION PROCESSING | NECESSARY (STRONG) | NECESSARY (WEAK) OR UNNECESSARY | NECESSARY (WEAK) OR UNNECESSARY |

*FIG.21*

EP 4 219 677 A1

FIG.22

*FIG.23*

## INTERNATIONAL SEARCH REPORT

| International application No. |
| --- |
| **PCT/JP2021/035198** |

### A. CLASSIFICATION OF SUBJECT MATTER

*C12M 1/26*(2006.01)i; *C12M 1/08*(2006.01)i; *C12N 5/071*(2010.01)i; *G01N 1/00*(2006.01)i; *G01N 1/04*(2006.01)i
FI:  C12M1/26; C12N5/071; G01N1/00 101A; G01N1/04 H; G01N1/04 W; C12M1/08

According to International Patent Classification (IPC) or to both national classification and IPC

### B. FIELDS SEARCHED

Minimum documentation searched (classification system followed by classification symbols)

C12M1/26; C12M1/08; C12N5/071; G01N1/00; G01N1/04

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
| --- | --- | --- |
| X | JP 2010-172231 A (OLYMPUS CORP.) 12 August 2010 (2010-08-12) paragraphs [0030], [0061]-[0079], fig. 5, 6 | 1-3, 8, 11, 16-18 |
| A | WO 2019/163270 A1 (YAMAHA MOTOR CO., LTD.) 29 August 2019 (2019-08-29) fig. 8 (B) | 1-19 |
| A | WO 2019/086313 A1 (1CELL BIO) 09 May 2019 (2019-05-09) fig. 3 (D) | 1-19 |
| A | PARK, Il Song et al. On-chip micromanipulation using a magnetically driven micromanipulator with an acoustically oscillating bubble. Sensors and Actuators A, 2016, 248, pp. 214-222 particularly, fig. 6 | 1-19 |
| P, X | WO 2020/196635 A1 (NIKON CORP.) 01 October 2020 (2020-10-01) claims, fig. 2, 3, 5, 7, 8 | 1-19 |

☐ Further documents are listed in the continuation of Box C.   ☑ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br><br>**12 October 2021** | Date of mailing of the international search report<br><br>**26 October 2021** |
| Name and mailing address of the ISA/JP<br><br>**Japan Patent Office (ISA/JP)**<br>**3-4-3 Kasumigaseki, Chiyoda-ku, Tokyo 100-8915**<br>**Japan** | Authorized officer<br><br><br><br>Telephone No. |

Form PCT/ISA/210 (second sheet) (January 2015)

INTERNATIONAL SEARCH REPORT
Information on patent family members

International application No.

**PCT/JP2021/035198**

| Patent document cited in search report | | | Publication date (day/month/year) | Patent family member(s) | | | Publication date (day/month/year) |
|---|---|---|---|---|---|---|---|
| JP | 2010-172231 | A | 12 August 2010 | (Family: none) | | | |
| WO | 2019/163270 | A1 | 29 August 2019 | US | 2020/0398265 | A1 | |
| | | | | fig. 8B | | | |
| | | | | EP | 3739035 | A1 | |
| WO | 2019/086313 | A1 | 09 May 2019 | US | 2020/0338552 | A1 | |
| | | | | fig. 3 (D) | | | |
| WO | 2020/196635 | A1 | 01 October 2020 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019030263 A
- JP HEI713083 A
- JP 3814869 B